# EUROPEAN PATENT SPECIFICATION

(11) **EP 0 975 800 B1**
(45) Date of publication and mention of the grant of the patent: **21.05.2003**
(21) Application number: 98910911.1
(22) Date of filing: 23.02.1998
(51) Int. Cl.: C12Q 1/68

(54) **ASSAYING NUCLEOTIDES IN SOLUTION USING PNA PROBES**
UNTERSUCHUNG VON NUKLEOTIDEN IN LOESUNG MIT HILFE VON PNA-SONDEN
DOSAGE DE NUCLEOTIDES DANS UNE SOLUTION A L'AIDE DE SONDES D'ACIDES NUCLEIQUES PEPTIDIQUES

(30) Priority: 27.02.1997 US 807901; 06.06.1997 US 870370
(43) Date of publication of application: 02.02.2000
(73) Proprietor: INGENEUS CORP., Bridgetown (BB)
(72) Inventor: NIE, Eileen, Xiao-Feng, Thornhill, Ontario L3T 1L9 (CA); WU, Yuan, Min, Thornhill, Ontario L3T 1L9 (CA)
(74) Representative: Jump, Timothy John Simon
(86) International application number: IB9800521
(87) International publication number: WO98038334

(56) References cited:
- EP-A- 0 232 967
- WO-A-92/18650
- WO-A-93/24652
- WO-A-94/25477
- WO-A-97/12995
- CARLSSON C ET AL: "SCREENING FOR GENETIC MUTATIONS" NATURE, vol. 380, 21 March 1996, page 207 XP002022963

## Description

### BACKGROUND OF THE INVENTION

### Field of Invention

The invention relates to probes comprising peptide nucleic acids (PNAs), and to a method of using PNA probes to sequence or to assay nucleotides in solution, without solid support or adjacent double stranded nucleotide construct.

### Description of Related Art

PNAs are polyamide analogs of DNA and RNA. See, e.g., U.S. Patent No. 5,539,082 to Nielsen et al. Nielsen et al. discloses that PNAs mimic natural polynucleotides by binding complementary single stranded (ss) DNA and RNA strands. PNAs generally comprise ligands linked to a peptide backbone. Representative ligands include either the four main naturally occurring DNA bases (i.e., thymine, cytosine, adenine or guanine) or other naturally occurring nucleobases (e.g., inosine, uracil, 5-methylcytosine or thiouracil) or artificial bases (e.g., bromothymine, azaadenines or azaguanines, etc.) attached to a peptide backbone through a suitable linker.

Probes comprising PNA sequences have been employed to detect target nucleotide sequences. U.S. Patent No. 5,503,980 to Cantor suggests employing PNA probes in a method of sequencing a nucleic acid by hybridizing the nucleic acid with a set of PNA probes containing random, but determinable, base sequences within the single stranded portion adjacent to a double stranded portion, wherein the single stranded portion of the set preferably comprises every possible combination of sequences over a predetermined range. Hybridization occurs by complementary recognition of the single stranded portion of a target with the single stranded portion of the probe and is thermodynamically favored by the presence of adjacent double strandedness of the probe.

However, although Cantor discloses that the nucleic acids can be PNAs, it does not disclose or suggest utilizing such probes in the absence of a solid support. Moreover, the present invention does not require the adjacent construct of DNA material being tested.

In addition to teaching the use of a solid support like Cantor, Perry-O'Keefe et al., "Peptide Nucleic Acid Pre-Gel Hybridization: An Alternative to Southern Hybridization," 93 Proc. Natl. Acad. Sci. USA 14670 (December 1996) also teaches that PNA does not generally bind well to double stranded DNA (dsDNA). See Perry-O'Keefe et al. at page 14673, footnote. Moreover, the homopyrimidine PNA constructs which have been found to bind dsDNA well would not be useful as probes. Applicants have discovered that the qualification which suggests that only homopyrimidine can bind with dsDNA by strand inversion is incorrect and arises from the hybridization conditions employed.

Smulevitch et al., "Enhancement of Strand Inversion by Oligonucleotides Through Manipulation of Backbone Charge," 14 Nature Biotechnology 1700 (Dec. 1996) (disclosed in Landsdorp, "Close Encounters of the PNA Kind," 14 Nature Biotechnology 1653 (Dec. 1996)) discloses using PNA primers to hybridize with dsDNA. However, Smulevitch et al. teaches the use of gels in detecting hybridization, and does not suggest the use of fluorescent markers.

Many types of sample analysis rely upon the fluorescent properties of a stain. Fluorescence occurs when a molecule excited by light of one wavelength returns to the unexcited (ground) state by emitting light of a longer wavelength. The exciting and emitted light, being of different wavelengths, can be separated from one another using optical filters, a camera or a CCD. Fluorescence has been used to visualize certain molecules (and hence structures) by light microscopy for many years, and is also used in other analytical techniques, such as flow cytometry. Further, the emission of fluorescence showing different colors can be detected by a human eye, a camera or a charge coupled device (CCD).

For example, U.S. Patent No. 5,594,138 to Dykstra et al. discloses a method of fluorescent detection of a nucleic acid. The method comprises contacting the nucleic acid with a fluorescent marker that is a bis-dicationic aryl furan compound and exposing the nucleic acid to light at a frequency inducing fluorescence of the fluorescent marker. The fluorescent marker may be conjugated to a nucleotide sequence as a probe for hybridization studies, or it may be conjugated to numerous reagents for in situ labeling studies.

U.S. Patent No. 4,963,477 to Tchen discloses a probe of high sensitivity containing a modified nucleic acid, which can be recognized by specific antibodies.

Fluorescent In Situ Hybridization (FISH) is a technique comprising detecting fluorescent probe binding to human chromosomes by attaching DNA to a solid support, such as a glass slide. See, e.g., K.H. Andy Choo, Ed., "In Situ Hybridization Protocols," Chapters 2 and 4 (Humana Press, Totowa, NJ, 1994). Like all other conventional detection methods comprising hybridization with probes, this method relies on the solid support to keep the two complementary strands of DNA apart while the probe hybridizes with one of the strands. In addition, FISH requires a complicated buffer and temperature control protocol, with overnight incubation.

WO 93/24652 relates to in situ hybridization of DNA or PNA with nucleic acid-containing chromosomatic or extrachromosomal target objects. However, this document is silent regarding distinguishing between different hybridization complexes.

Carlsson et al., 380 Nature 207 (21 March 1996) relates to genetic mutation screening using capillary electrophoresis. This document does not teach or suggest an assay wherein the fluorescent intensity of the irradiated marker in the test medium is inversely proportional to the number of base mismatches between the target nucleotide sequence and the PNA probe, over a range inclusive of 0 base mismatches through at least 3 base mismatches.

WO 92/18650 relates to fluorescent anisotropy techniques for detecting nucleic acid hybridization, unlike the present invention, which measures total fluorescent emission intensity changes associated with hybridization.

Until the present invention, however, it has not been possible to rapidly test for the presence of nucleotide sequences in solution using a method which does not destroy the sample, is less hazardous to laboratory personnel than radiation based assays, does not require the cost and delay of preparing solid supports, and is readily automated. Time and cost efficient detection of mutant genetic sequences has been the rate limiting step in correlating mutant genotypes with altered phenotypes. Although conventional DNA sequencing methods have been considered to be the most accurate means of identifying mutations, these methods have been relatively slow and labor intensive, and are not particularly well-suited to rapidly screening large numbers of samples of genomic DNA.

### SUMMARY OF THE INVENTION

The present invention provides methods for detecting nucleic acid sequences and/or determining sequence information from nucleic acids. Probes according to the present invention include PNA.

The invention provides a method for detecting at least one single stranded or double stranded nucleotide sequence in a liquid medium, the method comprising adding to the liquid medium PNA probes having at least one marker each to form at least one hybridization complex with at least one nucleotide sequence in the medium, detecting the at least one nucleotide sequence by detecting at least one signal correlated with an amount of the at least one hybridization complex in the liquid medium.

The method can be conducted without binding the PNA probes, nucleotide sequences or hybridization complexes to a solid support or gel.

### BRIEF DESCRIPTION OF THE DRAWINGS

The invention will be described in conjunction with the following drawings in which like reference numerals designate like elements and wherein:
Figure 1 is a schematic depiction of an apparatus according to the invention; and
Figures 2, 3A, 3B, 3C, 4A, 4B, 4C, 5A, 5B, 6A, 6B, 7A, 7B, 8A, 8B, 8C, 9A, 9B, 9C, 9D, 10A, 10B, 11A, 11B, 12A, 12B, 13A, 13B, 13C, 14A, 14B, 15A, 15B, 16A, 16B, 16C, 16D, 17A, 17B, 17C, 18A, 18B, 19A, 19B, 19C, 19D and 19E are fluorescent spectra.

### DETAILED DESCRIPTION OF PREFERRED EMBODIMENTS

The invention utilizes PNA probes to detect and/or characterize nucleotide sequences in a sample. PNA probes are able to recognize dsDNA by binding one strand, thereby presumably hybridizing with the other strand to generate a PNA-DNA complex. Such recognition can take place to dsDNA target sequences 20 or more base pairs long. Probe sequences having any length from 8 to 20 bases are preferred since this is the range within which the smallest unique DNA sequences of prokaryotes and eukaryotes are found. Probes of 12 to 18 bases are particularly preferred since this is the length of the smallest unique sequences in the human genome. However, a plurality of shorter probes can be used to detect a nucleotide sequence having a plurality of non-unique target sequences therein, which combine to uniquely identify the nucleotide sequence.

The probes of the invention are able to form triplex complexes with dsDNA and duplex complexes with RNA or ssDNA. The compounds of the invention are also able to form triplex complexes wherein a first PNA probe binds with RNA or ssDNA and a second ssDNA strand binds with the resulting duplex complex. See, e.g., Egholm et al., "PNA Hybridizes to Complementary Oligonucleotides Obeying the Watson-Crick Hydrogen-Bonding Rules," 365 Nature 566 (1993), and Tomac et al., "Ionic Effects on the Stability and Conformation of Peptide Nucleic Acid Complexes," 118 J.Am.Chem.Soc. 5544 (1996).

In the PNA probes according to the invention, the bases attached to the polyamide backbone are primarily naturally occurring nucleobases attached at the position required by probe manufacture. Alternatively, the bases may be non-naturally occurring nucleobases (nucleobase analogs), other base-binding moieties, aromatic moieties, (C1-C4) alkanoyls, hydroxyls or even hydrogens. It will be understood that the term nucleobase includes nucleobases bearing removable protecting groups. Furthermore, at least one base on the polyamide skeleton can be replaced with, or substituted with, a DNA intercalator, a reporter ligand such as, for example, a fluorophore, radio label, spin label, hapten, or a protein-recognizing ligand such as biotin. Preferred detectable labels include a radioisotope, a stable isotope, an enzyme, a fluorescent chemical, a luminescent chemical, a chromatic chemical, a metal, an electric charge, or a spatial structure.

In particularly preferred embodiments, the PNA probe comprises a PNA sequence covalently bonded to a fluorescent marker, which fluoresces when irradiated with a laser. Preferred fluorescent markers include biotin, rhodamine and fluorescein.

It is preferred to provide the fluorescent marker at the 5' terminal of the PNA with a short linker to minimize interaction with the PNA.

In order to distinguish a mutant nucleotide sequence from a reference nucleotide sequence, wherein the two sequences differ by as little as a single base, it is preferred to design the PNA probe so that the mutant portion of the mutant nucleotide corresponds to the center of the PNA probe. This design results in a higher hybridization yield and a more stable hybrid than when the mutant portion of the nucleotide corresponds to a terminus of the probe, since the bonding mismatch between probe and nucleotide is located centrally within the probe.

PNA probes are added to a liquid medium suspected of containing at least one nucleotide sequence, and/or a mutant version of the at least one sequence. The liquid medium can be any conventional medium known to be suitable for preserving nucleotides. See, e.g., Sambrook et al., "Molecular Cloning: A Lab Manual," 2d (1989). For example, the liquid medium can comprise nucleotides, water, buffers and surfactants.

The nucleotides in the liquid medium can be obtained from clinical samples by any conventional method, including an automated method. Examples of such methods are summarized in, e.g., Sambrook et al., Vol. 2, pp. 9.16-9.19 and 7.6 to 7.7. An example of an automated nucleic acid purifying apparatus is the BioRobot 9600 manufactured by Quiagen.

For example, a variety of diseases are known to be linked with the presence of mutant DNA in an individual's genome. If the sequences of the wild type DNA and the mutant DNA are known, it is possible to isolate these nucleotide sequences from clinical samples using conventional technology. PCR is the preferred method of isolating nucleotides from clinical samples. PCR is conducted using a primer which is capable of amplifying the wild type DNA and the mutant DNA.

The nucleotide sequences are added to the liquid medium in a known concentration, since the concentration can affect the magnitude of the signal (e.g., fluorescent intensity) generated in subsequent steps in the inventive method. The nucleotide concentration can be determined by, e.g., measuring the UV absorption at 260 nm.

The isolated nucleotides are added to the liquid medium and denatured prior to being detected. Preferably, the denaturation is conducted at about 90°C to about 100°C from about 30 seconds to about 5 hours in the presence of PNA probe.

Preferably, PNA probes are added to the liquid medium in a concentration 1 to 20 times the concentration of the nucleotide sequence to be detected.

Hybridization between complementary bases occurs under a wide variety of conditions having variations in temperature, salt concentration, electrostatic strength, and buffer composition. Examples of these conditions and methods for applying them are known in the art. See, e.g., Perry-O'Keefe et al., Egholm et al., Tomac et al., Sambrook et al., Vol. 2 pp. 9.47-9.55 and the Pre-Gel Hybridization Technique taught in Vol. 4, No. 3 of PerSeptive Biosystems Magazine.

It is preferred that hybridization complexes be formed at a temperature of about 4°C to about 75°C for about 2 minutes to about 24 hours.. It is particularly preferred to conduct denaturing for no more than 60 minutes in the presence of PNA probe, after which the temperature is passively cooled to room temperature without quenching.

It is possible to facilitate hybridization in solution by using certain reagents. Preferred examples of these reagents include single stranded binding proteins such as Rec A protein, T4 gene 32 protein, E. coli single stranded binding protein, major or minor nucleic acid groove binding proteins, divalent ions, polyvalent ions, and intercalating substances such as ethidium bromide, actinomycin D, psoralen, and angelicin.

In embodiments of the inventive method, hybridization complexes are separated from unhybridized PNA probes prior to detecting the signal of the hybridized PNA probes. The separation is accomplished by at least one of filtration, centrifugation, precipitation, ion exchange resin separation and free solution electrophoresis (i.e., electrophoresis in a liquid medium as opposed to gel electrophoresis).

Separation can be accomplished by G50 column. After hybridization, the mixture of hybridization complexes and unhybridized DNA and PNA is transferred into a G50 column. The column is centrifuged at 500 to 1000 rpm for 1 to 2 minutes. The unbound PNA is filtered and retained in the column. The solution with the hybrids of PNA-DNA is passed through column and collected in a cuvette.

In fact, centrifugation can be avoided. The solution can flow through the column by gravity or washing using extra amounts of buffer. However, centrifugation is for the purpose of reducing the separation time and avoiding the sample being diluted.

Separation can be accomplished by centrifugation. After the hybridization, the sample (without transferring) is separated into two layers (gradient) by centrifugation at 1000 to 10000 rpm for 4 to 20 hours. The lighter unhybridized PNA is abundant in the upper layer, while the heavier hybridization complex is concentrated in the lower layer. The lower layer is collected in a cuvette for fluorescence measurement.

In certain embodiments, a filtration step is avoidable if an electrical method is used to concentrate hybridized PNA. In these embodiments, an electric field is applied to the liquid medium prior to or concurrently with detecting the desired nucleotide sequence, and a change in an intensity of fluorescent emission as a function of the electric field is detected as an indication of whether the PNA probe is hybridized to at least one of a completely complementary nucleotide sequence and an incompletely complementary nucleotide sequence.

The preferred markers for use in the invention are fluorophores. As will be appreciated by the skilled artisan, the wavelength preferably selected to induce fluorescence of the fluorescent marker is known in the art as the "excitation maximum," i.e., that wavelength which is absorbed by a molecule and excites that molecule to a higher electronic state. When the marker molecule passes from the higher to a lower electronic state, the molecule emits a type of visible radiation, i.e., fluorescence, at a wavelength referred to as the "emission maximum." It is this fluorescence that is detected in the present invention. The detectable signal emitted by the compound can be detected using techniques known in the art, for example by observation with the human eye, using electronic means for detecting a generated wavelength (e.g., cameras and CCDs), and the like. Advantageously, the wavelength of fluorescence is sufficiently removed from that of the exciting light to allow good separation of the two wavelengths by optical filters.

The excitation wavelength is selected (by routine experimentation and/or conventional knowledge) to correspond to this excitation maximum for the marker being used, and is preferably 400 to 1000 nm, more preferably 400 to 750 nm. For example, when the marker is fluoroscein, the preferred wavelength of excitation is about 488 nm.

In preferred embodiments, an argon ion laser is used to irradiate the marker with light having a wavelength in a range of 400 to 520 nm, and fluorescent emission is detected in a range of 500 to 750 nm. The duration of irradiation is preferably about 10 milliseconds to about 1 minute.

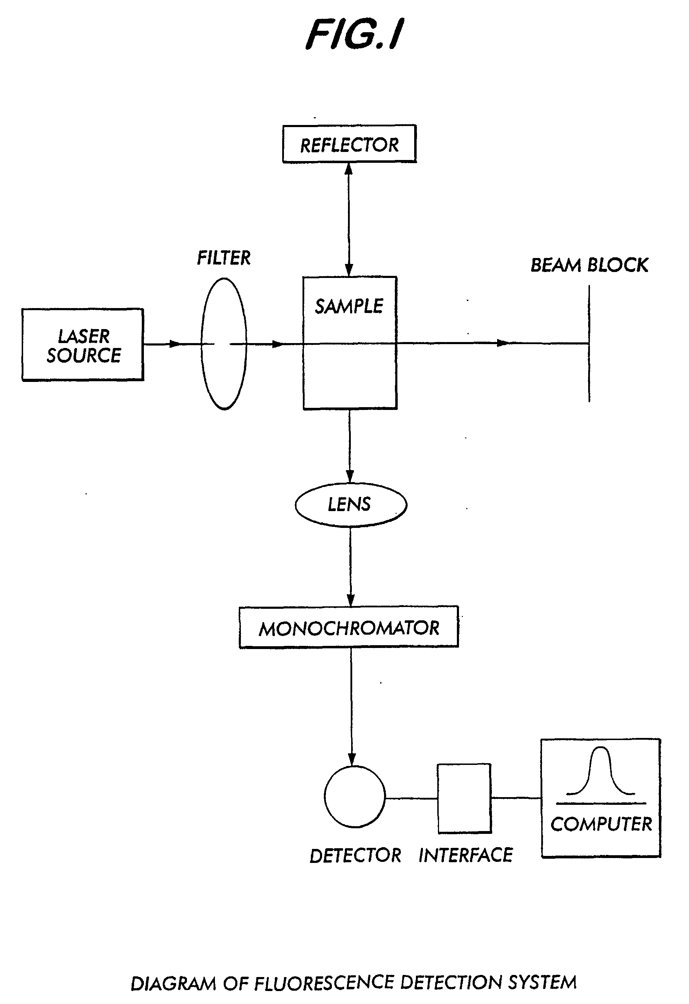

An apparatus for performing the inventive method can comprise a liquid medium container for containing the liquid medium; a laser for irradiating the nucleotide; a fluorescence detector for detecting fluorescence induced by the laser; a data analysis device for analyzing data generated by the fluorescence detector; and an output device which reports the data analysis generated by the data analysis device. See, e.g., Fig. 1, which shows a schematic diagram of a fluorescence detection system suitable for use with the method of the invention.

In certain embodiments, fluorescent emission generated by irradiating hybridized PNA probes with a light source is distinguished from fluorescent emission of unhybridized PNA probes, without separating hybridized and unhybridized PNA probes. In certain embodiments, the fluorescent emission of one type of PNA probe hybridized to one nucleotide sequence is distinguished from the fluorescent emission of another type of PNA probe hybridized to a nucleotide sequence other than the first nucleotide sequence. For example, the presence of either of two nucleotide sequences differing by as little as a single nucleotide can be detected on the basis that a fluorescent PNA probe precisely complementary to one of the two nucleotides will bond more effectively with the precise complement than with the imprecise complement, thus providing a higher concentration of hybridization complexes in solution and a higher fluorescent intensity after removal of unhybridized PNA probe.

A plurality of PNA probes can be employed simultaneously to achieve a variety of effects. Several probes targeted for different segments of a single nucleotide sequence can be employed to enhance the reliability of the detection method. Similarly, one probe can target one strand of dsDNA, while another probe can target the complementary strand of dsDNA.

A preferred method of detecting whether DNA is mutant type or the correspondng wild type comprises the simultaneous use of (a) a first type of PNA probe targeted to a sequence that occurs in both the wild type and mutant type DNA but is otherwise unique, and (b) a second type of PNA probe targeted to a sequence unique to the mutant type DNA, wherein the first and second types of PNA probe have different markers that produce distinguishable signals. Thus, detection of the first probe signal indicates that the test was run properly (i.e., the first probe functions as a positive control) and detection of the second probe signal indicates that the mutant type DNA is present. For example, one probe can have a fluorescein marker exhibiting a fluorescent emission intensity peak at 525 nm while the other probe can have a rhodamine marker exhibiting a fluorescent emission intensity peak at 580 nm.

In contrast to prior art detection methods, the present invention makes it possible to limit the total volume of the liquid medium (i.e., the sample to be analyzed) in certain embodiments to no more than about 200 microliters. It is also possible to limit the total volume in certain embodiments to no more than about 10 microliters.

When testing for mutant dsDNA using PNA, if a result is obtained for which there remains doubt, a further test may be immediately performed on the sample by adding the complementary PNA probe to test the complementary strand of DNA. If centrifugated as part of the first test, the sample would be removed to a new tube and hybridized with the complementary PNA. Alternatively, the PNA test can be done with both the PNA and complementary PNA probes hybridized to each of the denatured DNA strands in the first instance and at the same time.

For forensic applications, samples can be tested, stored and then retested because PNA is expelled from hybridization over a couple of days, and DNA recombines over time and does not degrade by this procedure. Accordingly, a sample frozen after testing can be subsequently retested in the same tube a number of times.

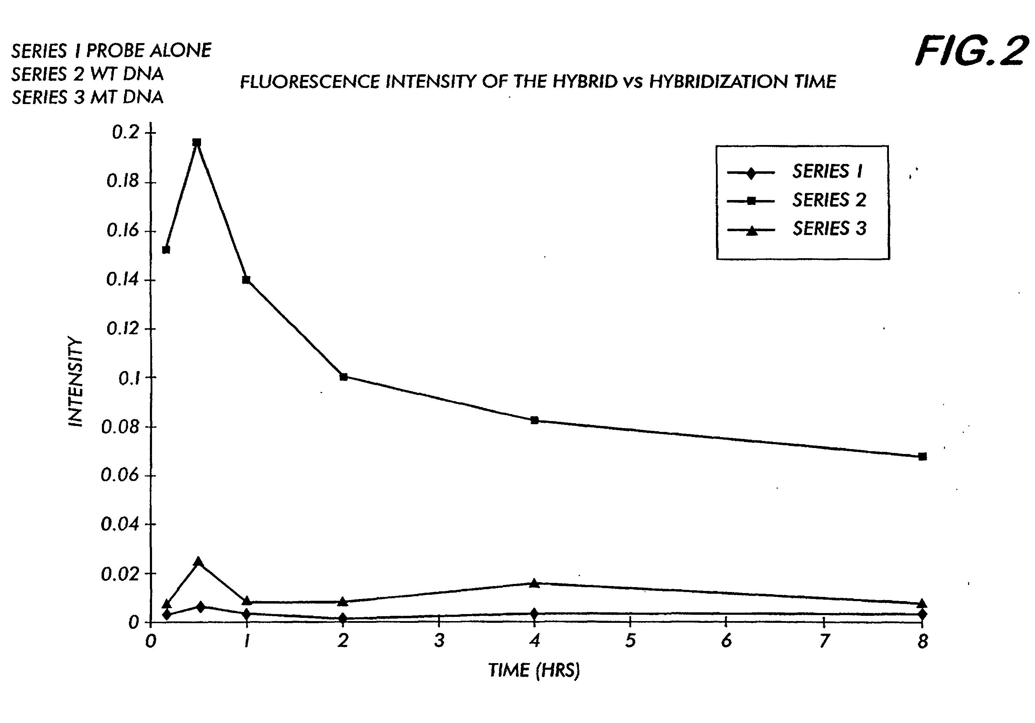

Figure 2 shows DNA hybridization over time. A PNA probe was allowed to hybridize with wild type DNA (WT DNA) and mutant DNA (MT DNA). Hybridization between the probe and the DNA was followed over time by measuring fluorescent intensity. Figure 2 shows that hybridization of the PNA probe with the DNA eventually decreases over time, suggesting that the DNA resumes its native structure over time by displacing PNA.

Clinical samples can be tested using at least 100 times less chemicals or genomic material (100 microliters vs. 10 milliliters) than is typical in conventional methods. Therefore, even using 10 or 20 times the concentration of PNA conventionally used, the tests still only consume 1/5th to 1/10th the amount of PNA , while obtaining a very decisive result.

The invention will be illustrated in more detail with reference to the following Examples, but it should be understood that the present invention is not deemed to be limited thereto.

### EXAMPLE 1

A 150 bp (base pair) fragment of genomic DNA from wild type p53 gene (SEQ ID NO:1) was amplified by PCR. PCR was conducted using a GeneAmp PCR system 2400 from Perkin Elmer, with a hot start at 95°C for 5 minutes. After adding Taq enzyme, 35 cycles were carried out as follows:
- Denaturing:: 94°C for 30 mins.
- Annealing:: 45°C for 45 mins. (45°C is 5°C lower than the primer Tm)
- Extension:: 72°C for 30 mins. (1 kb/min.) x 35.

PCR was conducted using a mixture comprising in 100 ml volume 10 x reaction buffer (10 µl) , 20 mM of MgCl₂ (10 µl) , 10 mM of dNTP mixture (2), 25 pmol/µl of primer 1 (1 µl), 25 pmol/µl of primer 2 (1 µl), 100 µg/µl of DNA template (1 µl), dd H₂O (74 µl) and Taq polymerase (1 µl) (5 unit/µl).

Similarly, a mutant fragment (SEQ ID NO:2) of the same genomic 150 bp fragment was also amplified by PCR. The mutant fragment was identical to the wild type fragment except for a point mutation at amino acid position 344 at which the DNA wild type sequence CTG was changed to CAG.

A 12-mer PNA probe was obtained from PerSeptive Biosystems, Inc. (Framingham, MA, USA) and was designed to be complementary to a 12 nucleotide segment of the 150 bp p53 wild type fragment (SEQ ID NO:1). The probe had the following structure:

The buffer solution for hybridization was 10mM Tris-HCl, pH 7.1, 1% by weight BSA (bovine serum albumin), and 0.1% Triton X-100 (t-octylphenoxypolyethoxyethanol). 50 pmol of wild type genomic DNA was added to 300 pmol of PNA probe and mixed in 100 ml of buffer. 50 pmol of mutant genomic DNA was added to 300 pmol of PNA probe and mixed in 100 microliters of buffer.

Each sample was heated at 95°C for 30 minutes. Each sample was then added to buffer preheated to 30°C and allowed to hybridize for 1 hour.

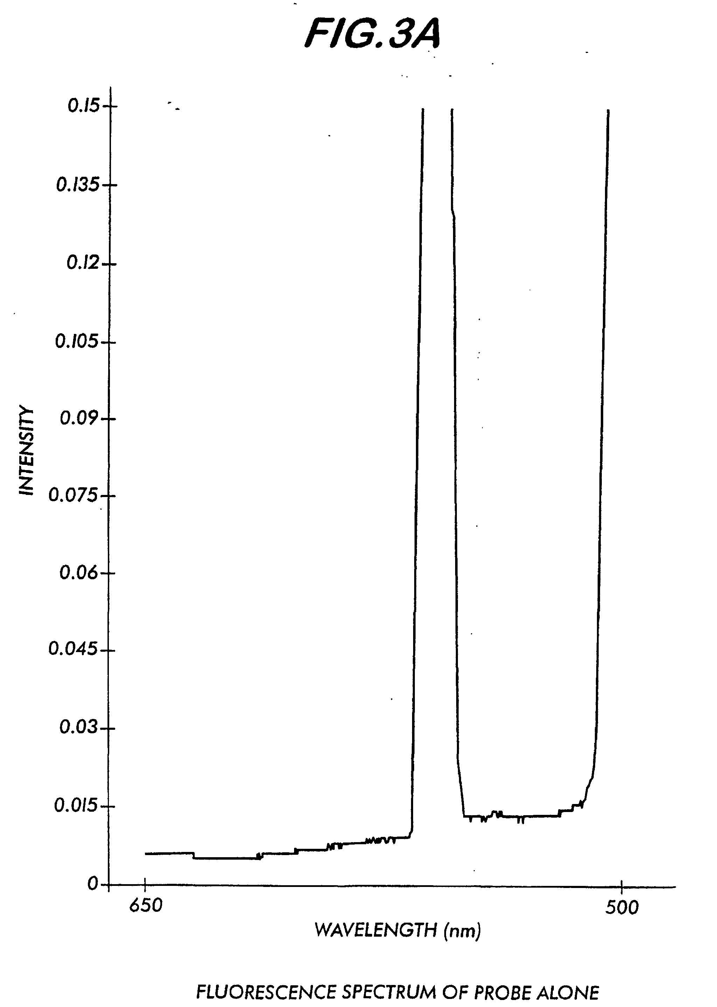

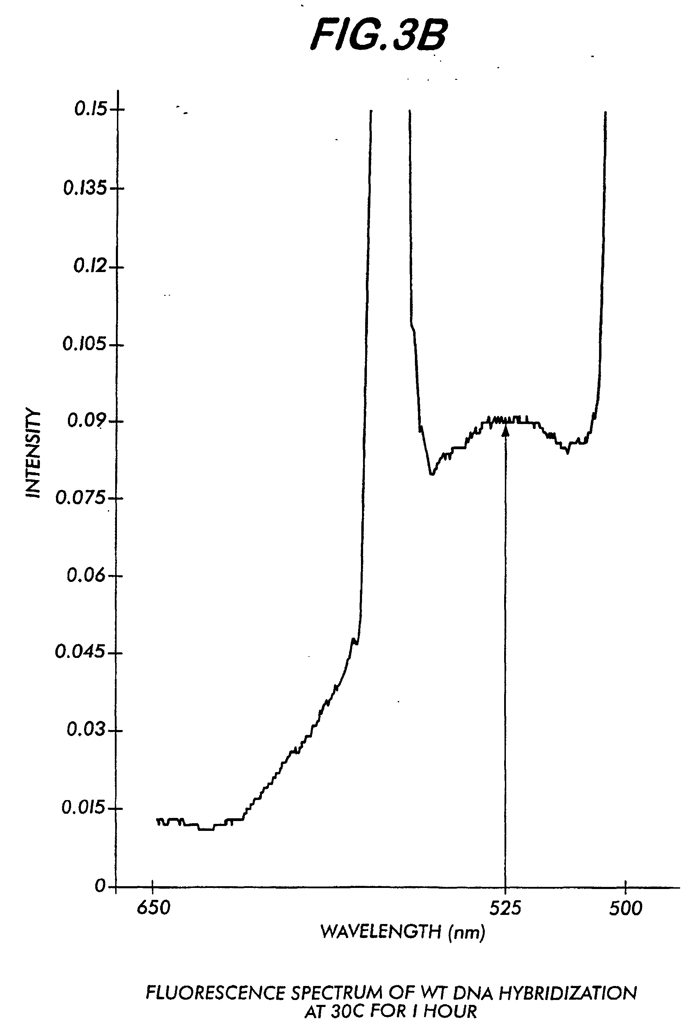

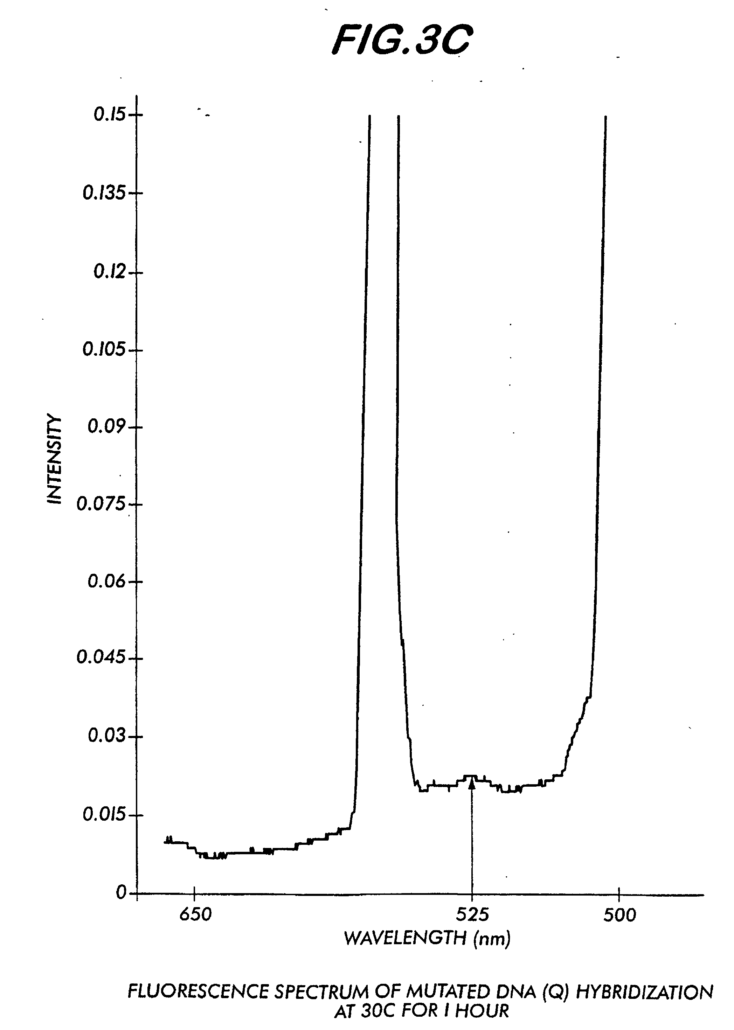

The components of each sample were separated by G50 spin columns (Pharmacia Biotech, Uppsala, Sweden) by spinning at 600g for 2 minutes. The unbound PNA was filtered and retained in the column. The solution with the hybrids of PNA-DNA passed through the column and was collected in a cuvette for fluorescent detection. The cuvette was then placed in a fluorescence spectrometer for laser induced fluorescence (irradiation wavelength of 488 nm in all of the Examples) and detection of the fluorescent probe attached to the target genomic DNA. As shown in Figures 3B and 3C, the relative fluorescence intensity at 525 nm of the wild type genomic DNA sample (Figure 3B) was four times higher than that of the mutant sample (Figure 3C), allowing the mutant genomic DNA sample to be distinguished from the wild type genomic DNA sample. Figure 3A shows the fluorescent intensity of the probe alone.

### EXAMPLE 2

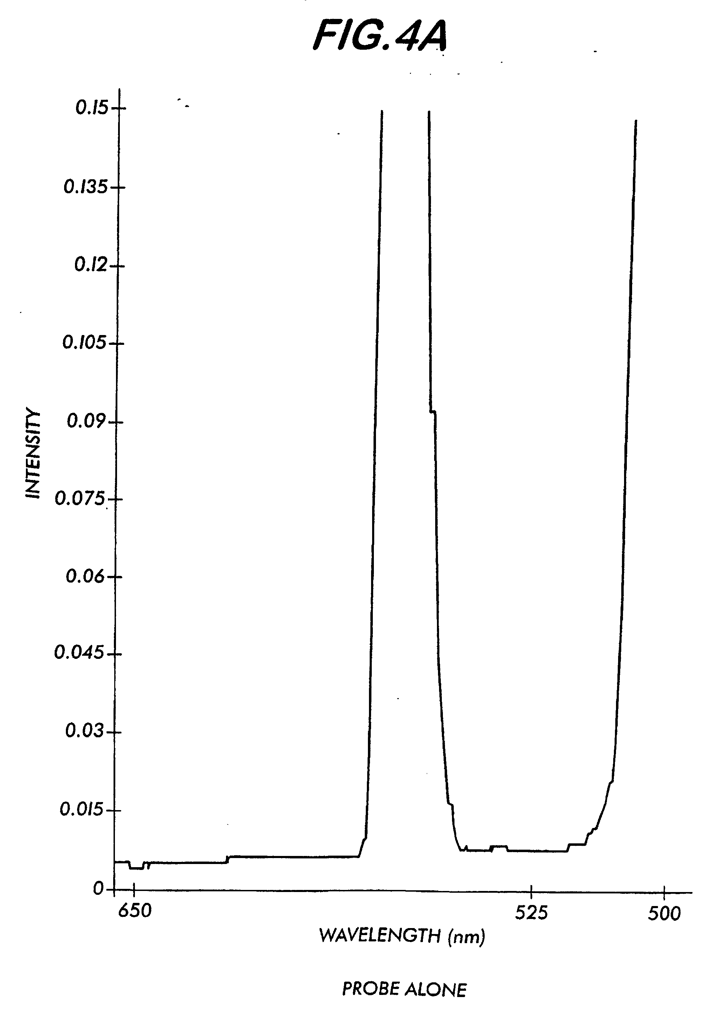

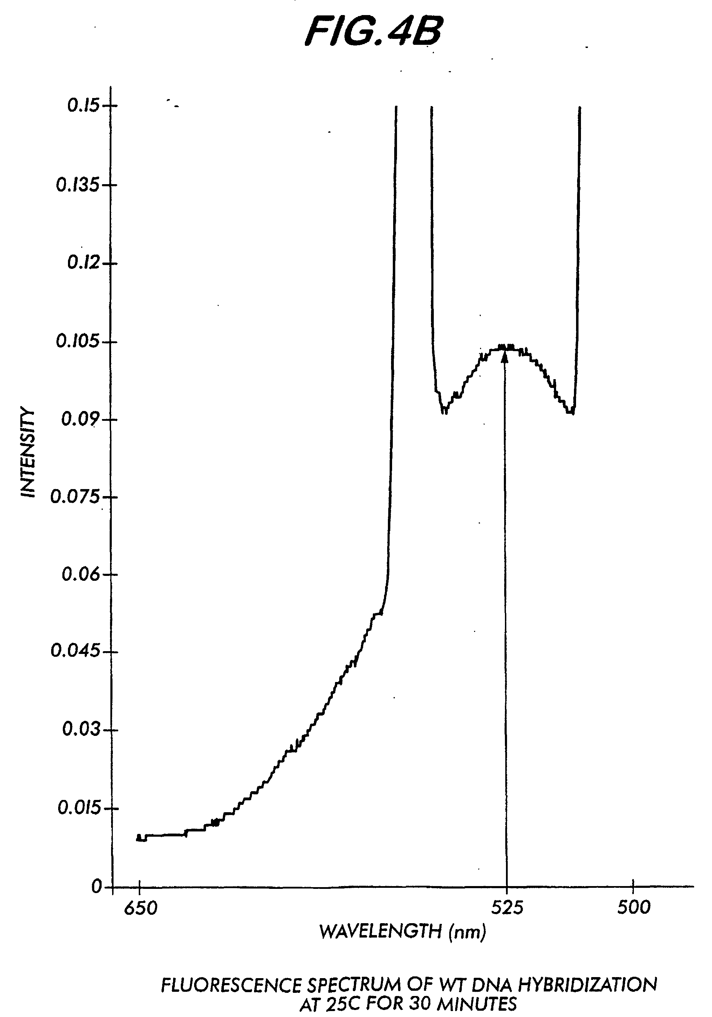

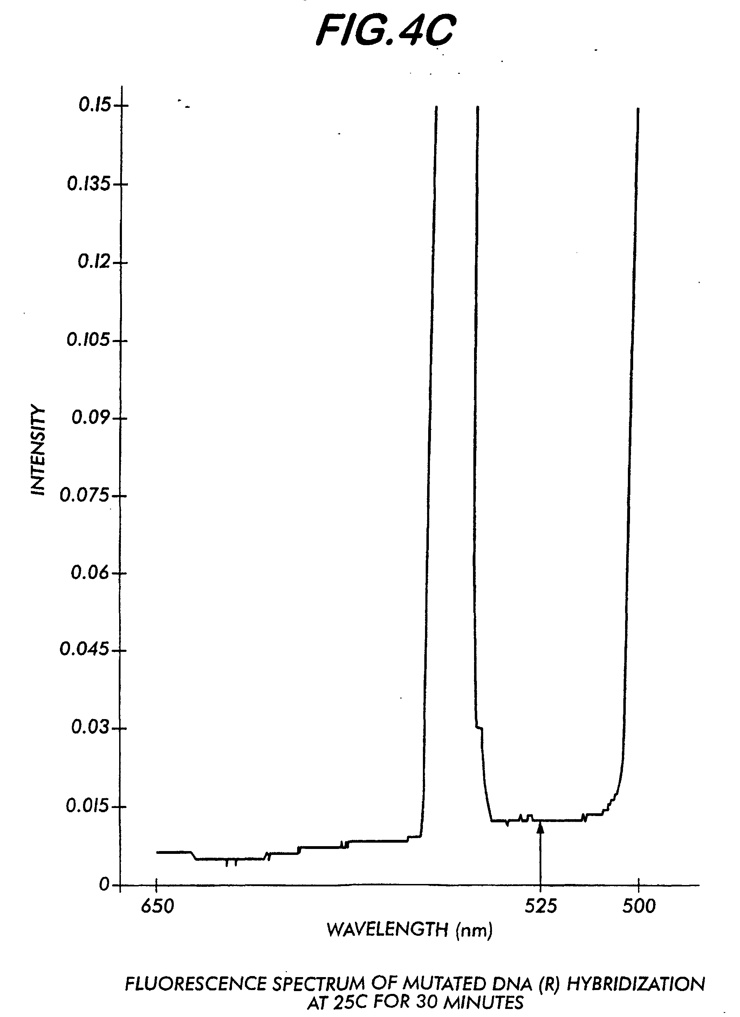

This Example was similar to Example 1 except for the following details. The mutant genomic DNA sample (SEQ ID NO:3) had the base sequence at amino acid position 344 changed from the wild type sequence CTG to CGG. Both samples were heated at 94°C for 20 minutes. The buffer was preheated to 25°C and the samples were allowed to hybrid for 30 minutes. The components of each sample were separated by using G50 spin columns and spinning at 800 rpm for 1.5 minutes. The relative intensity at 525 nm of the detected fluorescence from the wild type genomic DNA sample (Figure 4B) was six times higher than that of the mutated genomic DNA sample (Figure 4C), allowing the wild type genomic sample to be distinguished from the mutated genomic sample. Figure 4A shows the fluorescent intensity of the probe alone in the solution.

### EXAMPLE 3

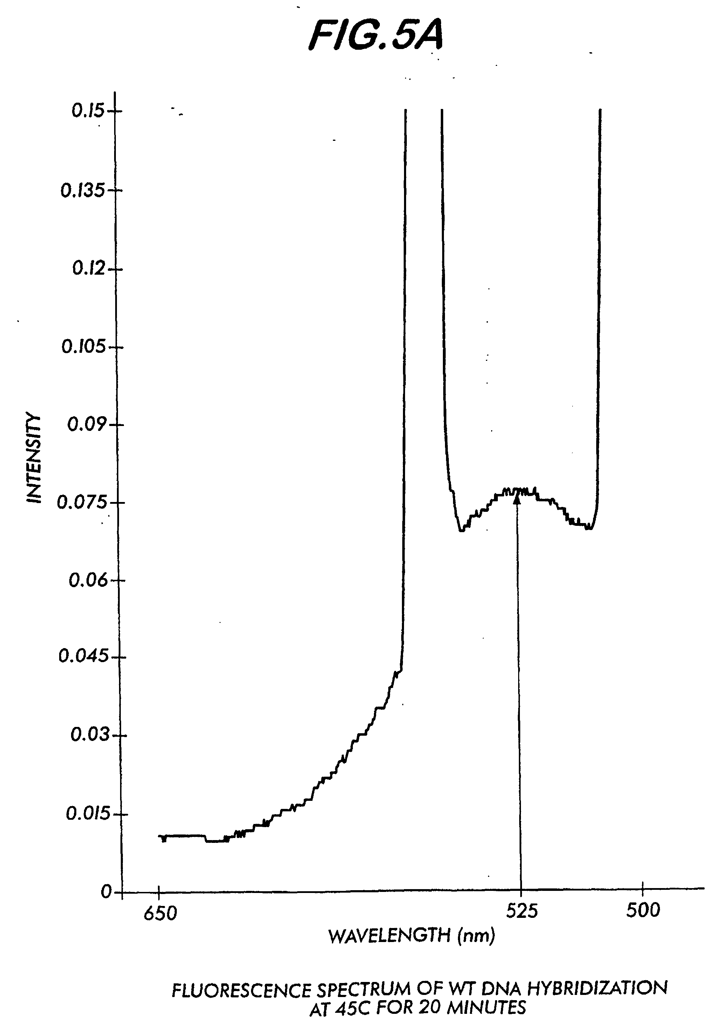

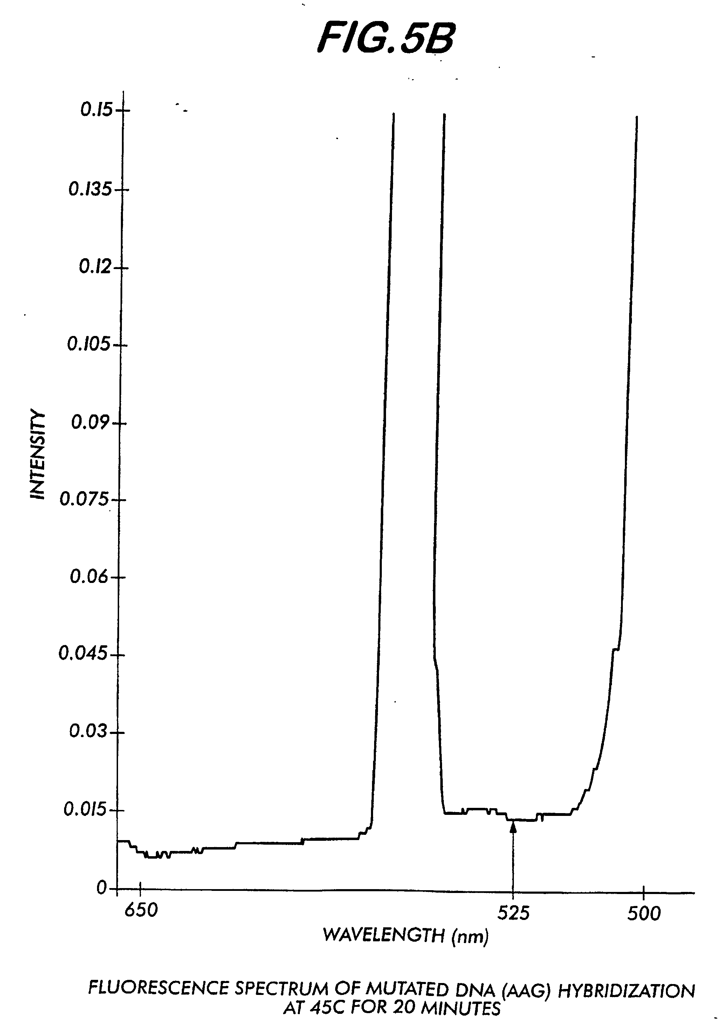

This Example was similar to Examples 1 and 2 except for the following details. The mutant genomic DNA sample (SEQ ID NO:4) had a 2 base change from the wild type genomic DNA at amino acid position 344, from CTG to AAG. Each sample was initially heated at 100°C for 45 minutes. The buffer was preheated to 45°C and each sample was allowed to hybridize for 20 minutes. The components of each sample were separated by using G75 spin columns and spinning at 1000 rpm for 1 minute. The relative intensity at 525 nm of the detached fluorescence from the wild type genomic DNA sample (Figure 5A) was eight times higher than that of the mutated genomic sample (Figure 5B), allowing the wild type genomic sample to be distinguished from the mutated genomic sample.

### EXAMPLE 4

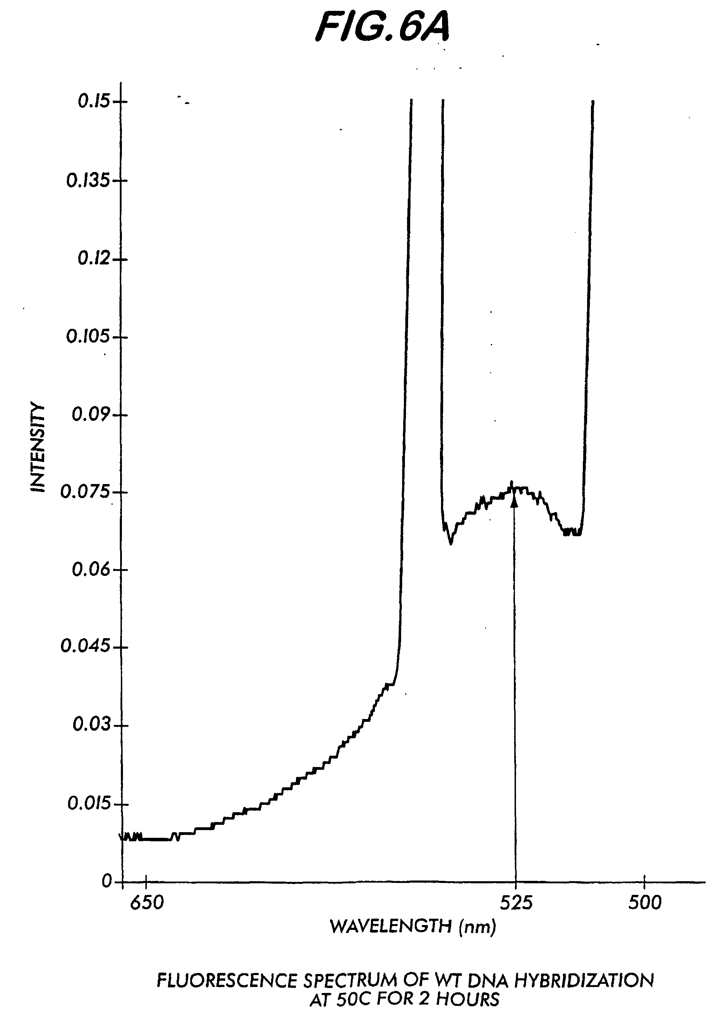

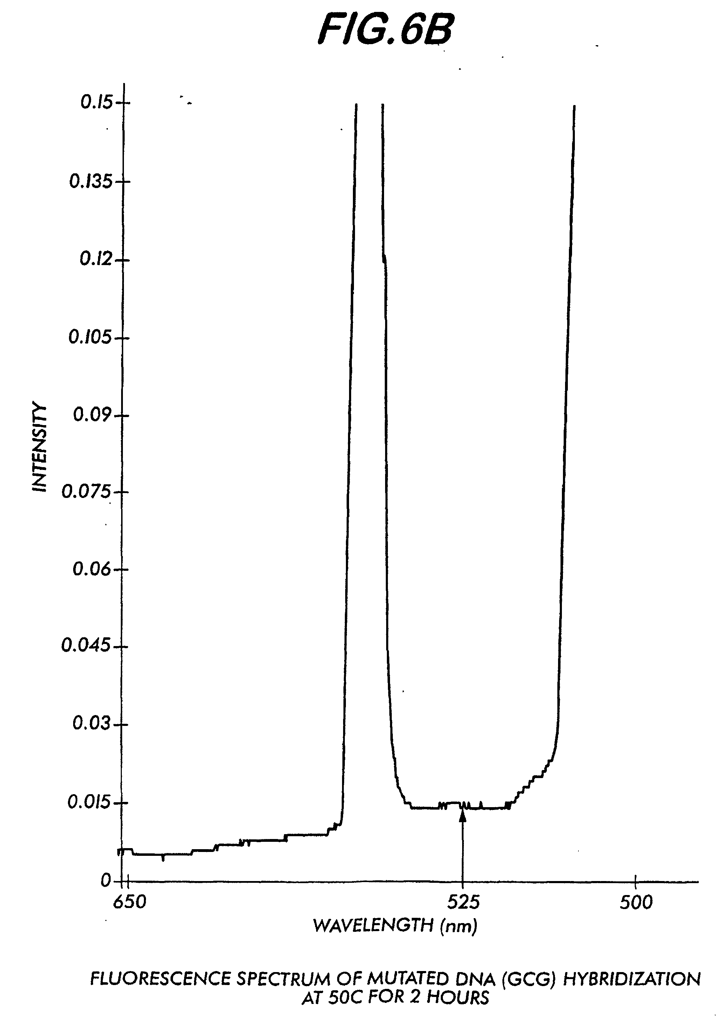

This Example was similar to Example 3 except for the following details. The mutant genomic DNA sample (SEQ ID NO:5) had a 2 base change from the wild type genomic DNA at amino acid position 344, from CTG to GCG. Each sample was initially heated at 100°C for 15 minutes. The buffer was preheated 50°C and each sample was allowed to hybridize for 2 hours. The components of each sample were separated using G50 spin columns and spinning at 1200 rpm for 1 minute. The relative intensity at 525 nm of the detected fluorescence from the wild type genomic DNA sample was seven times higher than that from the mutated genomic sample, allowing the wild type genomic sample (Figure 6A) to be distinguished from the mutated genomic sample (Figure 6B).

### EXAMPLE 5

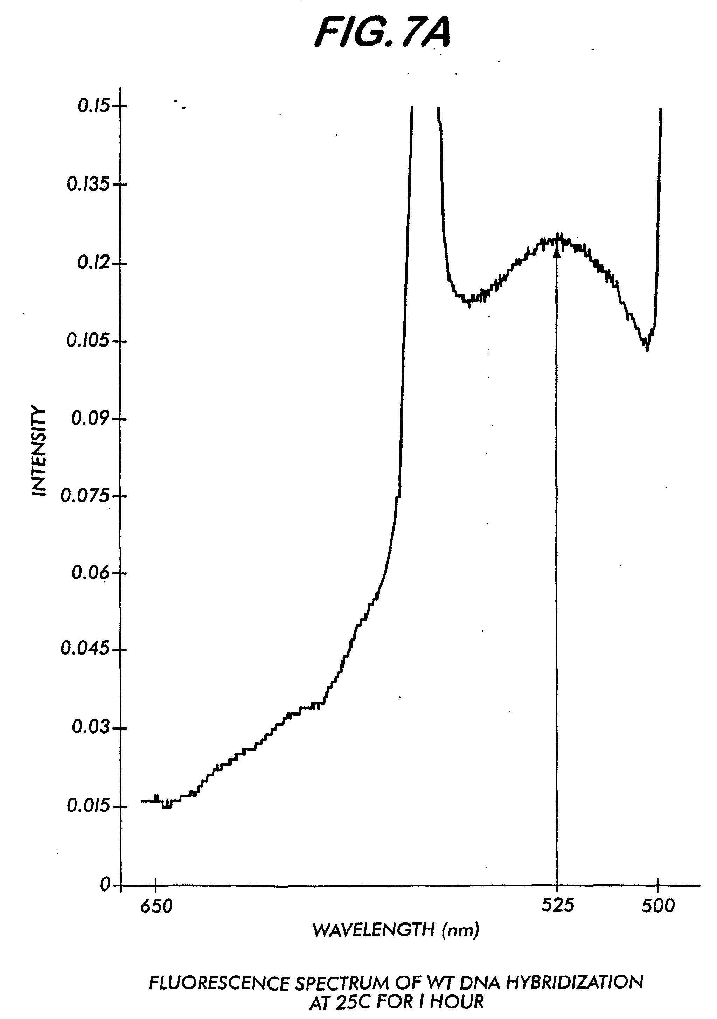

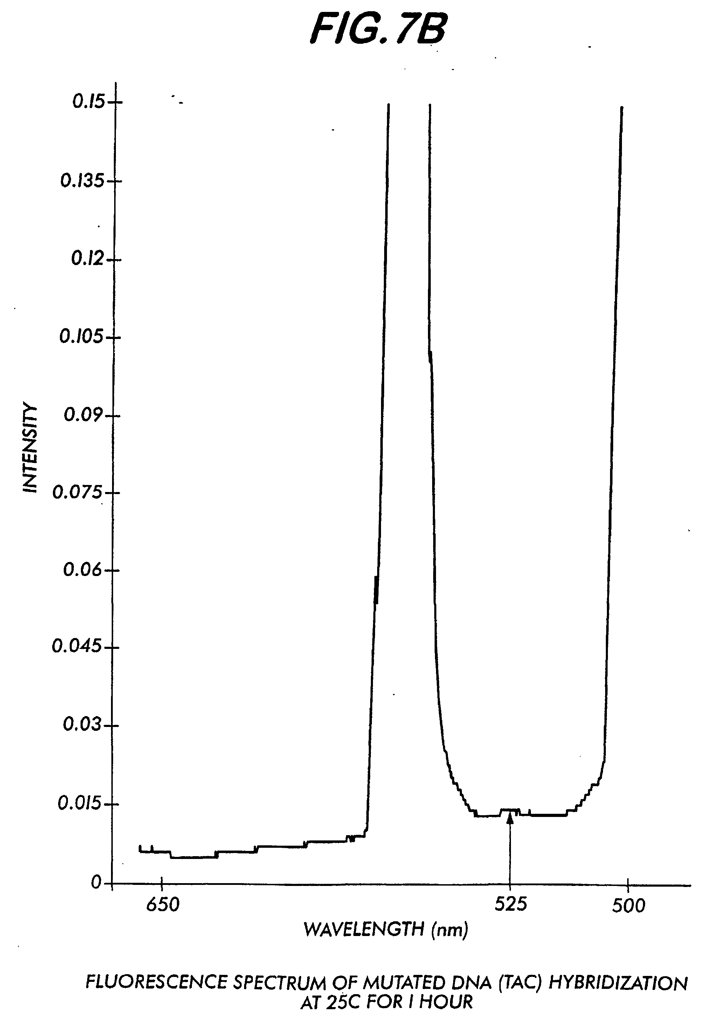

This Example was similar to the other Examples except for the following details. The mutant genomic DNA sample (SEQ ID NO:6) had a 3 base change from the wild type genomic DNA at amino acid position 344, from CTG to TAC. Each sample was initially heated at 95°C for 30 minutes. The buffer was preheated to 25°C and each sample was allowed to hybridize for 60 minutes. The components of each sample were separated using G50 spin columns and spinning at 750 rpm for 2 minutes. As shown in Figures 7A and 7B, the relative intensity at 525 nm of the detected fluorescence from the wild type genomic DNA sample (Figure 7A) was ten times higher than that from the mutated genomic sample (Figure 7B), allowing the wild type genomic sample to be distinguished from the mutated genomic sample.

### EXAMPLE 6

This Example was similar to Example 1 except for the following details. 100 pmol of the 12 bp PNA of Example 1 labeled with fluorescein (PerSeptive Biosystems), 25 pmol of 150 bp PCR amplified dsDNA, including wild type (CTG) (SEQ ID NO:1) and one base mutated DNA (CAG, SEQ ID NO:2, and CGG, SEQ ID NO:3) and 115 microliters buffer (0.5xTBE working solution (0.0225M Trisborate/0.0005M EDTA), pH 6.5) were mixed at room temperature. The mixture was then heated for 30 minutes at 95°C and hybridized at room temperature for about 1 hour.

Two metal wires serving as electrodes were placed into each sample. Fluorescent intensity was monitored at 525 nm while a voltage of 5 volts at a current of 10mA was switched on and off, with an electrode separation of about 7 or 8 mm. When the voltage was applied, the DNA and DNA-PNA migrated to the anode, since the DNA is negative charged, while the unbound PNA did not migrate, since it is neutral.

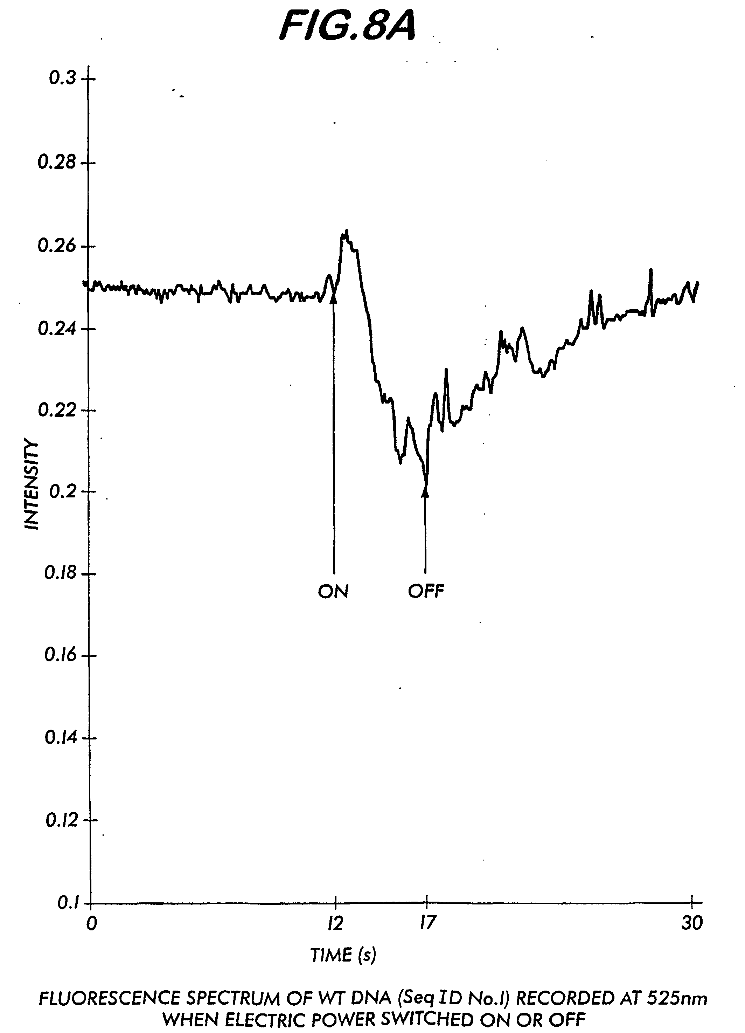

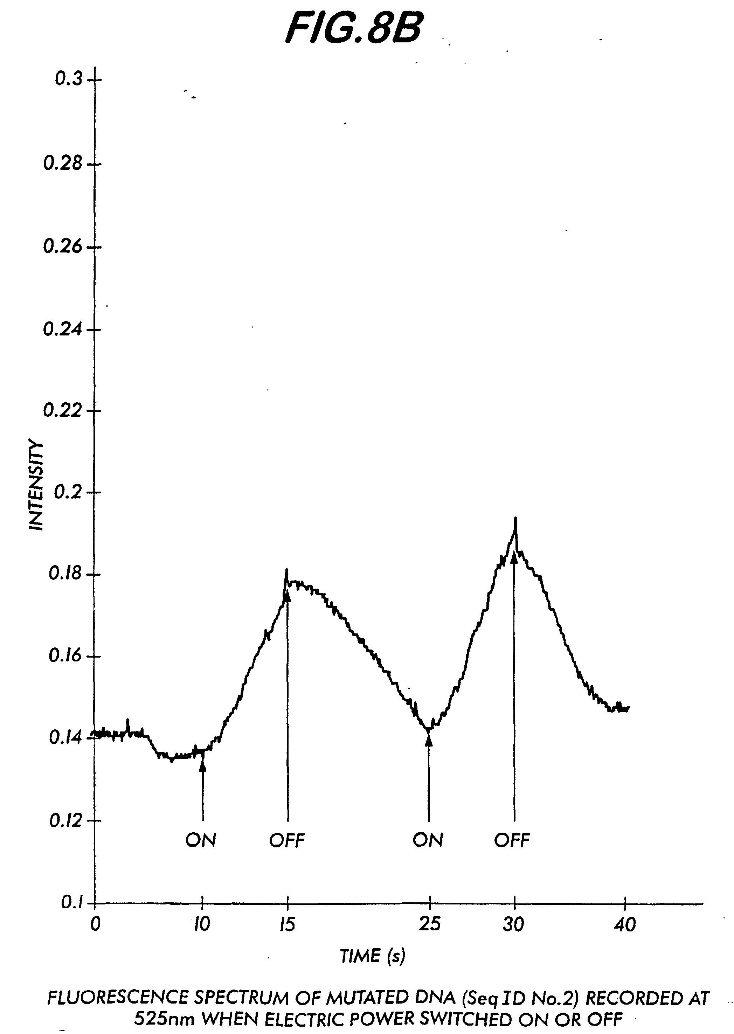

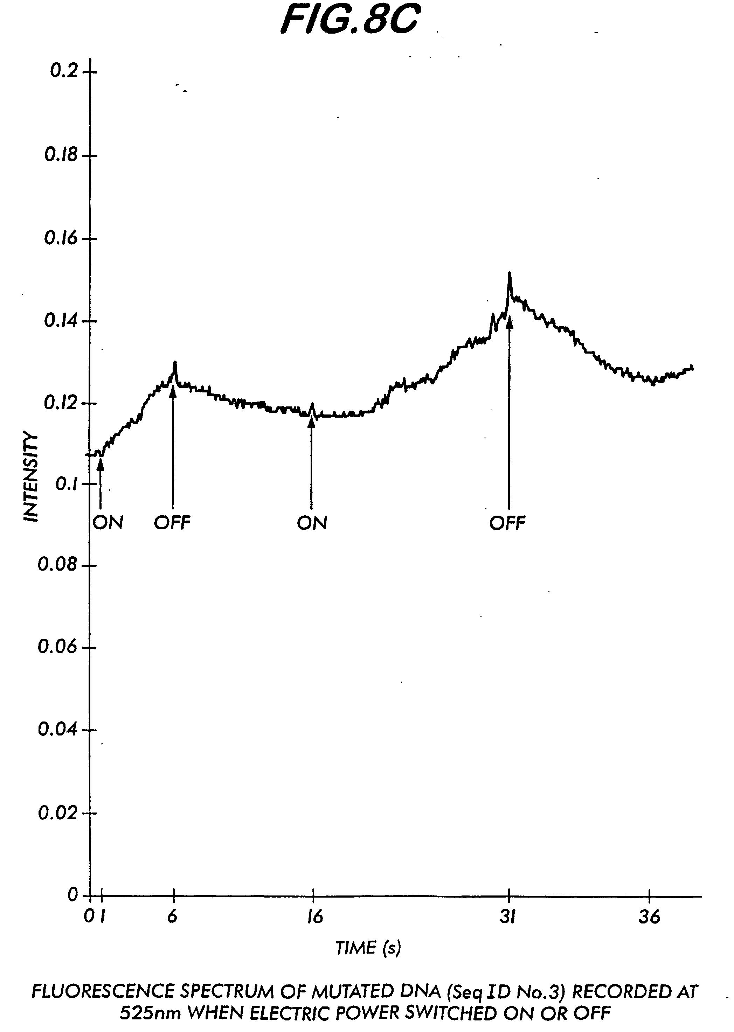

Figure 8A shows the fluorescent spectrum of the wild type DNA. After hybridizing at 25°C for 1 hour, the solution was transferred into a cuvette. The cuvette was placed in a fluorescence spectrometer and then two electrodes were put into the solution for detecting the change of fluorescent intensity at a wavelength of 525 nm with the applied electric field. The fluorescent intensity at 525 nm decreased with the application of the voltage and increased with the removal of the voltage. Figures 8B and 8C show the fluorescent spectra of one base pair mismatched DNA (SEQ ID NO: 2 and SEQ ID NO:3, respectively). After hybridizing at 25°C for 1 hour, the fluorescent spectra of -mutant DNA showed a difference from the spectrum of wild type DNA. When voltage was applied, the intensity quickly increased, and when voltage was removed, the intensity decreased.

### EXAMPLE 7

This Example was similar to Example 1 except for the following details. A 375 bp fragment of p53 wild type DNA (SEQ ID NO:7), a corresponding 375 bp mutant type DNA fragment (SEQ ID NO:8), a corresponding 375 bp mutant type DNA fragment (SEQ ID NO:9) and a 633 bp DNA fragment (SEQ ID NO:10) were obtained by PCR. The 375 bp wild type DNA contained a target sequence of DNA complementary to the 12 base PNA from Example 1. The 633 bp DNA lacked such a target sequence and was used as a negative control in this example. SEQ ID NO:8 was identical to the wild type fragment (SEQ ID NO:7) except for a point mutation at amino acid position 344 at which the DNA wild type sequence CTG was changed to CAG. SEQ ID NO:9 was identical to the wild type fragment (SEQ ID NO:7) except for a point mutation at amino acid position 344 at which the DNA wild type sequence CTG was changed to CGG.

Each sample comprised 50 pmol of the respective DNA fragment, 200 pmol of PNA probe and 130 ml of buffer (0.5xTBE at pH 6.5). Each sample was heated at 95°C for about 30 minutes. Each sample was then allowed to hybridize for 1 hour under ambient conditions (25°C). Before fluorescence measurement, unbound probe was filtered from solution using a G50 column.

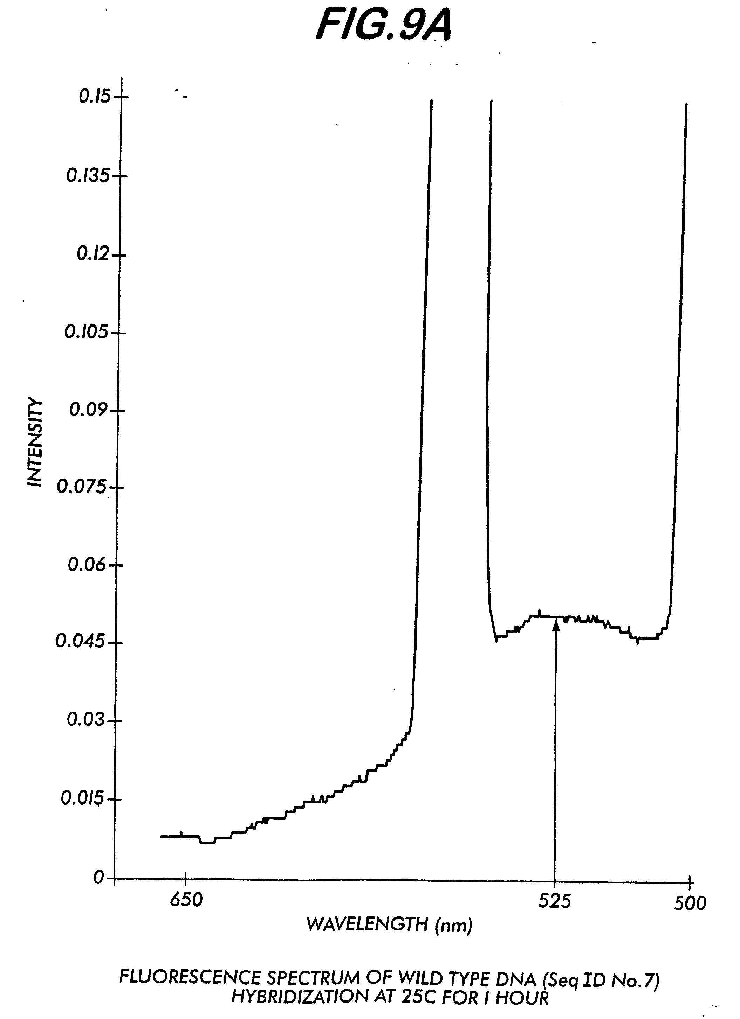

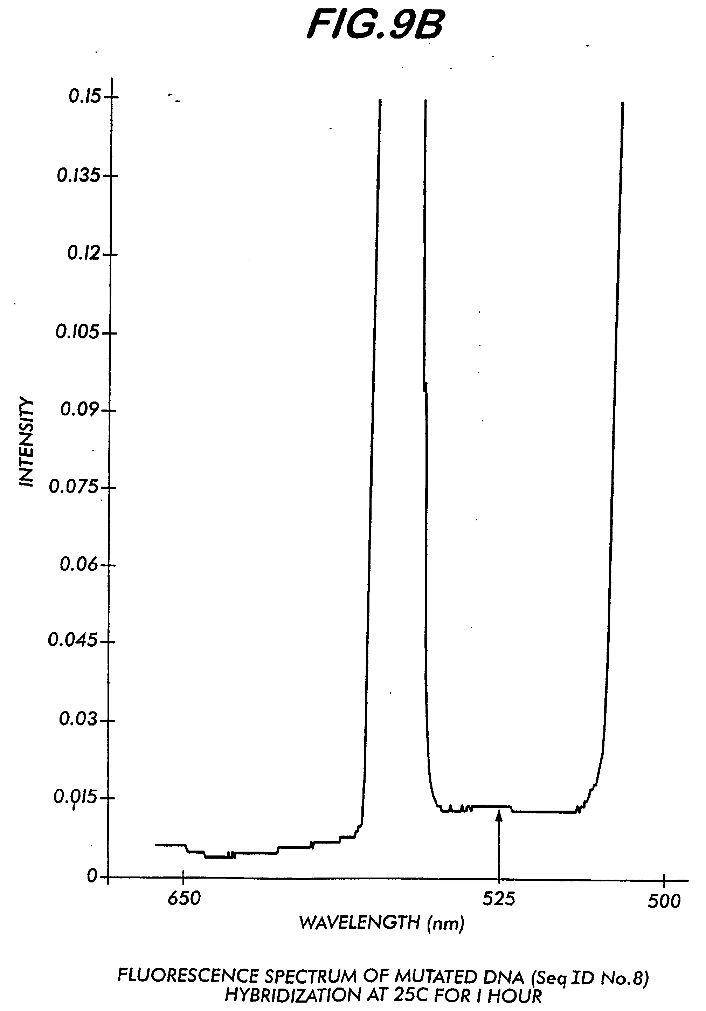

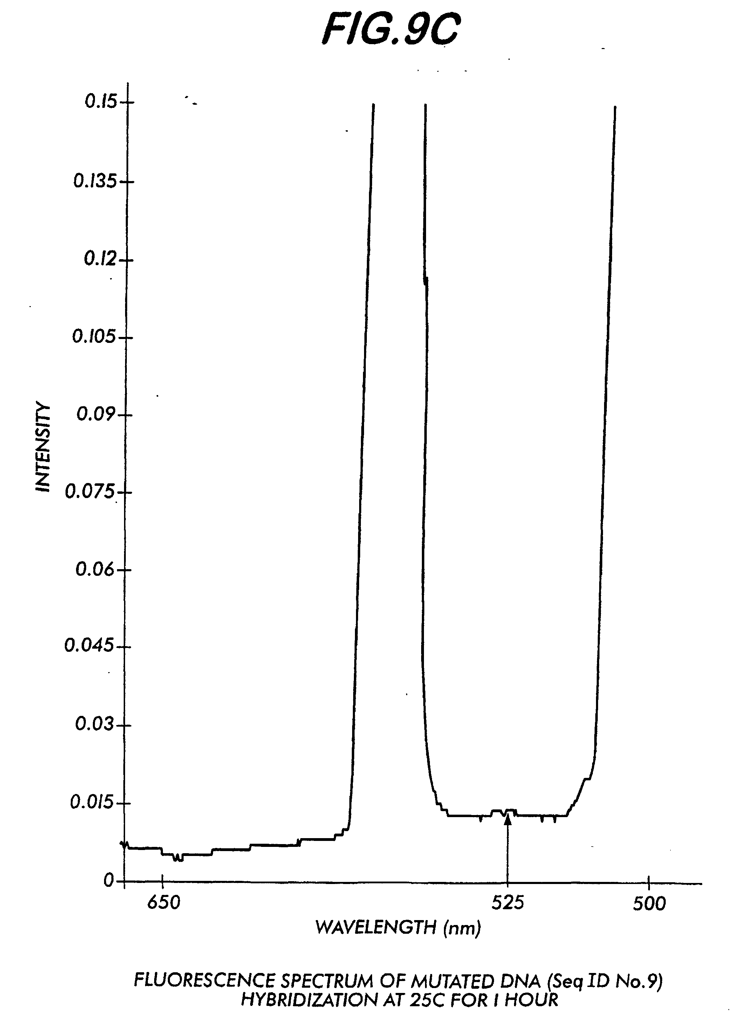

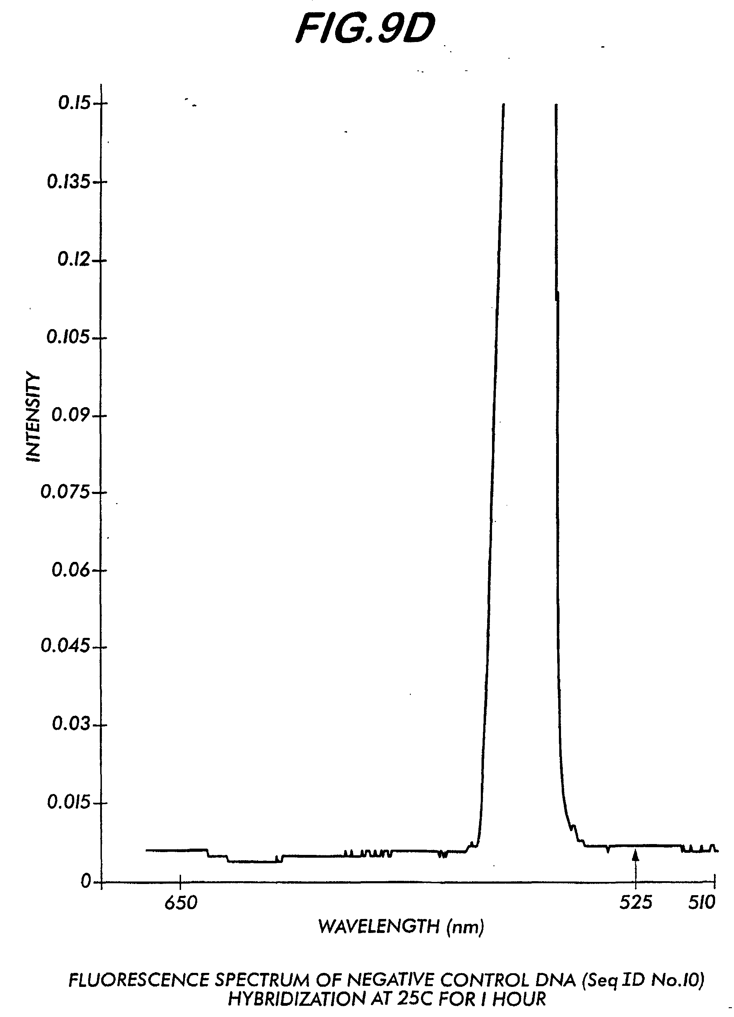

Each sample was placed in a fluorescence spectrometer for detection of the fluorescent probe attached to the target genomic DNA. As with the previous examples, the relative fluorescence intensity at 525 nm was highest for samples containing DNA fragments completely complementary to the probe, thus allowing the samples containing mutant DNA fragments (Figures 9B-9C) to be distinguished from the sample containing wild type DNA fragments (Figure 9A). As expected, the negative control DNA fragment- showed the lowest fluorescent intensity of all (Figure 9D).

### EXAMPLE 8

A clinical sample is obtained from an individual suspected of suffering from a disease which is known to be caused by a single nucleotide mutation within a known segment of the human genome. A fragment of this gene segment is amplified by PCR using a primer capable of amplifying the fragment regardless of whether it contains wild type DNA or mutant type DNA.

A PNA probe of 12 bases is provided which is complementary to a portion of the mutant type DNA fragment containing the mutant nucleotide, wherein the mutant nucleotide has a base complementary to a centrally located base of the probe. The probe is marked with fluorescein at its 5' end.

50 pmol of the DNA obtained from PCR and 300 pmol of PNA probe are added to 100 ml of buffer solution. The sample is heated at 95°C for 30 minutes. The sample is then added to buffer preheated to 30°C and allowed to hybridize for 1 hour.

The components of the sample are separated by G50 spin columns by spinning at 600 rpm for 2 minutes. The unhybridized PNA is filtered with a column, and the liquid medium containing hybrids and DNA passes through the column and is collected in a cuvette.

The cuvette is placed in a fluorescence spectrometer for detection of the fluorescent probe attached to the DNA. If the sample's fluorescent intensity at 525 nm exceeds a predetermined intensity value, then the mutant type DNA has been detected in the individual. If the sample's fluorescent intensity at 525 nm does not exceed a predetermined intensity value, then the mutant type DNA has not been detected in the individual. The predetermined intensity value is previously determined by calibrating the spectrometer using negative and positive control samples.

### EXAMPLE 9

This Example is similar to Example 8, except for the following details. Two unlabeled DNA (one is wild type (SEQ ID NO:1), another is mutated type (SEQ ID NO:3)) need to be identified. A probe, which is complementary to the mutated DNA (SEQ ID NO:3), with the following sequence 5'CAT TCC GCT CTC (synthesized by PerSeptive Biosystems) was used to identify the two unknown DNA.

Each sample comprising 100 pmol probe, 25 pmol DNA (WT DNA or mutated DNA) and 115 µl buffer (0.5xTBE, pH 6.5) was heated at 95°C for 30 minutes. Each sample was then allowed to hybridize for 1 hour at 25°C. Before fluorescent measurement, each sample was transferred into a G50 column and centrifuged at 750 rpm for 2 minutes. The hybrid flowed through the column and was collected in a cuvette, while the unhybridized probe was filtered and retained in the column. The cuvette was placed into a fluorescent spectrometer for fluorescence measurement.

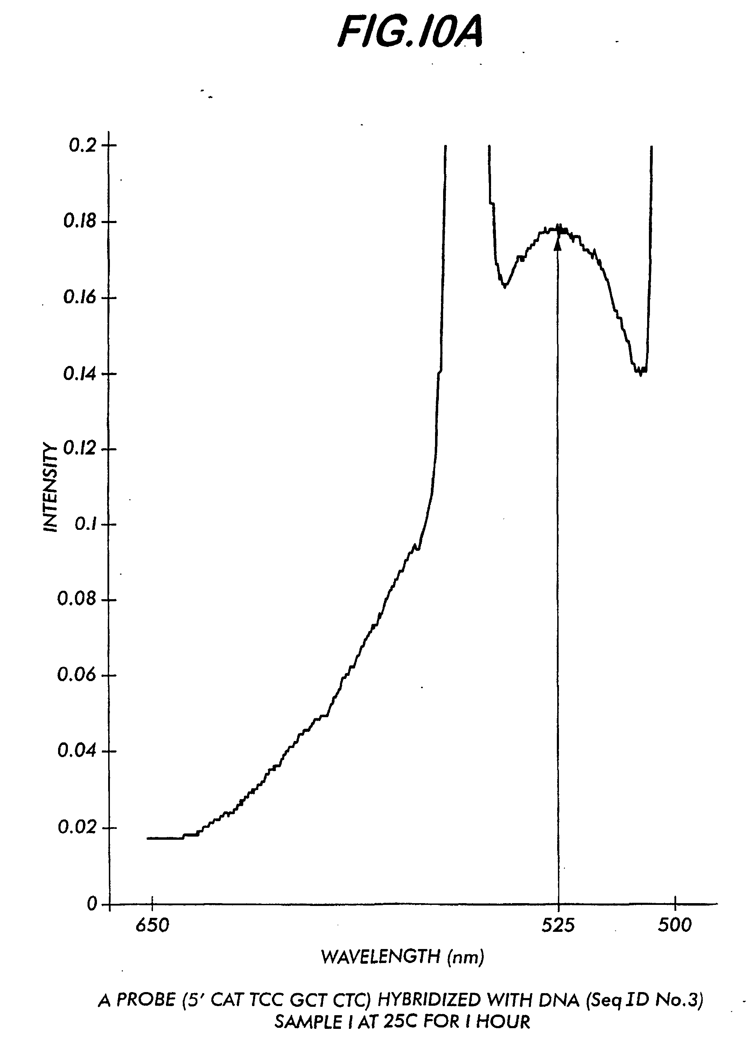

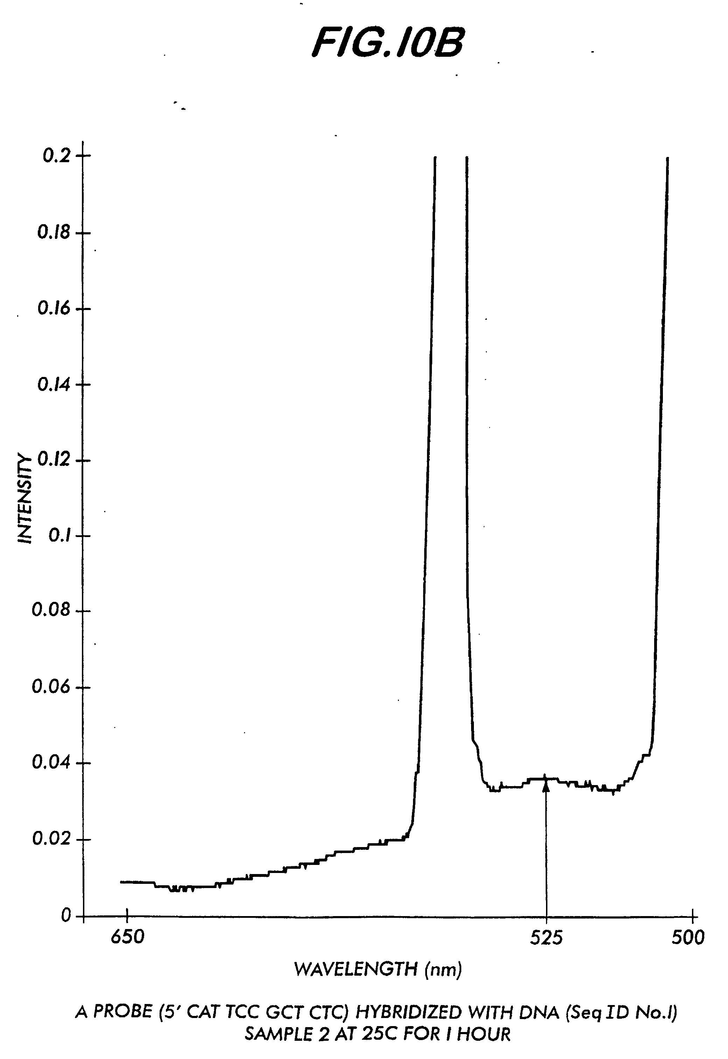

As shown in Figures 10A and 10B, the relative fluorescent intensity at 525 nm shows a large difference in spectra. The sample with a stronger fluorescent signal (Figure 10A) is identified as the mutant DNA (SEQ ID NO:3). The other sample with weaker fluorescent intensity is wild type DNA (SEQ ID NO:1).

### EXAMPLE 10

This Example is similar to Example 8 except for the following details. Rather than using a single probe as in Example 8, two probes are used in this example. the first probe is the probe of Example 8, which is completely complementary to a portion of the mutant type DNA fragment. The second probe is identical to the first probe, except that it has the wild type nucleobase sequence instead of the mutant type nucleobase sequence. A first portion of the sample is probed in accordance with Example 8 using the first probe. A second portion equivalent in size to the first portion is similarly probed using the second probe. After denaturation, hybridization and separation, the fluorescence of each portion is measured. If the fluorescent intensity of the first portion exceeds that of the second portion, then the mutant type DNA has been detected in the individual. If the fluorescent intensity of the second portion exceeds that of the first portion, then the mutant type DNA has not been detected in the individual.

The method of this example could be adapted for use with a diagnostic device comprising a solid support having the two contrasting type of PNA probes bonded to different areas of the support. The sample is not divided, but rather is uniformly dispersed onto the support so as to contact both probe types equally. The fluorescent intensity of the two areas of the device could be compared in the same way that the two separate samples are in Example 9.

### EXAMPLE 11

Biotinylated double stranded DNA (SEQ ID NO:1 and SEQ ID NO:3) was amplified by PCR. Single stranded DNA was prepared using Dynabeads (from DYNAL company) as follows. 25 µl of 2x binding and washing buffer (from M-280 streptativin kit) and 25 µl (25 pmol) of biotinylated DNA (SEQ NO:1 and NO:2) were added into 2mg of the prewashed dynabeads of M-280 streptativin. The mixture was incubated at room temperature for 15 minutes, keeping the beads suspended. The mixture was then placed onto a magnetic particle concentrator (MPC, Dynal, Inc.) at room temperature for 2 minutes, followed by removal of the supernatant with a pipette. 20 µl of freshly made 0.1N NaOH was added and the mixture was kept at room temperature for 5 minutes. The dynabeads were collected with the immobilized biotinylated strand on the side of the tube by using the MPC, and the NaOH supernatant was transferred with the non-biotinylated single stranded DNA to a clean tube. The NaOH supernatant was neutralized with 2.5 µl of freshly made 0.2N HCl.

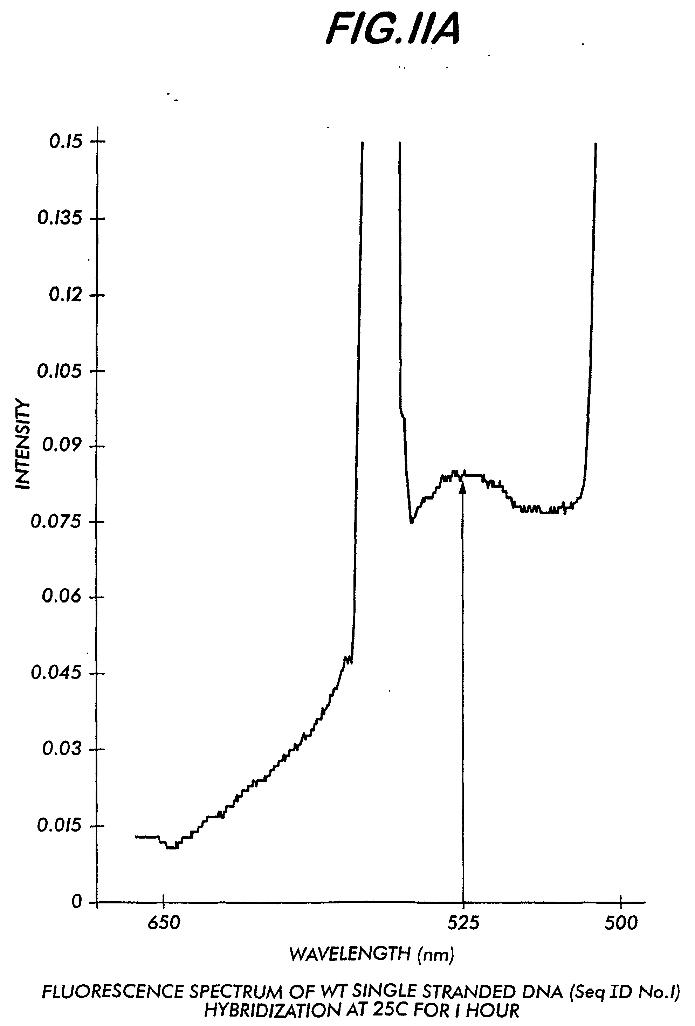

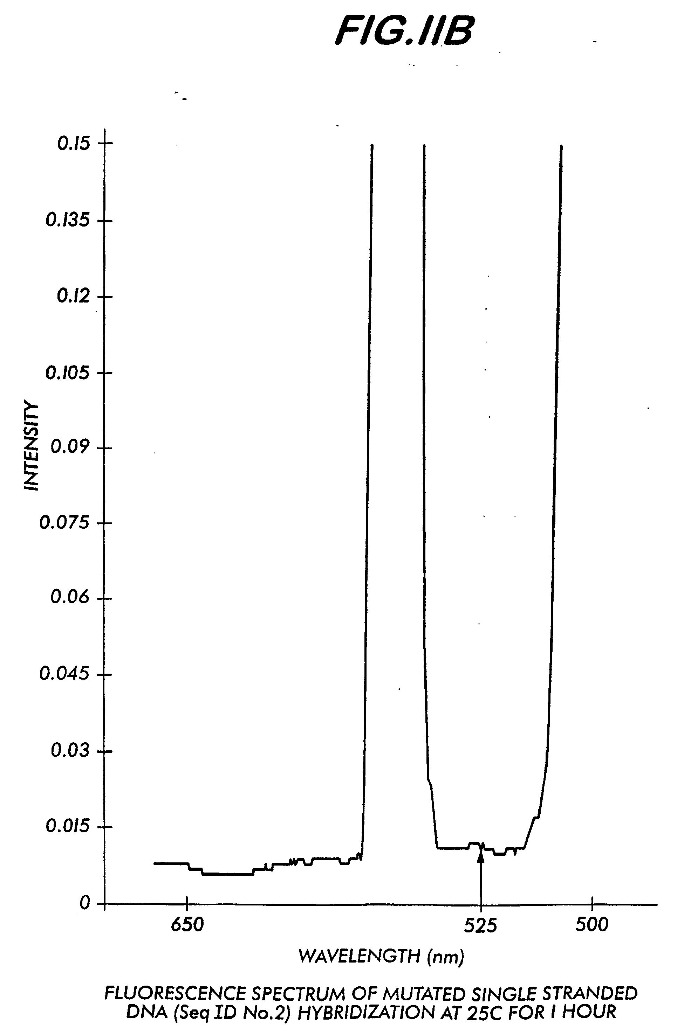

200 pmol of probe and 150 µl of 0.5xTBE buffer were added into the supernatant containing a single stranded DNA. The mixture was heated at 94°C for 5 minutes and then incubated at room temperature for 1 hour. After separation by G50 column at 650 rpm for 2 minutes, the unbound PNA was filtered in the column and the hybrid passed through the column and collected in a cuvette. The sample was placed in a fluorescent spectrometer for fluorescence detection. As shown in Figures 11A and 11B, the relative fluorescent intensity at 525 nm of single stranded wild type DNA sample (Figure 11A) was five times higher than that of mutant single stranded DNA (Figure 11B).

### EXAMPLE 12

Each sample comprising 400 pmol probe, 100 pmol DNA (SEQ ID NO.:1) and 100 µl buffer (0.5xTBE, pH 6.5) was heated at 95°C for 30 minutes. Each sample was then allowed to hybridize for 1 hour at 25°C. Before fluorescent measurement, each sample was transferred into a G50 column and centrifuged at 720 rpm for 2 minutes. The hybrids flowed through the column and were collected in a cuvette, while the unhybridized probe was filtered and retained in the column. The cuvette was placed into a fluorescent spectrometer for fluorescence measurement.

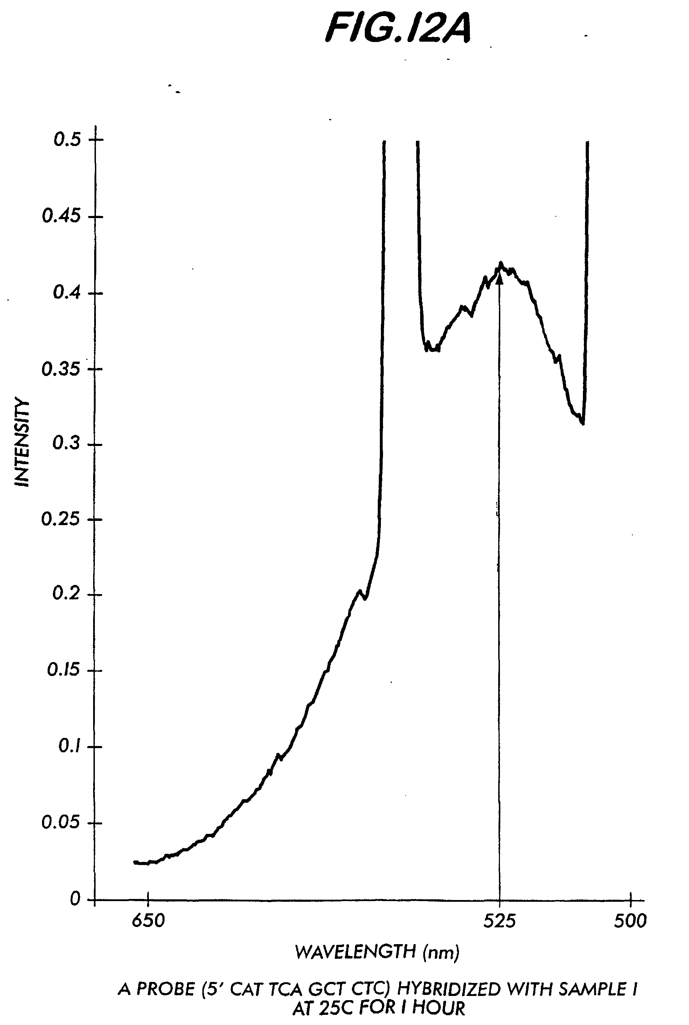

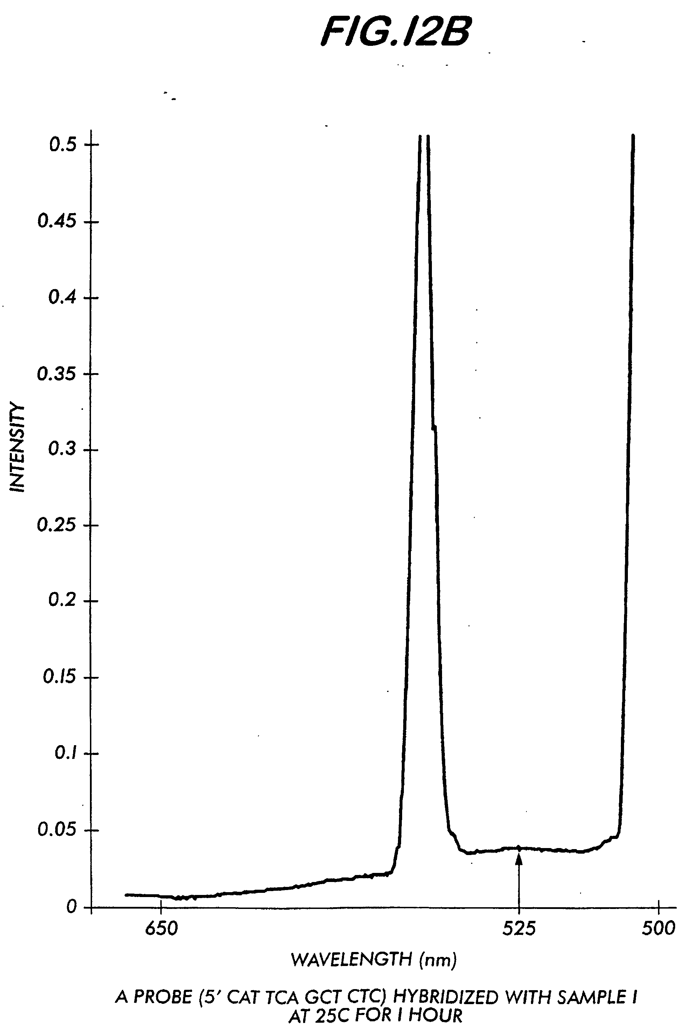

As shown in Figures 12A and 12B, the relative fluorescence intensity at 525 nm shows a large difference in the spectra. The spectrum with a stronger fluorescent signal (Figure 12A) shows a perfect matched probe hybridization with WT DNA (SEQ ID NO:1). The other spectrum with weaker fluorescent intensity (Figure 12B) shows a one bp mismatched probe hybridization with WT DNA (SEQ ID NO:1).

### EXAMPLE 13

Two PNA probes having the same dye (fluorescein) hybridized with two perfectly matched targets in different strands.

### Example 13a: One probe perfectly matching one target sequence.

A 150 bp fragment of genomic DNA from mutated type p53 DNA (SEQ ID NO:11) was amplified by PCR and purified using the QIAquick PCR Purification Kit (QIAGEN Inc., Chatsworth, CA, USA). The mutated fragment was identical to the wild type fragment (SEQ ID NO:1) except for a two base mutation at amino acid position 340 (i.e., bases 88-90) at which the DNA wild type sequence ATG was changed to GAG.

A 12-mer PNA probe (Probe No. 1) was synthesized by PerSeptive Biosystems, Inc. of Framingham, MA, USA. The probe, having the structure: was designed to be complementary to a 12 nucleotide segment of the 150 bp p53 ME DNA, starting at base pair position 95 and ending at base pair position 106 (see SEQ ID NO:11).

0.5xTBE solution (0.0225 M Tris-borate/0.0005 M EDTA, pH 6.5) was used as hybridization buffer. 5 pmol of DNA (SEQ ID NO:11) was added into 115 µl 0.5xTBE buffer. Then 2.5 pmol of Probe No. 1 was added into the solution. The sample was heated at 95°C for 10 minutes and hybridized at 25°C for 30 minutes.

Before fluorescence measurement, the unbound probe was filtered from the solution by G50 spin column (purchased from Pharmacia Biotech, AB, Uppsala, Sweden). The solution with the hybrids of PNA-DNA was placed into a cuvette and subjected to fluorescence measurement.

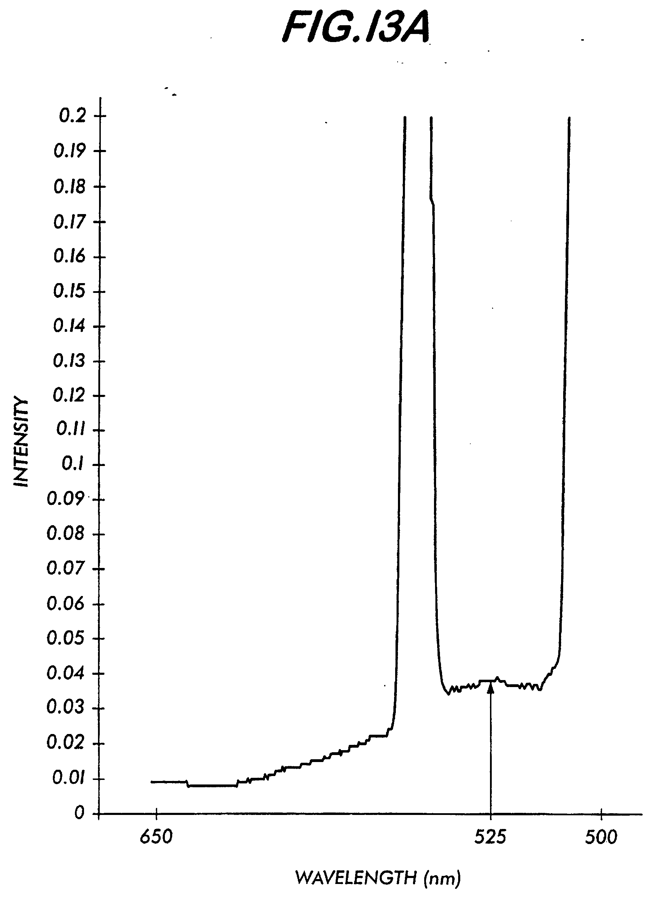

Fig. 13A shows the fluorescence spectrum of 5 pmol of DNA (SEQ ID NO:11) hybridized with 2.5 pmol of Probe No.1. A peak appears at 525 nm.

### Example 13b: One probe perfectly matching one target sequence.

A 12-mer PNA probe (Probe No. 2) synthesized by PerSeptive Biosystems, Inc., having the structure: was prepared to be completely complementary to a target in the strand of ME DNA complementary to the strand targeted in Example 13a, starting at base pair position 83 and ending at base pair position 94 (see SEQ ID NO:11).

5 pmol of DNA (SEQ ID NO:11) was added into 115 µl 0.5xTBE buffer. Then the probe was added into the solution. The sample was heated at 95°C for 10 minutes and hybridized at 25°C for 30 minutes.

Before fluorescence measurement, the unbound probe was filtered from the solution by G50 spin column. The solution with the hybrids of PNA-DNA was placed into a cuvette and subjected to fluorescence measurement.

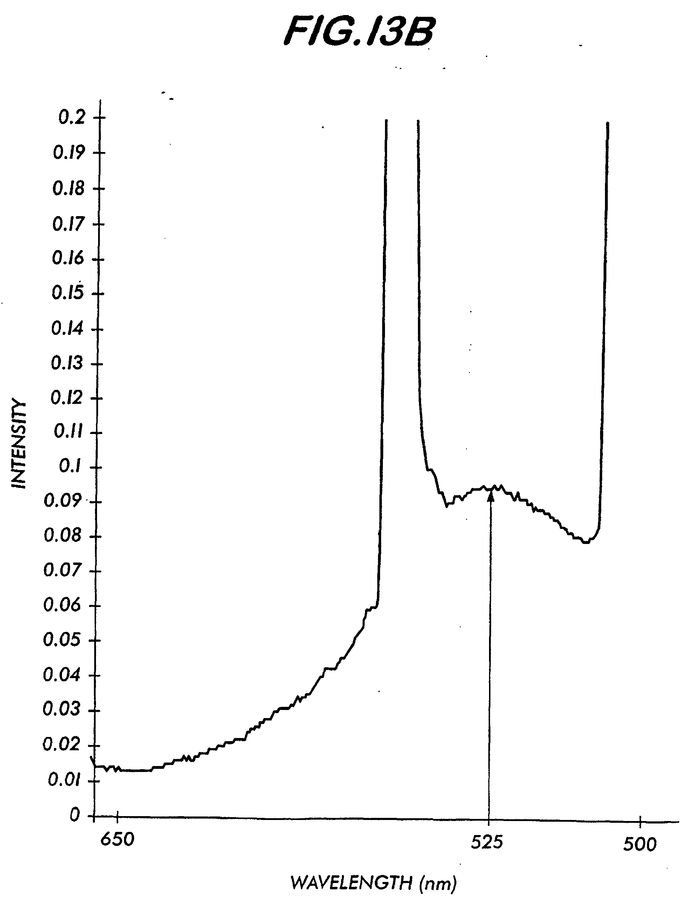

Fig. 13B shows the fluorescence spectrum of 5 pmol of DNA (SEQ ID NO:11) hybridized with 5 pmol of Probe No. 2.

### Example 13c: Two probes perfectly matching two target sequences in different strands.

5 pmol of DNA (SEQ ID NO:11) was added into 115 µl 0.5xTBE buffer. Then 2.5 pmol of Probe No. 1 and 5 pmol of Probe No. 2 were added into the solution. The sample was heated at 95°C for 10 minutes and hybridized at 25°C for 30 minutes.

Before fluorescence measurement, the unbound probe was filtered from the solution by G50 spin column. The solution with the hybrids of PNA-DNA was placed into a cuvette and subjected to fluorescence measurement.

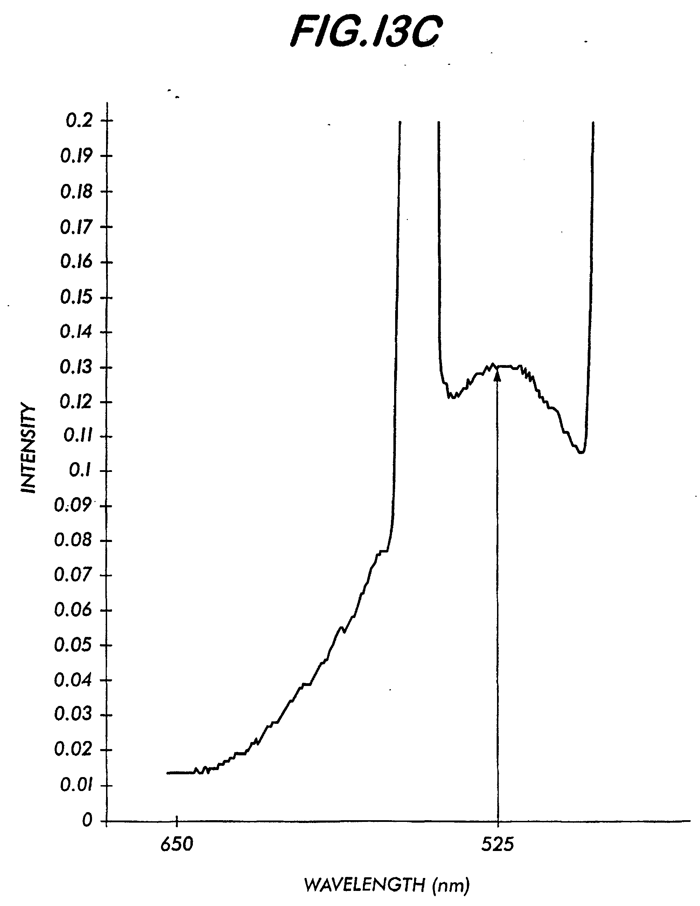

Fig. 13C shows the fluorescence spectrum of 5 pmol of DNA (SEQ ID NO:11) hybridized with 2.5 pmol of Probe No. 1 and 5 pmol of Probe No. 2. As shown in Fig. 13C, the fluorescent intensity at 525 nm is approximately about the sum of the intensities at 525 nm shown in Figs. 13A and 13B.

### EXAMPLE 14

Two PNA probes having different dyes (fluorescein and rhodamine) hybridized with one perfectly matched target.

### Example 14a

A 150 bp fragment of genomic DNA from wild type p53 DNA (SEQ ID NO:1) was amplified by PCR and purified by using the QIAquick PCR Purification Kit.

A 12-mer rhodamine labeled PNA probe (Probe No. 3) synthesized by PerSeptive Biosystems, Inc., having the structure: was designed to be complementary to a 12 nucleotide segment of the 150 bp p53 DNA, starting at base pair position 95 and ending at base pair position 106 (see SEQ ID NOS: 1, 11, and 12).

20 pmol of DNA (SEQ ID NO:1) was added into 50 µl 0.5xTBE buffer. Then 20 pmol of the Probe No. 3 was added into the solution. The sample was heated at 95°C for 10 minutes and hybridized at 25°C for 30 minutes.

Before fluorescence measurement, the unbound probe was filtered from the solution by G50 spin column. The solutions with the hybrids of PNA-DNA were placed into a cuvette and subjected to fluorescence measurement.

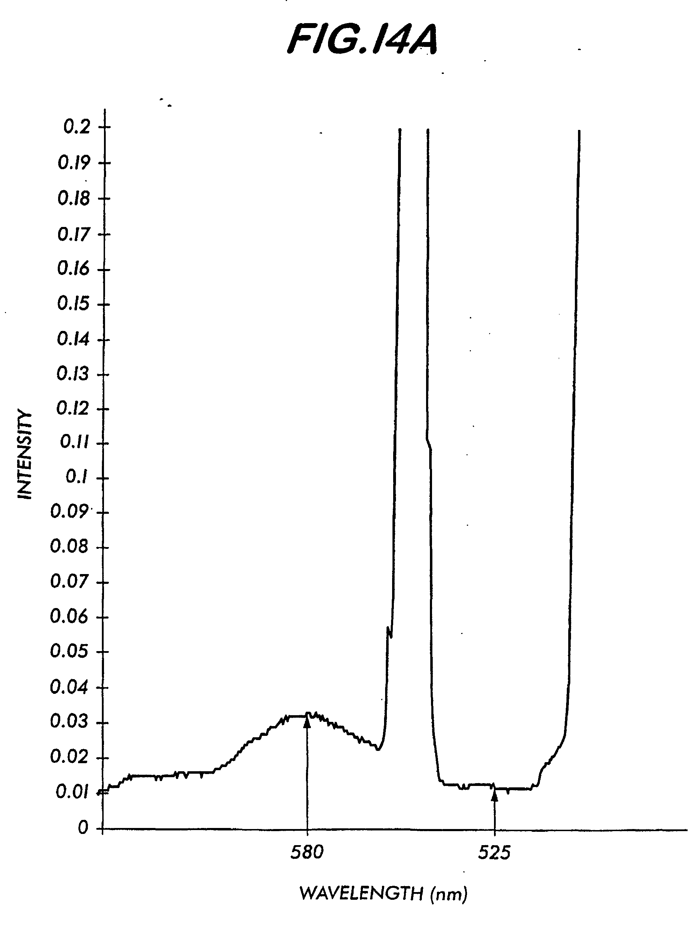

Fig. 14A shows a fluorescence spectrum of DNA hybridized with the rhodamine labeled PNA probe. The emission peak in the fluorescence spectrum emerges at about 580 nm.

### Example 14b

20 pmol of DNA (SEQ ID NO:1) was added into 50 µl 0.5xTBE buffer. Then 15 pmol of rhodamine labeled probe (Probe No. 3) and 5 pmol of fluorescein labeled probe (Probe No. 1) were added into the solution. The sample was heated at 95°C for 10 minutes and then the sample was hybridized at 25°C for 30 minutes.

Before fluorescence measurement, the unbound probe was filtered from the solution by G50 spin column. The solution with the hybrids of PNA-DNA was placed into a cuvette and subjected to fluorescence measurement.

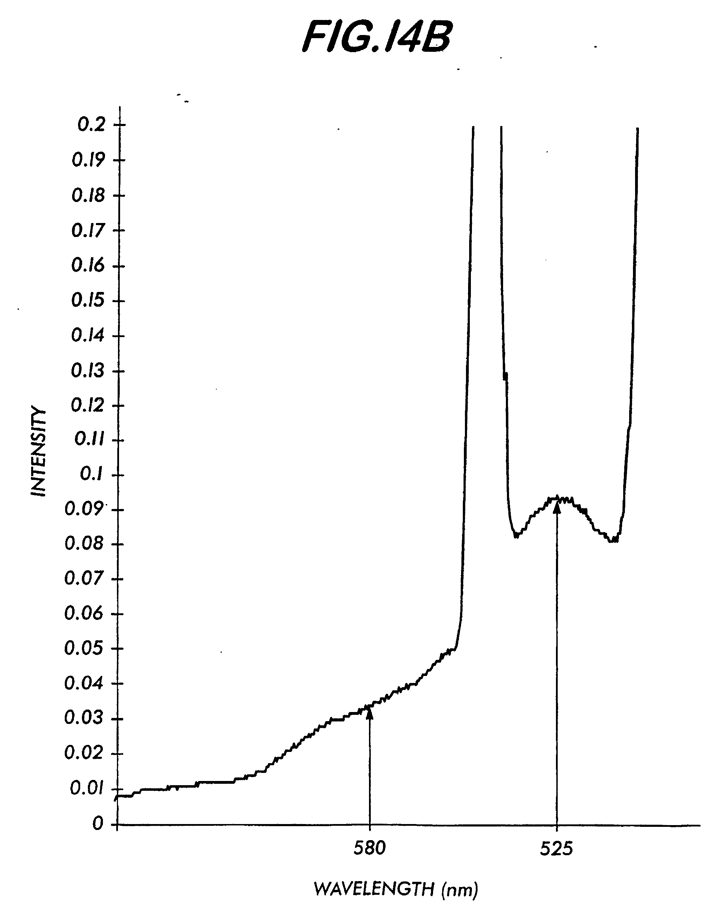

Fig. 14B shows a fluorescence spectrum of DNA hybridized with the mixed probes of rhodamine labeled PNA probe (Probe No. 3) and fluorescein labeled PNA probe(Probe No. 1). A shoulder emerged at 580 nm due at least in part to rhodamine labeled probe hybridized to the target sequence and a peak emerged at 525 nm partly contributed by the fluorescein labeled probe hybridized to the target sequence.

### EXAMPLE 15

Two PNA probes having different dyes (fluorescein and rhodamine) hybridized with two perfectly matched targets on two strands.

### Example 15a

20 pmol of DNA (SEQ ID NO:11) was added into 50 µl 0.5xTBE buffer. Then 20 pmol of rhodamine labeled probe (Probe No. 3) was added into the solution. The sample was heated at 95°C for 10 minutes and then the sample was hybridized at 25°C for 30 minutes.

Before fluorescence measurement, the unbound probe was filtered from the solution by G50 spin column. The solution with the hybrids of PNA-DNA was placed into a cuvette and subjected to fluorescence measurement.

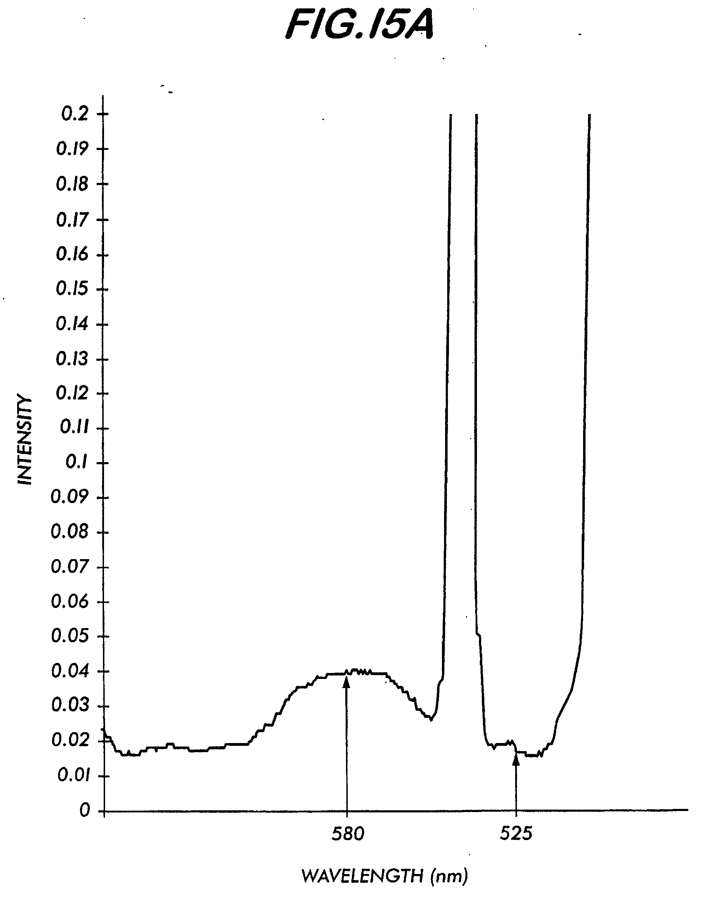

Fig. 15A shows a fluorescence spectrum of DNA hybridized with the rhodamine labeled PNA probe (Probe No. 3). A broad peak emerges at 580 nm contributed by hybridization complexes labeled with rhodamine.

### Example 15b

20 pmol of DNA (SEQ ID NO:11) was added into 50 µl 0.5xTBE buffer. Then 20 pmol of rhodamine labeled probe (Probe No. 3) and 5 pmol of fluorescein labeled probe (Probe No. 2) were added into the solution. The sample was heated at 95°C for 10 minutes and then the sample was hybridized at 25°C for 30 minutes.

Before fluorescence measurement, the unbound probe was filtered from the solution by G50 spin column. The solution with the hybrids of PNA-DNA was placed into a cuvette and subjected to fluorescence measurement.

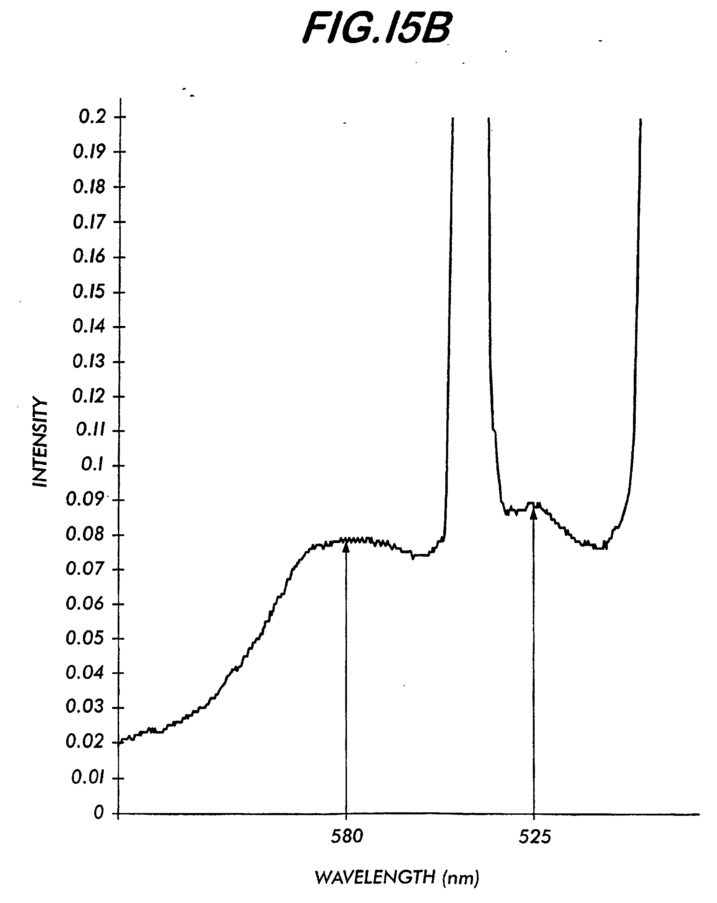

Fig. 15B shows a fluorescence spectrum of DNA hybridized with Probe Nos. 2 and 3. A broad peak emerges at 580 nm contributed by the rhodamine labeled probe and a peak rises at 525 nm contributed by the fluorescein labeled probe.

### EXAMPLE 16

A PCR amplified and purified 633 bp DNA (SEQ ID NO:10) lacking a target sequence was used as a negative control for probes.

5 pmol DNA (SEQ ID NO:10) was added into 115 µl 0.5xTBE buffer and then Probe Nos. 1-3 were added into the solution. Each sample was heated at 95°C for 10 minutes and then hybridized at 25°C for 30 minutes.

Before the fluorescence measurement, the unbound probe was filtered from the solution by G50 spin column. The solution was placed into a cuvette and subjected to fluorescence measurement.

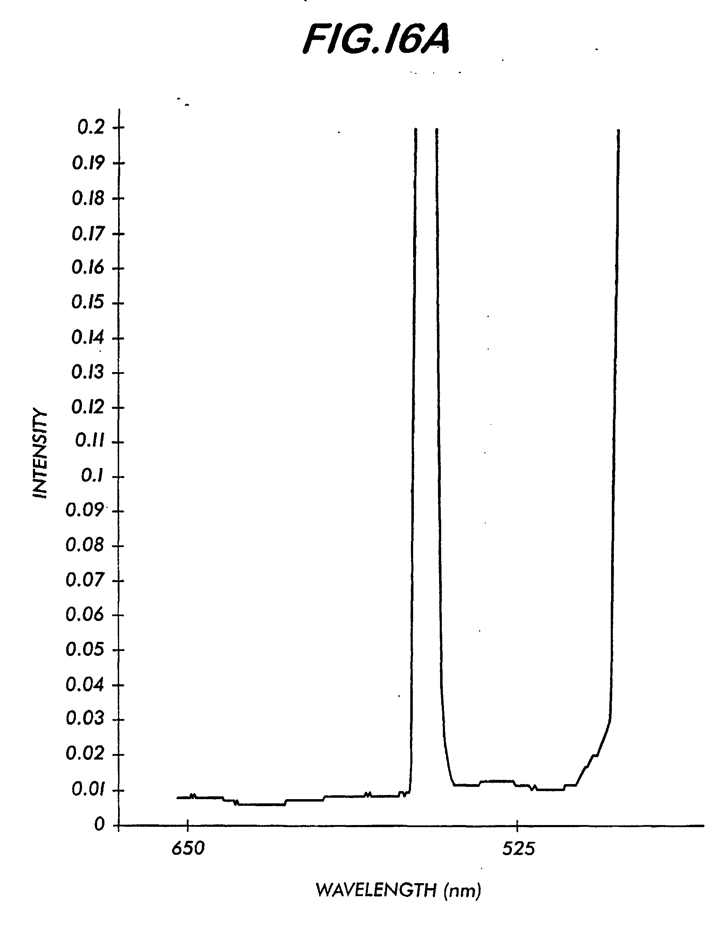

Fig. 16A shows a fluorescence spectrum of probe alone (i.e., no DNA was added to the sample, which included 15 pmol of the probes -- 5 pmol of each of Probe Nos. 1, 2 and 3).

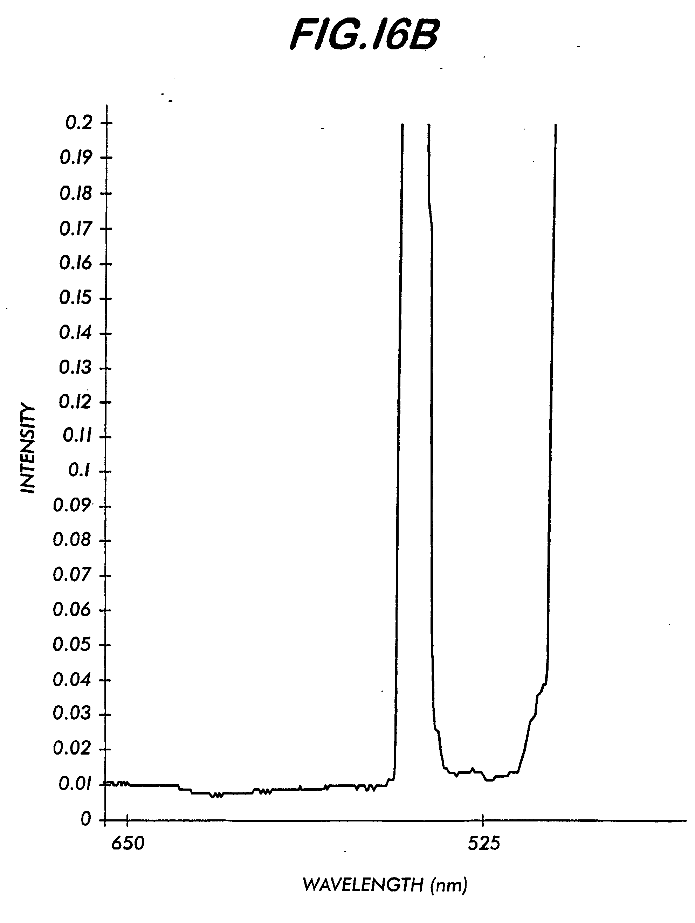

Fig. 16B shows a fluorescence spectrum of 5 pmol of Probe No. 1 and negative control DNA (SEQ ID NO:10).

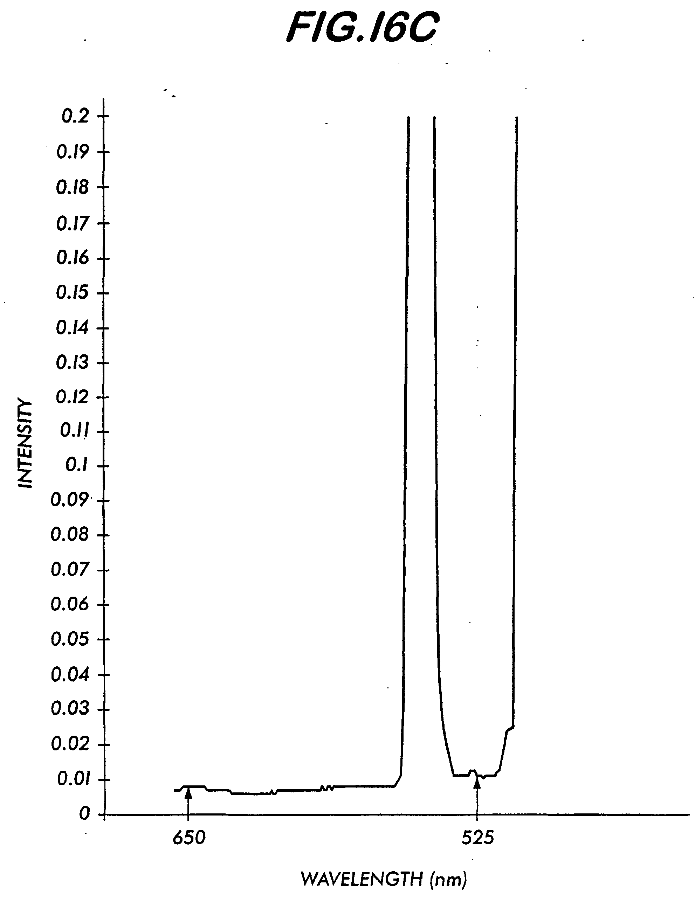

Fig. 16C shows a fluorescence spectrum of 5 pmol of Probe No. 2 and negative control DNA (SEQ ID NO:10).

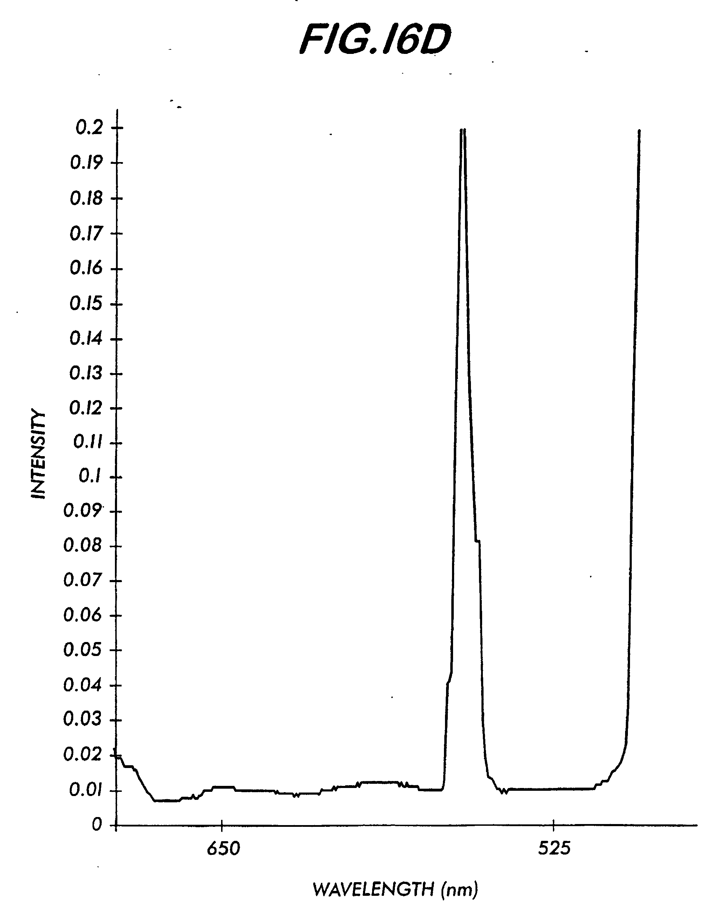

Fig. 16D shows a fluorescence spectrum of 5 pmol of Probe No. 3 and negative control DNA (SEQ ID NO:10).

All the spectra show the fluorescent intensity at background level at 525 nm and 580 nm.

### Example 17

Two PNA probes having the same dye (fluorescein) hybridized with a one base pair mismatched target sequence and a perfectly matched target sequence.

### Example 17a

A 150 bp fragment of genomic DNA from p53 DNA gene MQ (SEQ ID NO:12) was amplified by PCR and purified by using the QIAquick PCR Purification Kit. The mutated fragment was identical to the ME DNA (SEQ ID NO:11) fragment except for a one base mutation at amino acid position 340 (bases 88-90) at which the DNA sequence CTC was changed to GTC.

5 pmol of DNA (SEQ ID NO:12) was added into 115 µl 0.5xTBE buffer. Then 5 pmol of Probe No. 2, which has a one base pair mismatch to the target sequence, was added into the solution. The sample was heated at 95°C for 10 minutes and hybridized at 25°C for 30 minutes.

Before fluorescence measurement, the unbound probe was filtered from the solution by G50 spin column. The solution with the hybrids of PNA-DNA was placed into a cuvette and subjected to fluorescence measurement.

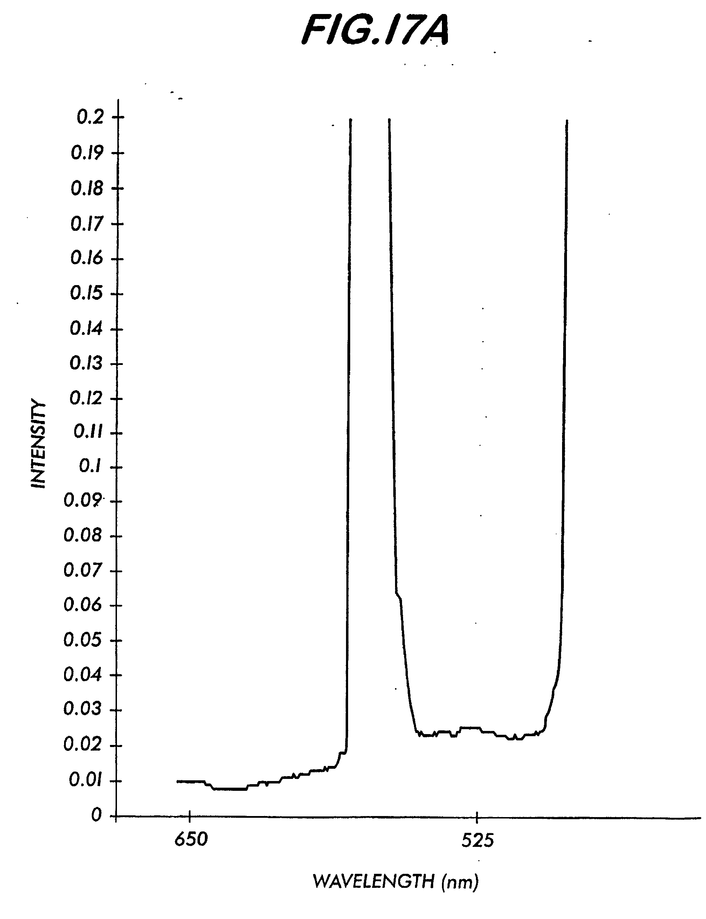

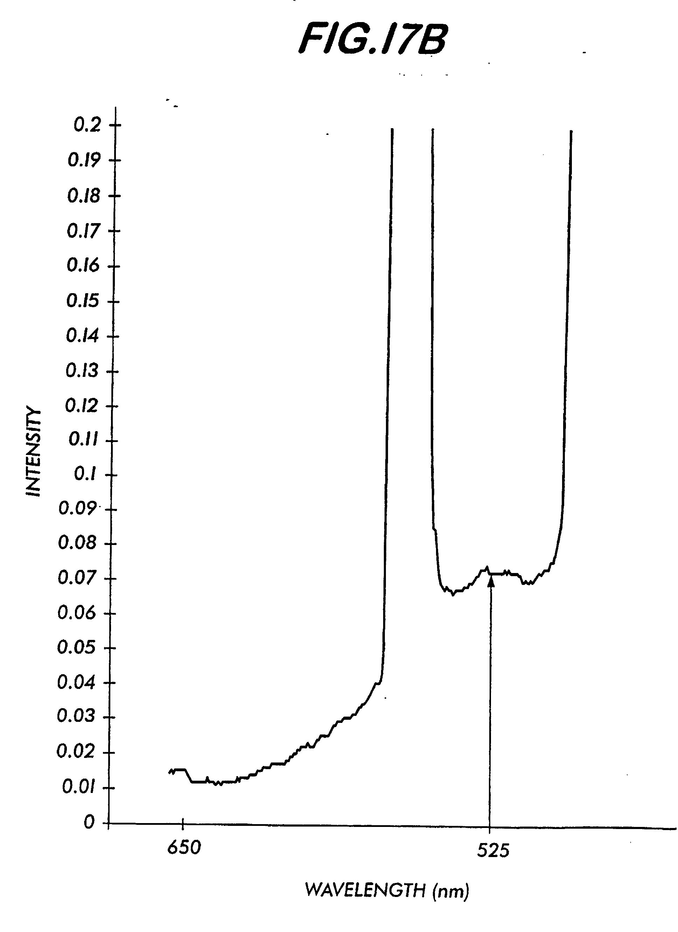

Fig. 17A shows the fluorescence spectrum of 5 pmol of DNA (SEQ ID NO:12) hybridized with 5 pmol of Probe No. 2. The spectrum shows the fluorescent intensity at background level.

### Example 17b

5 pmol of DNA (SEQ ID NO:12) was added into 115 µl 0.5xTBE buffer. Then Probe No. 1 was added into the solution. This probe was perfectly matched with the DNA target. The sample was heated at 95°C for 10 minutes and hybridized at 25°C for 30 minutes.

Before fluorescence measurement, the unbound probe was filtered from the solution by G50 spin column. The solution with the hybrids of PNA-DNA was placed into a cuvette and subjected to fluorescence measurement.

Fig. 17B gives the fluorescence spectrum of 5 pmol of DNA (SEQ ID NO:12) hybridized with 5 pmol of Probe No. 1. The positive signal at 525 nm is clearly shown in Fig. 17B.

### Example 17c

5 pmol of DNA (SEQ ID NO:12) was added into 115 µl 0.5xTBE buffer. Then 5 pmol Probe No. 1 and 5 pmol Probe No. 2 were added into the solution. The sample was heated at 95°C for 10 minutes and hybridized at 25°C for 30 minutes.

Before fluorescence measurement, the unbound probe was filtered from the solution by G50 spin column. The solution with the hybrids of PNA-DNA was placed into a cuvette and subjected to fluorescence measurement.

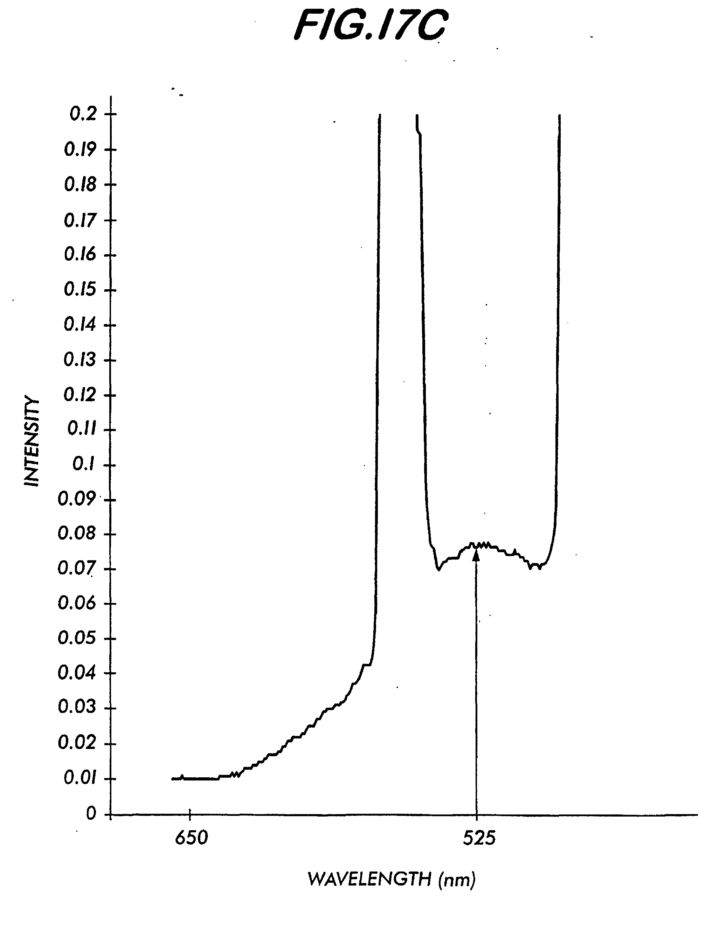

Fig. 17C shows the fluorescence spectrum of 5 pmol of DNA (SEQ ID NO:12) hybridized with 5 pmol of Probe No. 1 and 5 pmol of Probe No. 2. The fluorescent intensity at 525 nm is approximately equal to the intensity shown in Fig. 17B.

### Example 18

Two PNA probes having different dyes (fluorescein and rhodamine) hybridized with a one bp mismatched target and a perfectly matched target.

### Example 18a

5 pmol of DNA (SEQ ID NO:12) was added into 115 µl 0.5xTBE buffer. Then 5 pmol of rhodamine labeled probe (Probe No. 3) was added into the solution. This probe was perfectly matched to the target sequence. The sample was heated at 95°C for 10 minutes and hybridized at 25°C for 30 minutes.

Before fluorescence measurement, the unbound probe was filtered from the solution by G50 spin column. The solution with the hybrids of PNA-DNA was placed into a cuvette and subjected to fluorescence measurement.

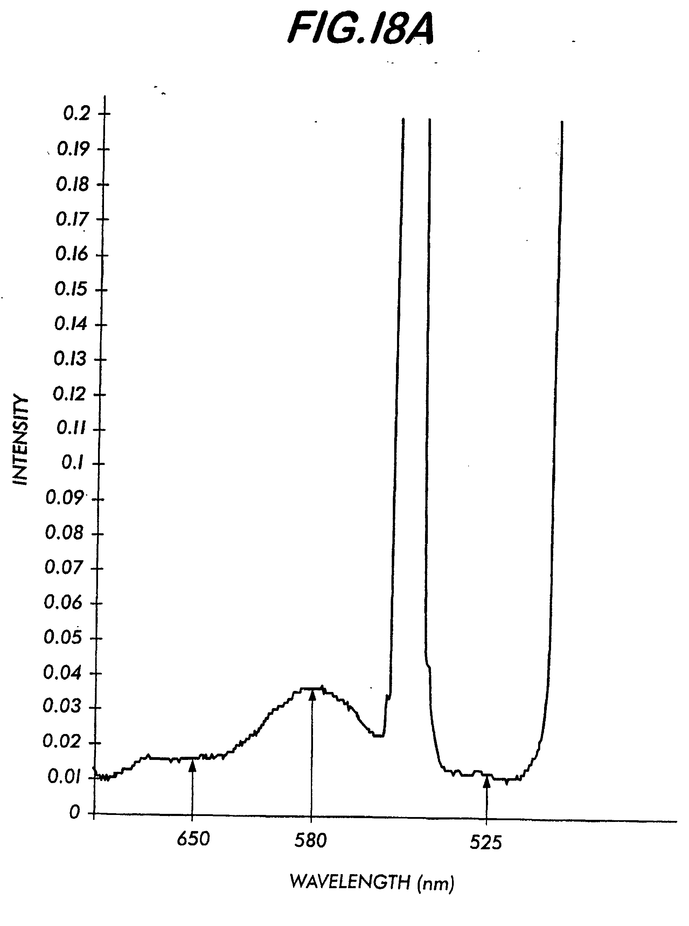

Fig. 18A shows the fluorescence spectrum of 5 pmol of DNA (SEQ ID NO:12) hybridized with 5 pmol of Probe No. 3. The positive signal at 580 nm is clearly shown in Fig. 18A.

### Example 18b

5 pmol of DNA (SEQ ID NO:12) was added into 115 µl 0.5xTBE buffer. Then 5 pmol of Probe No. 3, which was perfectly matched to the target sequence, and 5 pmol of Probe No. 2, which has one base pair mismatched to the target sequence, were added to the solution. The sample was heated at 95°C for 10 minutes and hybridized at 25°C for 30 minutes.

Before fluorescence measurement, the unbound probe was filtered from the solution by G50 spin column. The solution with the hybrids of PNA-DNA was placed into a cuvette and subjected to fluorescence measurement.

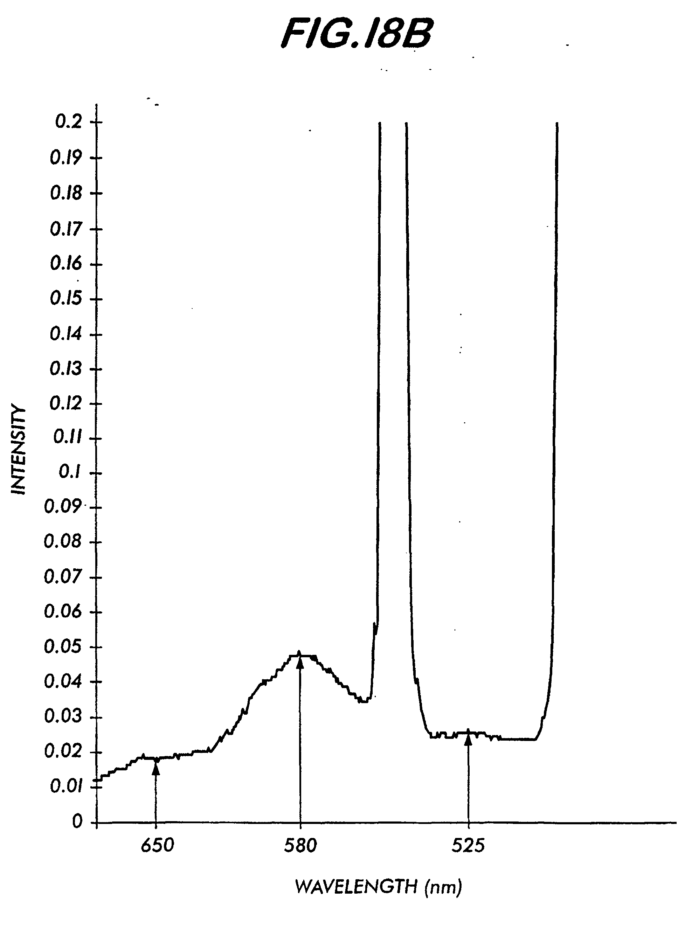

Fig. 18B shows the fluorescence spectrum of 5 pmol of DNA (SEQ ID NO:12) hybridized with 5 pmol of Probe No. 3 and 5 pmol of Probe No. 2. The fluorescent intensity at 525 nm is approximately equal to the intensity shown in Fig. 18A. The difference between the intensity at 580 nm and at 525 nm is approximately equal to that shown in Fig. 18A.

### EXAMPLE 19

Three PNA probes having the same dye (fluorescein) hybridized with two one base pair mismatched target sequences and one perfectly matched target.

### Example 19a

A 150 bp fragment of genomic DNA from p53 DNA gene QR (SEQ ID NO:13) was amplified by PCR and purified by using the QIAquick PCR Purification Kit. The mutated fragment was identical to the ME DNA (SEQ ID NO:11) fragment except for one base mutation at amino acid position 340 (bases 85-87) at which the DNA sequence CTC was changed to GTC and one base mismatch at amino acid position 344 (bases 100-102) at which the DNA sequence CTG was changed to CGG.

5 pmol of DNA (SEQ ID NO:13) was added into 115 µl 0.5xTBE buffer. Then 5 pmol of Probe No. 1, which has a one base pair mismatch to the target sequence at base pair position 101, was added into the solution. The sample was heated at 95°C for 10 minutes and hybridized at 25°C for 30 minutes.

Before fluorescence measurement, the unbound probe was filtered from the solution by G50 spin column. The solution with the hybrids of PNA-DNA was placed into a cuvette and subjected to fluorescence measurement.

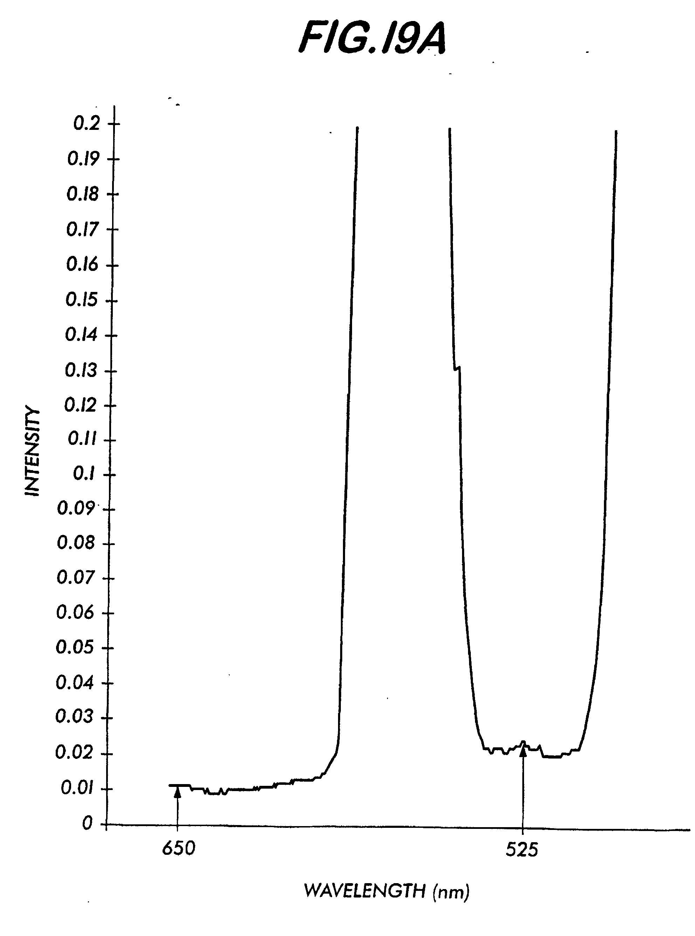

Fig. 19A shows the fluorescence spectrum of 5 pmol of DNA (SEQ ID NO:13) hybridized with 5 pmol of Probe No. 1. The spectrum shows the fluorescent intensity at 525 nm at background level.

### Example 19b

5 pmol of DNA (SEQ ID NO:13) was added into 115 µl 0.5xTBE buffer. Then Probe No. 2, which has a one base pair mismatch to the target sequence at base pair position 85, was added into the solution. The sample was heated at 95°C for 10 minutes and hybridized at 25°C for 30 minutes.

Before fluorescence measurement, the unbound probe was filtered from the solution by G50 spin column. The solution with the hybrids of PNA-DNA was placed into a cuvette and subjected to fluorescence measurement.

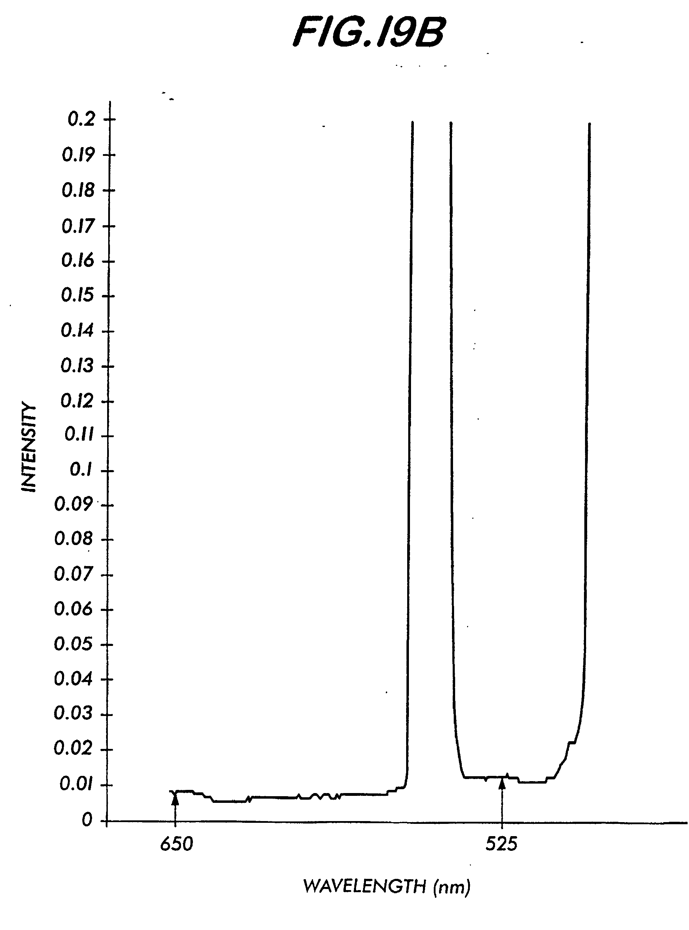

Fig. 19B shows the fluorescence spectrum of 5 pmol of DNA (SEQ ID NO:13) hybridized with 5 pmol of Probe No. 2. The spectrum shows the fluorescent intensity at 525 nm at background level.

### Example 19c

5 pmol of DNA (SEQ ID NO:13) was added into 115 µl 0.5xTBE buffer. A 12-mer fluorescein labeled PNA probe (Probe No. 4) synthesized by PerSeptive Biosystems, Inc., having the structure: was designed to be completely complementary to a 12 nucleotide segment of SEQ ID NO:13, starting at base pair position 95 and ending at base pair position 106. This probe was added into the solution. The sample was heated at 95°C for 10 minutes and hybridized at 25°C for 30 minutes.

Before fluorescence measurement, the unbound probe was filtered from the solution by G50 spin column. The solution with the hybrids of PNA-DNA was placed into a cuvette and subjected to fluorescence measurement.

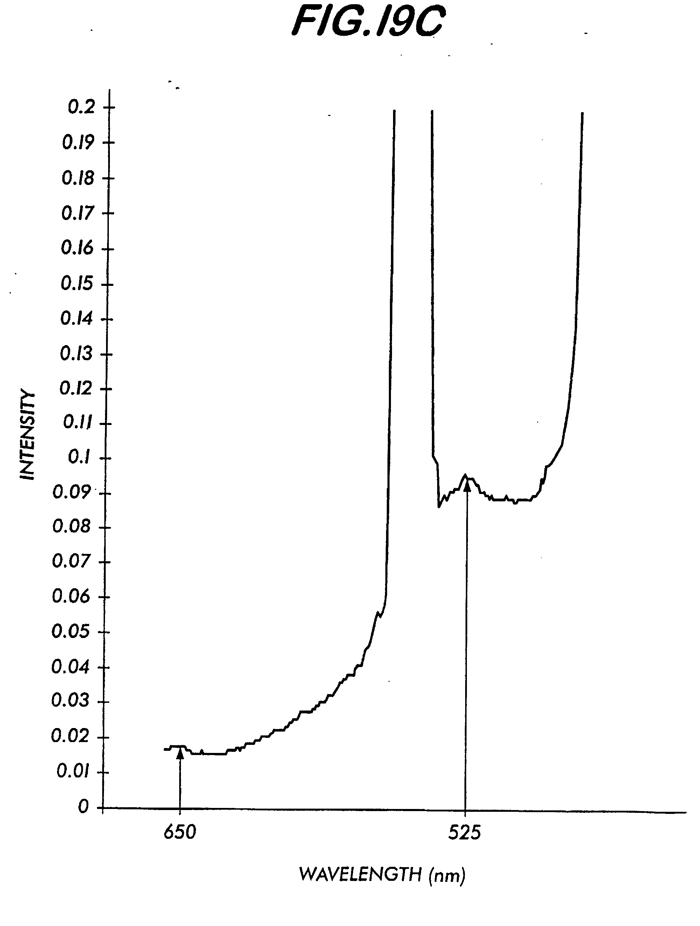

Fig. 19C shows the fluorescence spectrum of 5 pmol of DNA (SEQ ID NO:13) hybridized with 5 pmol of Probe No. 4. The positive signal at 525 nm is clearly shown in Fig. 19C.

### Example 19d

5 pmol of DNA (SEQ ID NO:13) was added into 115 µl 0.5xTBE buffer. Then 5 pmol of Probe No. 1 and 5 pmol of Probe No. 2 were added into the solution. The sample was heated at 95°C for 10 minutes and hybridized at 25°C for 30 minutes.

Before fluorescence measurement, the unbound probe was filtered from the solution by G50 spin column. The solution with the hybrids of PNA-DNA was placed into a cuvette and subjected to fluorescence measurement.

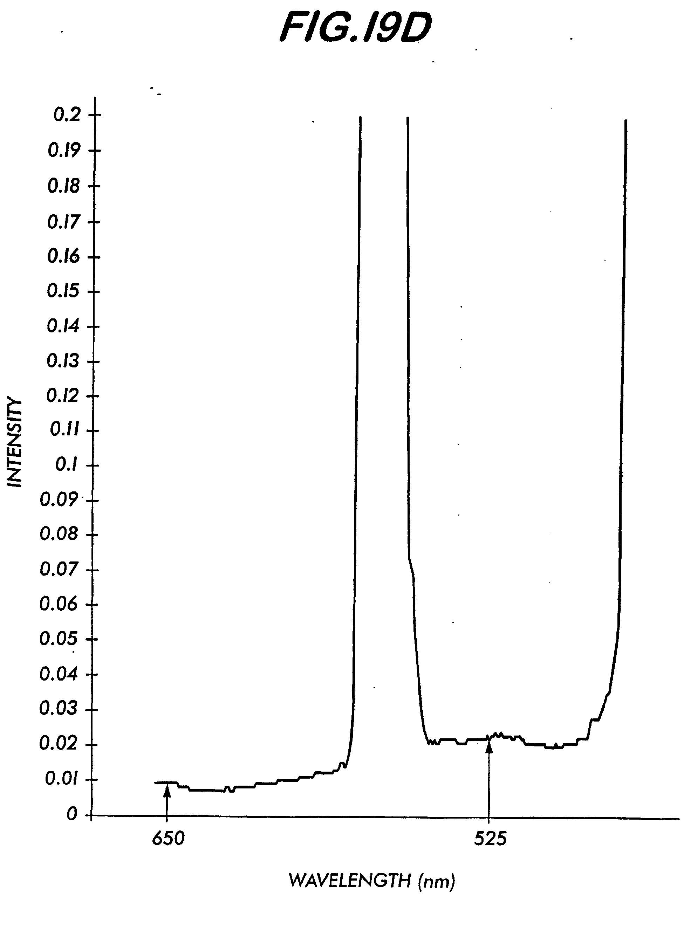

Fig. 19D gives the fluorescence spectrum of 5 pmol of DNA (SEQ ID NO:13) hybridized with 5 pmol of Probe No. 1 and 5 pmol of Probe No. 2. The spectrum shows the fluorescent intensity at 525 nm at background level.

### Example 19e

5 pmol of DNA (SEQ ID NO:13) was added to 115 µl 0.5xTBE buffer. Then 5 pmol of Probe No. 1, 5 pmol of Probe No. 2 and 5 pmol of Probe No. 4 were added into the solution. The sample was heated at 95°C for 10 minutes and hybridized at 25°C for 30 minutes.

Before fluorescence measurement, the unbound probe was filtered from the solution by G50 spin column. The solution with the hybrids of PNA-DNA was placed into a cuvette and subjected to fluorescence measurement.

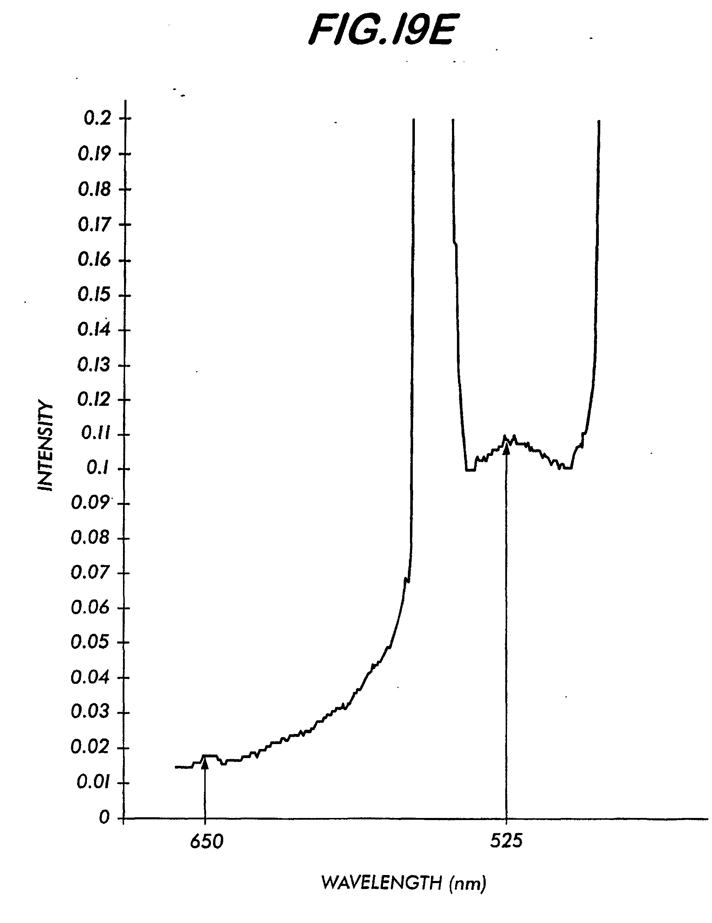

Fig. 19E gives the fluorescence spectrum of QR DNA (SEQ ID NO:13) hybridized with PNA Probe Nos. 1, 2 and 4. The fluorescent intensity at 525 nm is approximately equal to the intensity shown in Fig. 19C.

## Claims

1. A method for detecting at least one single stranded or double stranded nucleotide target sequence in a liquid medium, said method comprising:
adding to said liquid medium a PNA probe capable of forming a hybridization complex with said at least one target sequence, wherein said PNA probe comprises a fluorescent marker;
separating unhybridized PNA probe from said hybridization complex to form a test medium;
irradiating said test medium with a laser beam having a wavelength which excites said fluorescent marker and causes said fluorescent marker to emit fluorescent light;
measuring an intensity of said emitted fluorescent light; and
comparing said measured intensity with a reference intensity to detect whether said liquid medium contains said at least one target sequence and to determine the number of base mismatches between said at least one target sequence and said PNA probe, over a range inclusive of 0 base mismatches through at least 3 base mismatches;
and wherein said method other than said separating step is entirely conducted without binding said PNA probe, said nucleotide sequence or said hybridization complex to a solid support or gel.

2. The method for detecting at least one nucleotide sequence according to claim 1, wherein said separation is accomplished by at least one of filtration, centrifugation, precipitation and free solution electrophoresis.

3. The method for detecting at least one nucleotide sequence according to claim 1, wherein said laser beam has a wavelength of about 450 to about 530 nm.

4. The method for detecting at least one nucleotide sequence according to claim 1, wherein said emitted fluorescent light is measured in a range of 400 to 1000 nm.

5. The method for detecting at least one nucleotide sequence according to claim 1, wherein at least two different PNA probes capable of forming a hybridization complex with said at least one target sequence are added to said liquid medium, a first of said at least two different PMA probes is complementary to a first segment of a first nucleotide target sequence, a second of said at least two different PNA probes is complementary to a second segment of a second nucleotide target sequence, and said first and second segments differ from each other.

6. The method for detecting at least one nucleotide sequence according to claim 5, wherein said first and second probes are completely complementary to said first and second segments, respectively.

7. The method for detecting at least one nucleotide sequence according to claim 6, wherein said first nucleotide sequence is complementary to said second nucleotide sequence.

8. The method for detecting at least one nucleotide sequence according to claim 7, wherein said first nucleotide sequence and said second nucleotide sequence are coupled as double stranded DNA.

9. The method for detecting at least one nucleotide sequence according to claim 6, wherein said first nucleotide sequence and said second nucleotide sequence are in different genomes.

10. The method for detecting at least one nucleotide sequence according to claim 9, wherein said first probe has a first marker which has a first fluorescent emission intensity at a first wavelength, said second probe has a second marker which has a second fluorescent emission intensity at a second wavelength, said first and second wavelengths are different, said first nucleotide sequence is detected by monitoring fluorescent emission intensity at said first wavelength and said second nucleotide sequence is detected by monitoring fluorescent emission intensity at said second wavelength.

11. The method for detecting at least one nucleotide sequence according to claim 6, wherein said first probe has a first marker which has a first fluorescent emission intensity at a first wavelength, said second probe has a second marker which has a second fluorescent emission intensity at a second wavelength, said first and second wavelengths are different, said first nucleotide sequence is detected by monitoring fluorescent emission intensity at said first wavelength and said second nucleotide sequence is detected by monitoring fluorescent emission intensity at said second wavelength.

12. The method for detecting at least one nucleotide sequence according to claim 11, wherein said first nucleotide sequence is a positive control expected to be present in said liquid medium being analyzed, said first intensity is said reference intensity, said second intensity is said measured intensity, and said second nucleotide sequence is detected by comparing said first intensity and said second intensity.

13. The method for detecting at least one nucleotide sequence according to claim 12, wherein the second nucleotide sequence is found only in a mutant genome and the first nucleotide sequence is found in said mutant genome and in a corresponding wild-type genome.

14. The method for detecting at least one nucleotide sequence according to claim 13, wherein the first and second markers are selected from the group consisting of fluorescein and rhodamine.

15. The method for detecting at least one nucleotide sequence according to claim 12, wherein a third PNA probe having a third marker which has a third fluorescent emission intensity at a third wavelength differing from the first and second wavelengths is added to said liquid medium as a negative control which is not expected to hybridize with any nucleotide sequence, and said second nucleotide sequence is detected by comparing the first, second and third intensities.

16. The method for detecting at least one nucleotide sequence according to claim 1, wherein at least two different PNA probes capable of forming a hybridization complex with said at least one target sequence are added to said liquid medium, a first of said at least two different PNA probes is complementary to a first segment of said at least one target sequence, a second of said at least two different PNA probes is complementary to a second segment of said at least one target sequence, and said first and second segments differ from each other.

17. The method for detecting at least one nucleotide sequence according to claim 16, wherein said first probe has a first marker which has a first fluorescent emission intensity at a first wavelength, said second probe has a second marker which has a second fluorescent emission intensity at a second wavelength, said first and second wavelengths are different, said first segment is detected by monitoring fluorescent emission intensity at said first wavelength and said second segment is detected by monitoring fluorescent emission intensity at said second wavelength.

18. The method for detecting at least one nucleotide sequence according to claim 17, wherein said first segment is a positive control expected to be present in said liquid medium being analyzed, said first intensity is said reference intensity, said second intensity is said measured intensity, and said second segment is detected by comparing said first intensity and said second intensity.

19. The method for detecting at least one nucleotide sequence according to claim 18, wherein a third PNA probe having a third marker which has a third fluorescent emission intensity at a third wavelength differing from the first and second wavelengths is added to said liquid medium as a negative control which is not expected to hybridize with any nucleotide segment, and said second segment is detected by comparing the first, second and third intensities.

20. The method for detecting at least one nucleotide sequence according to claim 1, wherein said hybridization complex consists essentially of one PNA sequence, one fluorophore and said nucleotide sequence.

21. The method for detecting at least one nucleotide sequence according to claim 1, wherein all probes employed in said method consist of the same sequence and marker, said marker being the only marker detected in said method.

22. The method for detecting at least one nucleotide sequence according to claim 1, wherein said emitted fluorescent light has a wavelength longer than said laser beam wavelength.

23. The method for detecting at least one nucleotide sequence according to claim 1, wherein said at least one nucleotide sequence is double stranded DNA.

24. A method for detecting a single stranded or double stranded nucleotide sequence in a first liquid medium, comprising:
providing said first liquid medium- comprising sample nucleotide sequences;
adding to said first liquid medium a PNA probe capable of forming a hybridization complex with said nucleotide sequence, wherein said PNA probe comprises a fluorescent marker;
irradiating said first liquid medium with a laser beam having a wavelength which excites said fluorescent marker and causes said fluorescent marker to emit fluorescent light;
detecting a first intensity of said fluorescent light in said first liquid medium; and
determining whether said first intensity is equal to a second intensity of a second liquid medium containing said nucleotide sequence hybridized with said PNA probe, to detect whether said nucleotide sequence is in said first liquid medium,
wherein said PNA probe, said nucleotide sequence and said hybridization complex are not bonded to a solid support or gel, and said hybridization complex consists essentially of one PNA sequence, one fluorophore and said nucleotide sequence.

25. The method for detecting a nucleotide sequence according to claim 24, wherein said laser beam is produced by an argon ion laser which irradiates said marker with light having a wavelength of about 450 to about 530 nm.

26. The method for detecting a nucleotide sequence according to claim 24, wherein said fluorescent light is detected in a range of 400 to 1000 nm.

27. The method for detecting a nucleotide sequence according to claim 24, wherein (i) said nucleotide sequence is a mutant type DNA to be distinguished from a wild type DNA differing from said mutant type DNA, (ii) said PNA probe is completely complementary with a segment of said mutant type DNA and is not completely complementary with a segment of said wild type DNA, and (iii) said mutant type DNA is detected if said first intensity equals said second intensity.

28. The method for detecting a nucleotide sequence according to claim 24, wherein (i) said nucleotide sequence is a wild type DNA to be distinguished from a mutant type DNA differing from said wild type DNA, (ii) said PNA probe is completely complementary with a segment of said wild type DNA and is not completely complementary with a segment of said mutant type DNA, and (iii) said wild type DNA is detected if said first intensity equals said second intensity.

29. The method for detecting a nucleotide sequence according to claim 24, wherein said nucleotide sequence is denatured in said liquid medium prior to said detecting step, at a temperature of about 85°C to about 100°C for about 30 seconds to about 5 hours.

30. The method for detecting a nucleotide sequence according to claim 29, wherein said hybridization complex formation is conducted at a temperature of about 4°C to about 75°C for about 2 minutes to about 24 hours.

31. The method for detecting a nucleotide sequence according to claim 30, wherein said denaturing step is conducted for no more than 60 minutes, after which said temperature is passively cooled to room temperature without quenching.

32. The method for detecting a nucleotide sequence according to claim 24, wherein said PNA probe is added to said first liquid medium in a concentration 1 to 20 times a suspected concentration of said nucleotide sequence.

33. The method for detecting a nucleotide sequence according to claim 24, wherein a total volume of said first liquid medium in said detecting step is no more than about 5 milliliters.

34. The method for detecting a nucleotide sequence according to claim 33, wherein said total volume is no more than about 10 microliters.

35. The method for detecting a nucleotide sequence according to claim 24, wherein said nucleotide sequence is double stranded DNA.

36. The method for detecting a nucleotide sequence according to claim 29, wherein said PNA probe is added to said first liquid medium prior to completion of said denaturing step.

37. The method of claim 24, wherein all probes employed in said method consist of the same sequence and marker, said marker being the only marker detected in said method.

38. The method of claim 24, wherein said detected fluorescent light consists essentially of said fluorescent light emitted by said marker.

39. The method of claim 24, wherein said PNA probe is completely complementary to a segment of said nucleotide sequence.

40. The method of claim 24, wherein said PNA probe is a one-base mismatch of a segment of said nucleotide sequence.

41. The method of claim 24, wherein said PNA probe is a two-base mismatch of a segment of said nucleotide sequence.

42. The method of claim 24, wherein said PNA probe is a three-base mismatch of a segment of said nucleotide sequence.

43. The method of claim 24, wherein (i) said sample nucleotide sequences are identical to said nucleotide sequence or are analogs differing from said nucleotide sequence by at least one base, (ii) said PNA probe is completely complementary with a segment of said nucleotide sequence and is not complementary with a segment of said analogs, (iii) said nucleotide sequence is detected if said first intensity equals said second intensity, and (iv) said analogs are detected if said first intensity does not equal said second intensity.

44. The method of claim 43, wherein said analogs differ from said nucleotide sequence by one base.

45. The method of claim 43, wherein said analogs differ from said nucleotide sequence by two bases.

46. The method of claim 43, wherein said analogs differ from said nucleotide sequence by three bases.

47. The method of claim 43, wherein all probes employed in said method consist of the same sequence and marker, said marker being the only marker detected in said method.

48. The method of claim 47, wherein said detected fluorescent light consists essentially of said fluorescent light emitted by said marker.

49. The method of claim 48, wherein said analogs differ from said nucleotide sequence by one base.

50. The method of claim 24, wherein (i) said sample nucleotide sequences are identical to said nucleotide sequence or are analogs differing from said nucleotide sequence by at least one base, (ii) said PNA probe is completely complementary with a segment of said analogs and is not completely complementary with a segment of said nucleotide sequence, (iii) said nucleotide sequence is detected if said first intensity equals said second intensity, and (iv) said analogs are detected if said first intensity does not equal said second intensity.

51. The method of claim 50, wherein said analogs differ from said nucleotide sequence by one base.

52. The method of claim 50, wherein said analogs differ from said nucleotide sequence by two bases.

53. The method of claim 50, wherein said analogs differ from said nucleotide sequence by three bases.

54. The method of claim 50, wherein all probes employed in said method consist of the same sequence and marker, said marker being the only marker detected in said method.

55. The method of claim 54, wherein said detected fluorescent light consists essentially of .said fluorescent light emitted by said marker.

56. The method of claim 55, wherein said analogs differ from said nucleotide sequence by one base.

57. The method of claim 24, wherein said detected fluorescent light has a wavelength longer than said laser beam wavelength.

58. The method of claim 24, wherein said first intensity is inversely proportional to a number of base mismatches between said nucleotide sequence and said PNA probe, over a range inclusive of 0 base mismatches through at least 3 base mismatches.

59. A method for detecting a single stranded or double stranded nucleotide sequence in a first liquid medium, comprising:
providing said first liquid medium comprising sample nucleotide sequences;
adding to said first liquid medium a PNA probe capable of forming a hybridization complex with said nucleotide sequence, wherein said PNA probe comprises a fluorescent marker;
separating unhybridized PNA probe from said hybridization complex to form a first test medium;
irradiating said first test medium with a laser beam having a wavelength which excites said fluorescent marker and causes said fluorescent marker to emit fluorescent light;
directly detecting a first intensity of said emitted fluorescent light in said first test medium; and
comparing said first intensity with a reference intensity produced by probing a positive control sequence and a baseline intensity produced by probing a negative control sequence,
wherein-said nucleotide sequence is detected when said first intensity equals said reference intensity, said nucleotide sequence is not detected when said first intensity equals said baseline intensity, and a homologous sequence differing from said nucleotide sequence by at least one base is detected when said first intensity is between said baseline intensity and said reference intensity, and wherein said method other than said separating step is entirely conducted without binding said PNA probe, said nucleotide sequence or said hybridization complex to a solid support or gel.

60. The method of claim 59, wherein said homologous sequence differs from said nucleotide sequence by one base.

61. The method of claim 59, wherein said hybridization complex consists essentially of one PNA sequence, one fluorophore and said nucleotide sequence.

62. The method of claim 59, wherein all probes employed in said method consist of the same sequence and marker, said marker being the only marker detected in said method.

63. The method of claim 59, wherein said detected fluorescent light consists essentially of said fluorescent light emitted by said marker.

64. The method of claim 59, wherein said detected fluorescent light has a wavelength longer than said laser beam wavelength.

65. The method of claim 59, wherein said first intensity is inversely proportional to a number of base mismatches between said nucleotide sequence and said PNA probe, over a range inclusive of 0 base mismatches through at least 3 base mismatches.

66. A method for detecting a single stranded or double stranded nucleotide sequence in a liquid medium, comprising:
providing said liquid medium comprising first nucleotide sequences which are identical to said nucleotide sequence or second nucleotide sequences differing from said nucleotide sequence by at least one base;
adding to said liquid medium PNA probes completely complementary to a segment of said nucleotide sequence and not completely complementary to a segment of said second nucleotide sequences, wherein each of said PNA probes comprises a fluorescent marker;
hybridizing said PNA probes with said first or second nucleotide sequences;
irradiating said liquid medium to cause said fluorescent marker to emit fluorescent light having a wavelength of 400 to 1000 nm; and
detecting an intensity of said fluorescent light,
wherein an electric field is applied to said liquid medium prior to or concurrently with said detecting step, and a change in said intensity of said fluorescent light as a function of said electric field is detected as an indication of whether said PNA probes are hybridized to said first nucleotide sequences or to said second nucleotide sequences.

67. The method of claim 66, wherein said first and second nucleotide sequences differ by one base.

## Patentansprüche

1. Methode für das Erfassen mindestens einer einzelsträngigen oder doppelsträngigen Nucleotid-Zielsequenz in einem flüssigen Medium, welche Methode Folgendes umfasst:
Zugeben, zu dem flüssigen Medium, einer PNA-Sonde, die in der Lage ist, einen Hybridisierungskomplex mit der mindestens einen Zielsequenz zu bilden, wobei die PNA-Sonde einen fluoreszierenden Marker umfasst,
Abtrennen der nichthybridisierten PNA-Sonde von dem Hybridisierungskomplex unter Bildung eines Testmediums,
Bestrahlen des Testmediums mit einem Laserstrahl, der eine Wellenlänge aufweist, die den fluoreszierenden Marker anregt und den fluoreszierenden Marker veranlasst, fluoreszierendes Licht auszustrahlen,
Messen einer Intensität des ausgestrahlten fluoreszierenden Lichts, und
Vergleichen der gemessenen Intensität mit einer Bezugsintensität, um zu erfassen, ob das flüssige Medium die mindestens eine Zielsequenz enthält und um die Anzahl der Basenfehlpaarungen zwischen der mindestens einen Zielsequenz und der PNA-Sonde über einen Bereich, der 0 Basenfehlpaarungen bis mindestens 3 Basenfehlanpassungen umfasst, zu bestimmen,
und wobei die Methode, mit Ausnahme des Abtrennschritts, ausschließlich ohne Binden der PNA-Sonde, der Nucleotidsequenz oder des Hybridisierungskomplexes an einen festen Träger oder ein festes Gel durchgeführt wird.

2. Methode für das Erfassen mindestens einer Nucleotidsequenz nach Anspruch 1, wobei die Abtrennung durch mindestens eines unter Filtrieren, Zentrifugieren, Ausfällen und Freilösungselektrophorese ausgeführt wird.

3. Methode für das Erfassen mindestens einer Nucleotidsequenz nach Anspruch 1, wobei der Laserstrahl eine Wellenlänge von ca. 450 bis ca. 530 nm aufweist.

4. Methode für das Erfassen mindestens einer Nucleotidsequenz nach Anspruch 1, wobei das ausgestrahlte fluoreszierende Licht in einem Bereich von 400 bis 1000 nm gemessen wird.

5. Methode für das Erfassen mindestens einer Nucleotidsequenz nach Anspruch 1, wobei mindestens zwei verschiedene PNA-Sonden, die in der Lage sind, einen Hybridisierungskomplex mit der mindestens einen Zielsequenz zu bilden, dem flüssigen Medium zugegeben werden, eine erste von den mindestens zwei verschiedenen PNA-Sonden einem ersten Segment einer ersten Nucleotidzielsequenz komplementär ist, eine zweite der mindestens zwei verschiedenen PNA-Sonden einem zweiten Segment einer zweiten Nucleotidzielsequenz komplementär ist und die ersten und zweiten Segmente voneinander verschieden sind.

6. Methode für das Erfassen mindestens einer Nucleotidsequenz nach Anspruch 5, wobei die ersten und zweiten Sonden jeweils den ersten und zweiten Segmenten vollständig komplementär sind.

7. Methode für das Erfassen mindestens einer Nucleotidsequenz nach Anspruch 6, wobei die erste Nucleotidsequenz der zweiten Nucleotidsequenz komplementär ist.

8. Methode für das Erfassen mindestens einer Nucleotidsequenz nach Anspruch 7, wobei die erste Nucleotidsequenz und die zweite Nucleotidsequenz als doppelsträngige DNA gekoppelt sind.

9. Methode für das Erfassen mindestens einer Nucleotidsequenz nach Anspruch 6, wobei die erste Nucleotidsequenz und die zweite Nucleotidsequenz sich in verschiedenen Genomen befinden.

10. Methode für das Erfassen mindestens einer Nucleotidsequenz nach Anspruch 9, wobei die erste Sonde einen ersten Marker aufweist, der eine erste fluoreszierende Ausstrahlungsintensität bei einer ersten Wellenlänge aufweist, die zweite Sonde einen zweiten Marker aufweist, der eine zweite fluoreszierende Ausstrahlungsintensität bei einer zweiten Wellenlänge aufweist, die ersten und zweiten Wellenlängen verschieden sind, die erste Nucleotidsequenz durch Überwachen der fluoreszierenden Ausstrahlungsintensität bei der ersten Wellenlänge und die zweite Nucleotidsequenz durch Überwachen der fluoreszierenden Ausstrahlungsintensität bei der zweiten Wellenlänge erfasst wird.

11. Methode für das Erfassen mindestens einer Nucleotidsequenz nach Anspruch 6, wobei die erste Sonde einen ersten Marker aufweist, der eine erste fluoreszierende Ausstrahlungsintensität bei einer ersten Wellenlänge aufweist, die zweite Sonde einen zweiten Marker aufweist, der eine zweite fluoreszierende Ausstrahlungsintensität bei einer zweiten Wellenlänge aufweist, die ersten und zweiten Wellenlängen verschieden sind, die erste Nucleotidsequenz durch Überwachen der fluoreszierenden Ausstrahlungsintensität bei der ersten Wellenlänge und die zweite Nucleotidsequenz durch Überwachen der fluoreszierenden Ausstrahlungsintensität bei der zweiten Wellenlänge erfasst wird.

12. Methode für das Erfassen mindestens einer Nucleotidsequenz nach Anspruch 11, wobei die erste Nucleotidsequenz eine positive Kontrolle darstellt, von der zu erwarten ist, dass sie sich in dem zu analysierenden flüssigen Medium befindet, die erste Intensität die Bezugsintensität darstellt, die zweite Intensität die gemessene Intensität darstellt und die zweite Nucleotidsequenz durch Vergleich der ersten Intensität und der zweiten Intensität erfasst wird.

13. Methode für das Erfassen mindestens einer Nucleotidsequenz nach Anspruch 12, wobei die zweite Nucleotidsequenz nur in einem mutanten Genom zu finden ist und die erste Nucleotidsequenz in dem mutanten Genom und in einem entsprechenden Wildtyp-Genom zu finden ist.

14. Methode für das Erfassen mindestens einer Nucleotidsequenz nach Anspruch 13, wobei die ersten und zweiten Marker aus der Gruppe ausgewählt werden, die aus Fluorescein und Rhodamin besteht.

15. Methode für das Erfassen mindestens einer Nucleotidsequenz nach Anspruch 12, wobei eine dritte PNA-Sonde, die einen dritten Marker aufweist, der eine dritte fluoreszierende Ausstrahlungsintensität bei einer dritten Wellenlänge aufweist, die von den ersten und zweiten Wellenlängen verschieden ist, dem flüssigen Medium als negative Kontrolle zugegeben wird, von der nicht zu erwarten ist, dass sie sich mit irgendeiner Nucleotidsequenz hybridisiert, und die zweite Nucleotidsequenz durch Vergleichen der ersten, zweiten und dritten Intensitäten erfasst wird.

16. Methode für das Erfassen mindestens einer Nucleotidsequenz nach Anspruch 1, wobei mindestens zwei verschiedene PNA-Sonden, die in der Lage sind, einen Hybridisierungskomplex mit mindestens einer Zielsequenz zu bilden, dem flüssigen Medium zugegeben werden, eine erste von dem mindestens zwei verschiedenen PNA-Sonden einem ersten Segment der mindestens einen Zielsequenz komplementär ist, eine zweite der mindestens zwei verschiedenen PNA-Sonden einem zweiten Segment der mindestens einen Zielsequenz komplementär ist und die ersten und zweiten Segmente voneinander verschieden sind.

17. Methode für das Erfassen mindestens einer Nucleotidsequenz nach Anspruch 16, wobei die erste Sonde einen ersten Marker aufweist, der eine erste fluoreszierende Ausstrahlungsintensität bei einer ersten Wellenlänge aufweist, die zweite Sonde einen zweiten Marker aufweist, der eine zweite fluoreszierende Ausstrahlungsintensität bei einer zweiten Wellenlänge aufweist, die ersten und zweiten Wellenlängen verschieden sind, das erste Segment durch Überwachen der fluoreszierenden Ausstrahlungsintensität bei der ersten Wellenlänge und das zweite Segment durch Überwachen der fluoreszierenden Ausstrahlungsintensität bei der zweiten Wellenlänge erfasst wird.

18. Methode für das Erfassen mindestens einer Nucleotidsequenz nach Anspruch 17, wobei das erste Segment eine positive Kontrolle darstellt, von der zu erwarten ist, dass sie sich in dem zu analysierenden flüssigen Medium befindet, die erste Intensität die Bezugsintensität darstellt, die zweite Intensität die gemessene Intensität darstellt und das zweite Segment durch Vergleich der ersten Intensität und der zweiten Intensität erfasst wird.

19. Methode für das Erfassen mindestens einer Nucleotidsequenz nach Anspruch 18, wobei eine dritte PNA-Sonde, die einen dritten Marker aufweist, der eine dritte fluoreszierende Ausstrahlungsintensität bei einer dritten Wellenlänge aufweist, die von der ersten und zweiten Wellenlängen verschieden ist, dem flüssigen Medium als negative Kontrolle zugegeben wird, von der nicht zu erwarten ist, dass sie sich mit irgendeinem Nucleotidsegment hybridisiert, und das zweite Segment durch Vergleichen der ersten, zweiten und dritten Intensitäten erfasst wird.

20. Methode für das Erfassen mindestens einer Nucleotidsequenz nach Anspruch 1, wobei der Hybridisierungskomplex im wesentlichen aus einer PNA-Sequenz, einem Fluorophor und der Nucleotidsequenz besteht.

21. Methode für das Erfassen mindestens einer Nucleotidsequenz nach Anspruch 1, wobei alle bei dieser Methode verwendeten Sonden aus der gleichen Sequenz und dem gleichen Marker bestehen, wobei der Marker der einzige durch die Methode erfasste Marker ist.

22. Methode für das Erfassen mindestens einer Nucleotidsequenz nach Anspruch 1, wobei das ausgestrahlte fluoreszierende Licht eine Wellenlänge aufweist, die länger ist als die Laserstrahlwellenlänge.

23. Methode für das Erfassen mindestens einer Nucleotidsequenz nach Anspruch 1, wobei mindestens eine Nucleotidsequenz eine doppelsträngige DNA ist.

24. Methode für das Erfassen einer einzelsträngigen oder doppelsträngigen Nucleotidsequenz in einem ersten flüssigen Medium, welche Methode Folgendes umfasst:
Bereitstellen des ersten flüssigen Mediums, das Probe-Nucleotidsequenzen umfasst,
Zugeben, zu dem ersten flüssigen Medium, einer PNA-Sonde, die in der Lage ist, einen Hybridisierungskomplex mit der Nucleotidsequenz zu bilden, wobei die PNA-Sonde einen fluoreszierenden Marker umfasst,
Bestrahlen des ersten flüssigen Mediums mit einem Laserstrahl, der eine Wellenlänge aufweist, die den fluoreszierenden Marker anregt und den fluoreszierenden Marker veranlasst, fluoreszierendes Licht auszustrahlen,
Erfassen einer ersten Intensität des fluoreszierenden Lichts in dem ersten flüssigen Medium, und
Feststellen, ob die erste Intensität einer zweiten Intensität eines zweiten flüssigen Mediums entspricht, das die mit der PNA-Sonde hybridisierte Nucleotidsequenz enthält, um zu erfassen, ob die Nucleotidsequenz sich in dem ersten flüssigen Medium befindet,
wobei die PNA-Sonde, die Nucleotidsequenz und der Hybridisierungskomplex nicht an einen festen Träger oder ein festes Gel gebunden sind und der Hybridisierungskomplex im wesentlichen aus einer PNA-Sequenz, einem Fluorophor und der Nucleotidsequenz besteht.

25. Methode für das Erfassen einer Nucleotidsequenz nach Anspruch 24, wobei der Laserstrahl durch einen Argonionenlaser hergestellt wird, der den Marker mit Licht von einer Wellenlänge von ca. 450 bis ca. 530 nm bestahlt.

26. Methode für das Erfassen einer Nucleotidsequenz nach Anspruch 24, wobei das fluoreszierende Licht in einem Bereich von 400 bis 1000 nm erfasst wird.

27. Methode für das Erfassen einer Nucleotidsequenz nach Anspruch 24, wobei (i) es sich bei der Nucleotidsequenz um eine Mutanttyp-DNA handelt, die von einer Wildtyp-DNA zu unterscheiden ist, die von der Mutanttyp-DNA verschieden ist, (ii) die PNA-Sonde einem Segment der Mutanttyp-DNA vollständig komplementär und einem Segment der Wildtyp-DNA nicht vollständig komplementär ist und (iii) die Mutanttyp-DNA erfasst wird, wenn die erste Intensität der zweiten Intensität entspricht.

28. Methode für das Erfassen einer Nucleotidsequenz nach Anspruch 24, wobei (i) es sich bei der Nucleotidsequenz um eine Wildtyp-DNA handelt, die von einer Mutanttyp-DNA zu unterscheiden ist, die von der Wildtyp-DNA verschieden ist, (ii) die PNA-Sonde einem Segment der Wildtyp-DNA vollständig komplementär und einem Segment der Mutanttyp-DNA nicht vollständig komplementär ist und (iii) die Wildtyp-DNA erfasst wird, wenn die erste Intensität der zweiten Intensität entspricht.

29. Methode für das Erfassen einer Nucleotidsequenz nach Anspruch 24, wobei die Nucleotidsequenz in dem flüssigen Medium vor dem Erfassungsschritt bei einer Temperatur von ca. 85 °C bis ca. 100 °C ca. 30 Sekunden bis ca. 5 Stunden denaturiert wird.

30. Methode für das Erfassen einer Nucleotidsequenz nach Anspruch 29, wobei die Bildung des Hybridisierungskomplexes bei einer Temperatur von ca. 4 °C bis ca. 75 °C 2 Minuten bis ca. 24 Stunden durchgeführt wird.

31. Methode für das Erfassen einer Nucleotidsequenz nach Anspruch 30, wobei der Denaturierungsschritt nicht länger als 60 Minuten durchgeführt wird, woraufhin die Temperatur passiv ohne Abschrecken auf Raumtemperatur abgekühlt wird.

32. Methode für das Erfassen einer Nucleotidsequenz nach Anspruch 24, wobei die PNA-Sonde dem ersten flüssigen Medium in einer Konzentration zugegeben wird, die das 1- bis 20fache derjenigen einer vermuteten Konzentration der Nucleotidsequenz beträgt.

33. Methode für das Erfassen einer Nucleotidsequenz nach Anspruch 24, wobei ein Gesamtvolumen des ersten flüssigen Mediums in dem Erfassungsschritt nicht mehr als ca. 5 Milliliter beträgt.

34. Methode für das Erfassen einer Nucleotidsequenz nach Anspruch 33, wobei das Gesamtvolumen nicht mehr als ca. 10 Mikroliter beträgt.

35. Methode für das Erfassen einer Nucleotidsequenz nach Anspruch 24, wobei es sich bei der Nucleotidsequenz um doppelsträngige DNA handelt.

36. Methode für das Erfassen einer Nucleotidsequenz nach Anspruch 29, wobei die PNA-Sonde dem ersten flüssigen Medium vor Abschluss des Denaturierungsschritts zugegeben wird.

37. Methode nach Anspruch 24, wobei alle bei dieser Methode verwendeten Sonden aus der gleichen Sequenz und dem gleichen Marker bestehen, wobei der Marker der einzige Marker ist, der bei der Methode erfasst wird.

38. Methode nach Anspruch 24, wobei das erfasste fluoreszierende Licht im wesentlichen aus dem von dem Marker ausgestrahlten fluoreszierenden Licht besteht.

39. Methode nach Anspruch 24, wobei die PNA-Sonde einem Segment der Nucleotidsequenz vollständig komplementär ist.

40. Methode nach Anspruch 24, wobei die PNA-Sonde eine Einbasenfehlpaarung eines Segments der Nucleotidsequenz ist.

41. Methode nach Anspruch 24, wobei die PNA-Sonde eine Zweibasenfehlpaarung eines Segments der Nucleotidsequenz ist.

42. Methode nach Anspruch 24, wobei die PNA-Sonde eine Dreibasenfehlpaarung eines Segments der Nucleotidsequenz ist.

43. Methode nach Anspruch 24, wobei (i) die Probe-Nucleotidsequenzen der Nucleotidsequenz identisch oder Analoge sind, die sich von der Nucleotidsequenz durch mindestens eine Base unterscheiden, (ii) die PNA-Sonde einem Segment der Nucleotidsequenz vollständig komplementär und einem Segment der Analoge nicht komplementär ist, (iii) die Nucleotidsequenz erfasst wird, wenn die erste Intensität der zweiten Intensität entspricht und (iv) die Analoge erfasst werden, wenn die erste Intensität der zweiten Intensität nicht entspricht.

44. Methode nach Anspruch 43, wobei die Analoge sich von der Nucleotidsequenz durch eine Base unterscheiden.

45. Methode nach Anspruch 43, wobei die Analoge sich von der Nucleotidsequenz durch zwei Basen unterscheiden.

46. Methode nach Anspruch 43, wobei die Analoge sich von der Nucleotidsequenz durch drei Basen unterscheiden.

47. Methode nach Anspruch 43, wobei alle bei dieser Methode verwendeten Sonden aus der gleichen Sequenz und dem gleichen Marker bestehen, wobei der Marker der einzige durch diese Methode erfasste Marker ist.

48. Methode nach Anspruch 47, wobei das erfasste fluoreszierende Licht im wesentlichen aus dem von dem Marker ausgestrahlten fluoreszierenden Licht besteht.

49. Methode nach Anspruch 48, wobei die Analoge sich von der Nucleotidsequenz durch eine Base unterscheiden.

50. Methode nach Anspruch 24, wobei (i) die Probe-Nucleotidsequenzen der Nucleotidsequenz identisch oder Analoge sind, die sich von der Nucleotidsequenz durch mindestens eine Base unterscheiden, (ii) die PNA-Sonde einem Segment der Analoge vollständig komplementär ist und einem Segment der Nucleotidsequenz nicht vollständig komplementär ist, (iii) die Nucleotidsequenz erfasst wird, wenn die erste Intensität der zweiten Intensität entspricht und (iv) die Analoge erfasst werden, wenn die erste Intensität der zweiten Intensität nicht entspricht.

51. Methode nach Anspruch 50, wobei die Analoge sich von der Nucleotidsequenz durch eine Base unterscheiden.

52. Methode nach Anspruch 50, wobei die Analoge sich von der Nucleotidsequenz durch zwei Basen unterscheiden.

53. Methode nach Anspruch 50, wobei die Analoge sich von der Nucleotidsequenz durch drei Basen unterscheiden.

54. Methode nach Anspruch 50, wobei alle bei dieser Methode verwendeten Sonden aus der gleichen Sequenz und dem gleichen Marker bestehen, wobei der Marker der einzige durch diese Methode erfasste Marker ist.

55. Methode nach Anspruch 54, wobei das erfasste fluoreszierende Licht im wesentlichen aus dem von dem Marker ausgestrahlten fluoreszierenden Licht besteht.

56. Methode nach Anspruch 55, wobei die Analoge sich von der Nucleotidsequenz durch eine Basen unterscheiden.

57. Methode nach Anspruch 24, wobei das erfasste fluoreszierende Licht eine Wellenlänge besitzt, die länger ist als die Laserstrahlwellenlänge.

58. Methode nach Anspruch 24, wobei die erste Intensität einer Anzahl von Basenfehlpaarungen zwischen der Nucleotidsequenz und der PNA-Sonde über einen Bereich umgekehrt proportional, der 0 Basenfehlpaarungen bis mindestens 3 Basenfehlpaarungen umfasst.

59. Methode für das Erfassen einer einzelsträngigen oder doppelsträngigen Nucleotidsequenz in einem ersten flüssigen Medium, die Folgendes umfasst:
Bereitstellen des ersten flüssigen Mediums, das Probe-Nucleotidsequenzen umfasst,
Zugeben, zu dem ersten flüssigen Medium, einer PNA-Sonde, die in der Lage ist, einen Hybridisierungskomplex mit der Nucleotidsequenz zu bilden, wobei die PNA-Sonde einen fluoreszierenden Marker umfasst,
Abtrennen der nichthybridisierten PNA-Sonde von dem Hybridisierungskomplex unter Bildung eines ersten Testmediums,
Bestrahlen des ersten Testmediums mit einem Laserstrahl, der eine Wellenlänge aufweist, die den fluoreszierenden Marker anregt und den fluoreszierenden Marker veranlasst, fluoreszierendes Licht auszustrahlen,
direktes Erfassen einer ersten Intensität des ausgestrahlten fluoreszierenden Lichts in dem ersten Testmedium, und
Vergleichen der ersten Intensität mit einer Bezugsintensität, die durch Sondieren einer positiven Kontrollsequenz hergestellt wird, und einer Basislinienintensität, die durch Sondieren einer negativen Kontrollsequenz hergestellt wird,
wobei die Nucleotidsequenz erfasst wird, wenn die erste Intensität der Bezugsintensität entspricht, die Nucleotidsequenz nicht erfasst wird, wenn die erste Intensität der Basislinienintensität entspricht, und eine homologe Sequenz, die sich von der Nucleotidsequenz durch mindestens eine Base unterscheidet, erfasst wird, wenn die erste Intensität zwischen der Basislinienintensität und der Bezugsintensität liegt und wobei die Methode, mit Ausnahme des Abtrennschritts, vollkommen ohne Binden der PNA-Sonde, der Nucleotidsequenz oder des Hybridisierungskomplexes an einen festen Träger oder ein festes Gel durchgeführt wird.

60. Methode nach Anspruch 59, wobei die homologe Sequenz sich durch eine Base von der Nucleotidsequenz unterscheidet.

61. Methode nach Anspruch 59, wobei der Hybridisierungskomplex im wesentlichen aus einer PNA-Sequenz, einem Fluorophor und der Nucleotidsequenz besteht.

62. Methode nach Anspruch 59, wobei alle bei dieser Methode verwendeten Sonden aus der gleichen Sequenz und dem gleichen Marker bestehen, wobei der Marker der einzige durch diese Methode erfasste Marker ist.

63. Methode nach Anspruch 59, wobei das erfasste fluoreszierende Licht im wesentlichen aus dem von dem Marker ausgestrahlten fluoreszierenden Licht besteht.

64. Methode nach Anspruch 59, wobei das erfasste fluoreszierende Licht eine Wellenlänge aufweist, die länger ist als die Laserstrahlwellenlänge.

65. Methode nach Anspruch 59, wobei die erste Intensität einer Anzahl von Basenfehlpaarungen zwischen der Nucleotidsequenz und der PNA-Sonde über einen Bereich umgekehrt proportional ist, der 0 bis mindestens 3 Basenfehlpaarungen umfasst.

66. Methode für das Erfassen einer einzelsträngigen oder doppelsträngigen Nucleotid-Zielsequenz in einem flüssigen Medium, welche Methode Folgendes umfasst:
Bereitstellen des flüssigen Mediums, das erste Nucleotidsequenzen, die der Nucleotidsequenz identisch sind, oder zweite Nucleotidsequenzen umfasst, die sich von der Nucleotidsequenz durch mindestens eine Base unterscheiden,
Zugeben, zu dem flüssigen Medium, von PNA-Sonden, die einem Segment der Nucleotidsequenz vollständig komplementär und einem Segment der zweiten Nucleotidsequenzen nicht vollständig komplementär sind, wobei jede der PNA-Sonden einen fluoreszierenden Marker umfasst,
Hybridisieren der PNA-Sonden mit den ersten oder zweiten Nucleotidsequenzen,
Bestrahlen des flüssigen Mediums, um den fluoreszierenden Marker dazu zu bringen, fluoreszierendes Licht einer Wellenlänge von 400 bis 1000 nm auszustrahlen, und
Erfassen einer Intensität des fluoreszierenden Lichts,
wobei ein elektrisches Feld vor oder gleichzeitig mit dem Erfassungsschritt an das flüssige Medium angelegt wird und eine Änderung in der Intensität des fluoreszierenden Lichts als Funktion des elektrischen Felds erfasst wird als Anzeichen, ob die PNA-Sonden zu den ersten Nucleotidsequenzen oder den zweiten Nucleotidsequenzen hybridisiert werden.

67. Methode nach Anspruch 66, wobei die ersten und zweiten Nucleotidsequenzen sich durch eine Base voneinander unterscheiden.

## Revendications

1. Une méthode qui permet de détecter au moins une séquence nucléotidique monocaténaire ou bicaténaire cible dans un milieu liquide, ladite méthode comprenant :
l'addition audit milieu liquide d'une sonde de type PNA (analogues peptidiques de l'ADN) capable de former un complexe d'hybridation avec la ou lesdites séquences cibles, ladite sonde de type PNA comportant un marqueur fluorescent ;
la séparation de la sonde de type PNA non hybridée dudit complexe d'hybridation pour former un milieu d'analyse ;
l'irradiation dudit milieu d'analyse avec un faisceau laser à une longueur d'onde qui excite ledit marqueur fluorescent et cause l'émission d'une lumière fluorescente par ce marqueur fluorescent ;
la mesure de l'intensité de ladite lumière fluorescente émise ; et
la comparaison de ladite intensité mesurée avec une intensité de référence pour détecter si ledit milieu liquide contient la ou lesdites séquences cibles et pour déterminer le nombre de non-appariements entre la ou lesdites séquences cibles et ladite sonde de type PNA sur une plage qui va de 0 inclus à 3 défauts de complémentarité au moins ;
et, à l'exception de l'étape de séparation, ladite méthode étant effectuée sans fixation de ladite sonde de type PNA, de ladite séquence nucléotidique ou dudit complexe d'hybridation sur un support solide ou sur un gel.

2. La méthode de détection d'au moins une séquence nucléotidique selon la revendication 1, dans laquelle ladite séparation est accomplie par au moins une filtration, centrifugation, précipitation et électrophorèse en solution libre.

3. La méthode de détection d'au moins une séquence nucléotidique selon la revendication 1, dans laquelle la longueur d'onde dudit faisceau laser est comprise entre 450 et 530 nm environ.

4. La méthode de détection d'au moins une séquence nucléotidique selon la revendication 1, dans laquelle ladite lumière fluorescente émise est mesurée entre 400 et 1 000 nm.

5. La méthode de détection d'au moins une séquence nucléotidique selon la revendication 1, dans laquelle au moins deux sondes de type PNA différentes capables de former un complexe d'hybridation avec la ou lesdites séquences cibles sont ajoutées audit milieu liquide, la première de ces sondes de type PNA différentes utilisées au nombre de deux au moins étant complémentaire d'une première région sur une première séquence nucléotidique cible, une deuxième de ces sondes de type PNA différentes utilisées au nombre de deux au moins étant complémentaire d'une deuxième région sur une deuxième séquence nucléotidique cible, et cette première et deuxième région différant l'une de l'autre.

6. La méthode de détection d'au moins une séquence nucléotidique selon la revendication 5, dans laquelle la première et la deuxième desdites sondes sont entièrement complémentaires de la première et de la deuxième desdites régions, respectivement.

7. La méthode de détection d'au moins une séquence nucléotidique selon la revendication 6, dans laquelle ladite première séquence nucléotidique est complémentaire de ladite deuxième séquence nucléotidique.

8. La méthode de détection d'au moins une séquence nucléotidique selon la revendication 7, dans laquelle ladite première séquence nucléotidique et ladite deuxième séquence nucléotidique sont couplées pour former une molécule d'ADN bicaténaire.

9. La méthode de détection d'au moins une séquence nucléotidique selon la revendication 6, dans laquelle ladite première séquence nucléotidique et ladite deuxième séquence nucléotidique appartiennent à des génomes différents.

10. La méthode de détection d'au moins une séquence nucléotidique selon la revendication 9, dans laquelle ladite première sonde porte un premier marqueur qui produit une première émission fluorescente dont l'intensité est mesurée à une première longueur d'onde, ladite deuxième sonde porte un deuxième marqueur qui produit une deuxième émission fluorescente dont l'intensité est mesurée à une deuxième longueur d'onde, lesdites première et deuxième longueurs d'onde étant différentes, ladite première séquence nucléotidique étant détectée en mesurant l'intensité de l'émission fluorescente à ladite première longueur d'onde, et ladite deuxième séquence nucléotidique étant détectée en mesurant l'intensité de l'émission fluorescente à ladite deuxième longueur d'onde.

11. La méthode de détection d'au moins une séquence nucléotidique selon la revendication 6, dans laquelle ladite première sonde porte un premier marqueur qui produit une première émission fluorescente dont l'intensité est mesurée à une première longueur d'onde, ladite deuxième sonde porte un deuxième marqueur qui produit une deuxième émission fluorescente dont l'intensité est mesurée à une deuxième longueur d'onde, lesdites première et deuxième longueurs d'onde étant différentes, ladite première séquence nucléotidique étant détectée en mesurant l'intensité de l'émission fluorescente à ladite première longueur d'onde, et ladite deuxième séquence nucléotidique étant détectée en mesurant l'intensité de l'émission fluorescente à ladite deuxième longueur d'onde.

12. La méthode de détection d'au moins une séquence nucléotidique selon la revendication 11, dans laquelle ladite première séquence nucléotidique est un contrôle positif en théorie présent dans ledit milieu liquide testé, ladite première intensité est appelée intensité de référence, ladite deuxième intensité est appelée intensité mesurée, et ladite deuxième séquence nucléotidique est détectée par une comparaison entre ladite première intensité et ladite deuxième intensité.

13. La méthode de détection d'au moins une séquence nucléotidique selon la revendication 12, dans laquelle la deuxième séquence nucléotidique est détectée uniquement dans un génome mutant et la première séquence nucléotidique est détectée dans ledit génome mutant et dans un génome de type sauvage correspondant.

14. La méthode de détection d'au moins une séquence nucléotidique selon la revendication 13, dans laquelle le premier et le deuxième marqueurs sont sélectionnés parmi le groupe consistant en la fluorescéine et la rhodamine.

15. La méthode de détection d'au moins une séquence nucléotidique selon la revendication 12, dans laquelle une troisième sonde de type PNA portant un troisième marqueur qui produit une troisième émission fluorescente dont l'intensité est mesurée à une troisième longueur d'onde différente de la première et de la deuxième longueurs d'onde est ajoutée audit milieu liquide comme contrôle négatif, qui ne devrait en théorie s'hybrider à aucune des séquences nucléotidiques, et dans laquelle ladite deuxième séquence nucléotidique est détectée par une comparaison entre la première, la deuxième et la troisième intensités.

16. La méthode de détection d'au moins une séquence nucléotidique selon la revendication 1, dans laquelle au moins deux sondes de type PNA différentes capables de former un complexe d'hybridation avec la ou lesdites séquences cibles sont ajoutées audit milieu liquide, la première de ces sondes de type PNA différentes utilisées au nombre de deux au moins étant complémentaire d'une première région sur la ou lesdites séquences cibles, une deuxième de ces sondes de type PNA différentes utilisées au nombre de deux au moins étant complémentaire d'une deuxième région sur la ou lesdites séquences cibles, et cette première et deuxième régions différant l'une de l'autre.

17. La méthode de détection d'au moins une séquence nucléotidique selon la revendication 16, dans laquelle ladite première sonde porte un premier marqueur qui produit une première émission fluorescente dont l'intensité est mesurée à une première longueur d'onde, ladite deuxième sonde porte un deuxième marqueur qui produit une deuxième émission fluorescente dont l'intensité est mesurée à une deuxième longueur d'onde, lesdites première et deuxième longueurs d'onde étant différentes, ladite première région étant détectée en mesurant l'intensité de l'émission fluorescente à ladite première longueur d'onde, et ladite deuxième région étant détectée en mesurant l'intensité de l'émission fluorescente à ladite deuxième longueur d'onde.

18. La méthode de détection d'au moins une séquence nucléotidique selon la revendication 17, dans laquelle ladite première région est un contrôle positif en théorie présent dans ledit milieu liquide testé, ladite première intensité est appelée intensité de référence, ladite deuxième intensité est appelée intensité mesurée, et la dite deuxième région est détectée par une comparaison entre ladite première intensité et ladite deuxième intensité.

19. La méthode de détection d'au moins une séquence nucléotidique selon la revendication 18, dans laquelle une troisième sonde de type PNA portant un troisième marqueur qui produit une troisième émission fluorescente dont l'intensité est mesurée à une troisième longueur d'onde différente de la première et de la deuxième longueurs d'onde est ajoutée audit milieu liquide comme contrôle négatif, qui ne devrait en théorie s'hybrider à aucune des régions nucléotidiques, et dans laquelle ladite deuxième région est détectée par une comparaison entre la première, la deuxième et la troisième intensités.

20. La méthode de détection d'au moins une séquence nucléotidique selon la revendication 1, dans laquelle ledit complexe d'hybridation consiste essentiellement en une séquence de type PNA (analogues peptidiques de l'ADN), un fluorophore et ladite séquence nucléotidique.

21. La méthode de détection d'au moins une séquence nucléotidique selon la revendication 1, dans laquelle toutes les sondes employées dans ladite méthode consistent en la même séquence et un marqueur, ledit marqueur étant le seul marqueur détecté par ladite méthode.

22. La méthode de détection d'au moins une séquence nucléotidique selon la revendication 1, dans laquelle la longueur d'onde de ladite lumière fluorescente émise est plus longue que la longueur d'onde dudit faisceau laser.

23. La méthode de détection d'au moins une séquence nucléotidique selon la revendication 1, dans laquelle la ou lesdites séquences nucléotidiques sont une séquence d'ADN bicaténaire.

24. Une méthode qui permet de détecter au moins une séquence nucléotidique monocaténaire ou bicaténaire cible dans un premier milieu liquide, comprenant :
à condition que ledit premier milieu liquide comprenne les séquences nucléotidiques échantillons ;
l'addition audit premier milieu liquide d'une sonde de type PNA capable de former un complexe d'hybridation avec ladite séquence nucléotidique, ladite sonde de type PNA comportant un marqueur fluorescent ;
l'irradiation dudit premier milieu liquide avec un faisceau laser à une longueur d'onde qui excite ledit marqueur fluorescent et cause l'émission d'une lumière fluorescente par ce marqueur fluorescent ;
la détection d'une première intensité de ladite lumière fluorescente dans ledit premier milieu liquide ; et
l'examen de ladite première intensité pour déterminer si elle est égale à une seconde intensité produite par un second milieu liquide contenant ladite séquence nucléotidique hybridée avec ladite sonde de type PNA, pour détecter si ladite séquence nucléotidique est présente dans ledit premier milieu liquide,
dans laquelle ladite sonde de type PNA, ladite séquence nucléotidique et ledit complexe d'hybridation ne sont pas fixés sur un support solide ou sur un gel, et ledit complexe d'hybridation consiste essentiellement en une séquence de type PNA (analogues peptidiques de l'ADN), un fluorophore et ladite séquence nucléotidique.

25. La méthode de détection d'une séquence nucléotidique selon la revendication 24, dans laquelle ledit faisceau laser est produit par un laser argon-ion qui est utilisé pour irradier ledit marqueur avec une lumière à une longueur d'onde comprise entre 450 et 530 nm.

26. La méthode de détection d'une séquence nucléotidique selon la revendication 24, dans laquelle ladite lumière fluorescente est détectée entre 400 et 1 000 nm.

27. La méthode de détection d'une séquence nucléotidique selon la revendication 24, dans laquelle (i) ladite séquence nucléotidique est un ADN de type mutant qui doit être distingué d'avec un ADN de type sauvage différent dudit ADN de type mutant, (ii) ladite sonde de type PNA est entièrement complémentaire d'une région dudit ADN de type mutant et elle n'est pas entièrement complémentaire d'une région dudit ADN de type sauvage, et (iii) ledit ADN de type mutant est détecté si ladite première intensité est égale à ladite deuxième intensité.

28. La méthode de détection d'une séquence nucléotidique selon la revendication 24, dans laquelle (i) ladite séquence nucléotidique est un ADN de type sauvage qui doit être distingué d'avec un ADN de type mutant différent dudit ADN de type sauvage, (ii) ladite sonde de type PNA est entièrement complémentaire d'une région dudit ADN de type sauvage et elle n'est pas entièrement complémentaire d'une région dudit ADN de type mutant, et (iii) ledit ADN de type sauvage est détecté si ladite première intensité est égale à ladite deuxième intensité.

29. La méthode de détection d'une séquence nucléotidique selon la revendication 24, dans laquelle ladite séquence nucléotidique est soumise, avant ladite étape de détection, à une dénaturation dans ledit milieu liquide à une température de l'ordre de 85°C à 100°C sur une période qui va de 30 secondes à 5 heures environ.

30. La méthode de détection d'une séquence nucléotidique selon la revendication 29, dans laquelle la formation dudit complexe d'hybridation est effectuée à une température de l'ordre de 4°C à 75°C sur une période qui va de 2 minutes à 24 heures environ.

31. La méthode de détection d'une séquence nucléotidique selon la revendication 30, dans laquelle l'étape de dénaturation est réalisée sur 60 minutes au maximum, période après laquelle ladite température est ramenée passivement à température ambiante sans refroidissement brusque.

32. La méthode de détection d'une séquence nucléotidique selon la revendication 24, dans laquelle ladite sonde de type PNA est ajoutée audit premier milieu liquide à une concentration correspondant à 1 à 20 fois la concentration anticipée de ladite séquence nucléotidique.

33. La méthode de détection d'une séquence nucléotidique selon la revendication 24, dans laquelle le volume total dudit premier milieu liquide dans ladite étape de détection n'excède pas 5 millilitres environ.

34. La méthode de détection d'une séquence nucléotidique selon la revendication 33, dans laquelle le volume total n'excède pas 10 microlitres environ.

35. La méthode de détection d'une séquence nucléotidique selon la revendication 24, dans laquelle ladite séquence nucléotidique est une séquence d'ADN bicaténaire.

36. La méthode de détection d'une séquence nucléotidique selon la revendication 29, dans laquelle ladite sonde de type PNA est ajouté audit premier milieu liquide avant la terminaison de ladite étape de dénaturation.

37. La méthode selon la revendication 24, dans laquelle toutes les sondes employées dans ladite méthode consistent en la même séquence et un marqueur, ledit marqueur étant le seul marqueur détecté par ladite méthode.

38. La méthode selon la revendication 24, dans laquelle ladite lumière fluorescente détectée consiste essentiellement en ladite lumière fluorescente émise par ledit marqueur.

39. La méthode selon la revendication 24, dans laquelle ladite sonde de type PNA est entièrement complémentaire d'une région de ladite séquence nucléotidique.

40. La méthode selon la revendication 24, dans laquelle ladite sonde de type PNA présente un défaut de complémentarité par rapport à une région de ladite séquence nucléotidique.

41. La méthode selon la revendication 24, dans laquelle ladite sonde de type PNA présente deux défauts de complémentarité par rapport à une région de ladite séquence nucléotidique.

42. La méthode selon la revendication 24, dans laquelle ladite sonde de type PNA présente trois défauts de complémentarité par rapport à une région de ladite séquence nucléotidique.

43. La méthode selon la revendication 24, dans laquelle (i) lesdites séquences nucléotidiques échantillons sont identiques aux dites séquences nucléotidiques
ou sont des analogues qui diffèrent desdites séquences nucléotidiques au niveau d'au moins une base, (ii) ladite sonde de type PNA est entièrement complémentaire d'une région de ladite séquence nucléotidique et elle n'est pas complémentaire d'une région desdits analogues, (iii) ladite séquence nucléotidique est détectée si ladite première intensité est égale à ladite seconde intensité, et (iv) lesdits analogues sont détectés si ladite première intensité n'est pas égale à ladite deuxième intensité.

44. La méthode selon la revendication 43, dans laquelle lesdits analogues diffèrent de ladite séquence nucléotidique de une base.

45. La méthode selon la revendication 43, dans laquelle lesdits analogues diffèrent de ladite séquence nucléotidique de deux bases.

46. La méthode selon la revendication 43, dans laquelle lesdits analogues diffèrent de ladite séquence nucléotidique de trois bases.

47. La méthode selon la revendication 43, dans laquelle toutes les sondes employées dans ladite méthode consistent en la même séquence et un marqueur, ledit marqueur étant le seul marqueur détecté par ladite méthode.

48. La méthode selon la revendication 47, dans laquelle ladite lumière fluorescente détectée consiste essentiellement en ladite lumière fluorescente émise par ledit marqueur.

49. La méthode selon la revendication 48, dans laquelle lesdits analogues diffèrent de ladite séquence nucléotidique de une base.

50. La méthode selon la revendication 24, dans laquelle (i) lesdites séquences nucléotidiques échantillons sont identiques à ladite séquence nucléotidique ou sont des analogues qui diffèrent de ladite séquence nucléotidique au niveau d'au moins une base, (ii) ladite sonde de type PNA est entièrement complémentaire d'une région desdits analogues et elle n'est pas entièrement complémentaire d'une région de ladite séquence nucléotidique, (iii) ladite séquence nucléotidique est détectée si ladite première intensité est égale à ladite seconde intensité, et (iv) lesdits analogues sont détectés si ladite première intensité n'est pas égale à ladite deuxième intensité.

51. La méthode selon la revendication 50, dans laquelle lesdits analogues diffèrent de ladite séquence nucléotidique de une base.

52. La méthode selon la revendication 50, dans laquelle lesdits analogues diffèrent de ladite séquence nucléotidique de deux bases.

53. La méthode selon la revendication 50, dans laquelle lesdits analogues diffèrent de ladite séquence nucléotidique de trois bases.

54. La méthode selon la revendication 50, dans laquelle toutes les sondes employées dans ladite méthode consistent en la même séquence et un marqueur, ledit marqueur étant le seul marqueur détecté par ladite méthode.

55. La méthode selon la revendication 54, dans laquelle ladite lumière fluorescente détectée consiste essentiellement en ladite lumière fluorescente émise par ledit marqueur.

56. La méthode selon la revendication 55, dans laquelle lesdits analogues diffèrent de ladite séquence nucléotidique de une base.

57. La méthode selon la revendication 24, dans laquelle la longueur d'onde de ladite lumière fluorescente détectée est plus longue que la longueur d'onde dudit faisceau laser.

58. La méthode selon la revendication 24, dans laquelle ladite première intensité est inversement proportionnelle au nombre de non-appariements des paires de bases entre ladite séquence nucléotidique et ladite sonde de type PNA, sur une plage qui va de 0 inclus à 3 défauts de complémentarité au minimum.

59. Une méthode qui permet de détecter une séquence nucléotidique monocaténaire ou bicaténaire dans un premier milieu liquide, comprenant :
à condition que ledit premier milieu liquide comprenne les séquences nucléotidiques échantillons ;
l'addition audit premier milieu liquide d'une sonde de type PNA capable de former un complexe d'hybridation avec ladite séquence nucléotidique, ladite sonde de type PNA comportant un marqueur fluorescent ;
la séparation de la sonde de type PNA non hybridée dudit complexe d'hybridation pour former le premier milieu d'essai ;
l'irradiation dudit premier milieu d'essai avec un faisceau laser à une longueur d'onde qui excite ledit marqueur fluorescent et cause l'émission d'une lumière fluorescente par ce marqueur fluorescent ;
la détection directe d'une première intensité de ladite lumière fluorescente émise dans ledit premier milieu d'essai ; et
la comparaison de ladite première intensité avec une intensité de référence produite par une séquence servant de contrôle positif et avec une intensité de base produite par une séquence servant de contrôle négatif,
dans laquelle ladite séquence nucléotidique est détectée quand ladite première intensité est égale à ladite intensité de référence, ladite séquence nucléotidique n'est pas détectée si ladite première intensité est égale à l'intensité de base, et une séquence homologue différant d'une base au moins de ladite séquence nucléotidique est détectée quand ladite première intensité est comprise entre ladite intensité de base et ladite intensité de référence, et dans laquelle, et à l'exception de l'étape de séparation, ladite méthode est effectuée entièrement sans fixation de ladite sonde de type PNA, de ladite séquence nucléotidique ou dudit complexe d'hybridation sur un support solide ou sur un gel.

60. La méthode selon la revendication 59, dans laquelle ladite séquence homologue diffère d'une base de ladite séquence nucléotidique.

61. La méthode selon la revendication 59, dans laquelle ledit complexe d'hybridation consiste essentiellement en une séquence de type PNA (analogues peptidiques de l'ADN), un fluorophore et ladite séquence nucléotidique.

62. La méthode selon la revendication 59, dans laquelle toutes les sondes employées dans ladite méthode consistent en la même séquence et un marqueur, ledit marqueur étant le seul marqueur détecté par ladite méthode.

63. La méthode selon la revendication 59, dans laquelle ladite lumière fluorescente détectée consiste essentiellement en ladite lumière fluorescente émise par ledit marqueur.

64. La méthode selon la revendication 59, dans laquelle la longueur d'onde de ladite lumière fluorescente détectée est plus longue que la longueur d'onde dudit faisceau laser.

65. La méthode selon la revendication 59, dans laquelle ladite première intensité est inversement proportionnelle au nombre de non-appariements des paires de bases entre ladite séquence nucléotidique et ladite sonde de type PNA, sur une plage qui va de 0 inclus à 3 défauts de complémentarité au minimum.

66. Une méthode qui permet de détecter une séquence nucléotidique monocaténaire ou bicaténaire dans un milieu liquide, comprenant :
à condition que ledit milieu liquide comprenne des premières séquences nucléotidiques qui sont identiques à ladite séquence nucléotidique ou des deuxièmes séquences nucléotidiques qui diffèrent d'une base au moins de ladite séquence nucléotidique ;
l'addition audit milieu liquide de sondes de type PNA qui sont entièrement complémentaires d'une région de ladite séquence nucléotidique et qui ne sont pas entièrement complémentaires d'une région desdites deuxièmes séquences nucléotidiques, dans laquelle chacune desdites sondes de type PNA comprend un marqueur fluorescent ;
l'hybridation desdites sondes de type PNA aux dites premières et deuxièmes séquences nucléotidiques ;
l'irradiation dudit milieu liquide pour produire l'émission, par le marqueur fluorescent, d'une lumière fluorescente à une longueur d'onde comprise entre 400 et 1 000 nm ; et
la détection d'une intensité de ladite lumière fluorescente,
dans laquelle un champ électrique est appliqué audit milieu liquide avant ladite étape de détection ou en même temps, et une modification de ladite intensité de ladite lumière fluorescente proportionnelle audit champ électrique est détectée, démontrant si lesdites sondes de type PNA sont hybridées ou non aux dites premières séquences nucléotidiques ou aux dites deuxièmes séquences nucléotidiques.

67. La méthode selon la revendication 66, dans laquelle lesdites premières et deuxièmes séquences nucléotidiques diffèrent d'une base.


### SEQUENCE LISTING

### (1) GENERAL INFORMATION:

(i) APPLICANTS: Eileen Nie and Yuan Min Wu
(ii) TITLE OF THE INVENTION: PNA Diagnostic Methods
(iii) NUMBER OF SEQUENCES: 13
(iv) CORRESPONDENCE ADDRESS:
   (A) ADDRESSEE: Caesar, Rivise, Bernstein, Cohen & Pokotilow, Ltd.
   (B) STREET: 12th Floor, 7 Penn Center, 1635 Market Street
   (C) CITY: Philadelphia
   (D) STATE: Pennsylvania
   (E) COUNTRY: U.S.A.
   (F) ZIP: 19103-2212
(v) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.30
(vi) CURRENT APPLICATION DATA:
   (A) APPLICATION NUMBER:
   (B) FILING DATE:
(viii) ATTORNEY/AGENT INFORMATION:
   (A) NAME: Tener, David M.
   (B) REGISTRATION NUMBER: 37,054
   (C) REFERENCE/DOCKET NUMBER: E1047/20001
(ix) TELECOMMUNICATION INFORMATION:
   (A) TELEPHONE: 215-567-2010
   (B) TELEFAX: 215-751-1142

### (2) INFORMATION FOR SEQ ID NO:1:

(i) SEQUENCE CHARACTERISTICS:
   (A) LENGTH: 150 bases
   (B) TYPE: nucleotide
   (C) STRANDEDNESS: double-stranded
   (D) TOPOLOGY: linear
(ii) MOLECULE TYPE: genomic DNA
(xi) SEQUENCE DESCRIPTION: SEQ ID NO:1:
   AACACCAGCT CCTCTCCCCA GCCAAAGAAG AAACCACTGG ATGGAGAATA TTTCACCCTT  60
   CAGATCCGTG GGCGTGAGCG CTTCGAGATG TTCCGAGAGC TGAATGAGGC CTTGGAACTC 120
   AAGGATGCCC AGGCTGGGAA GGAGCCAGGG                                  150

### (2) INFORMATION FOR SEQ ID NO:2:

(i) SEQUENCE CHARACTERISTICS:
   (A) LENGTH: 150 bases
   (B) TYPE: nucleotide
   (C) STRANDEDNESS: double-stranded
   (D) TOPOLOGY: linear
(xi) SEQUENCE DESCRIPTION: SEQ ID NO:2:
   AACACCAGCT CCTCTCCCCA GCCAAAGAAG AAACCACTGG ATGGAGAATA TTTCACCCTT  60
   CAGATCCGTG GGCGTGAGCG CTTCGAGATG TTCCGAGAGC AGAATGAGGC CTTGGAACTC 120
   AAGGATGCCC AGGCTGGGAA GGAGCCAGGG                                  150

### (2) INFORMATION FOR SEQ ID NO:3:

(i) SEQUENCE CHARACTERISTICS:
   (A) LENGTH: 150 bases
   (B) TYPE: nucleotide
   (C) STRANDEDNESS: double-stranded
   (D) TOPOLOGY: linear
(xi) SEQUENCE DESCRIPTION: SEQ ID NO:3:
   AACACCAGCT CCTCTCCCCA GCCAAAGAAG AAACCACTGG ATGGAGAATA TTTCACCCTT   60
   CAGATCCGTG GGCGTGAGCG CTTCGAGATG TTCCGAGAGC GGAATGAGGC CTTGGAACTC  120
   AAGGATGCCC AGGCTGGGAA GGAGCCAGGG                                    150

### (2) INFORMATION FOR SEQ ID NO:4:

(i) SEQUENCE CHARACTERISTICS:
   (A) LENGTH: 150 bases
   (B) TYPE: nucleotide
   (C) STRANDEDNESS: double-stranded
   (D) TOPOLOGY: linear
(xi) SEQUENCE DESCRIPTION: SEQ ID NO:4:
   AACACCAGCT CCTCTCCCCA GCCAAAGAAG AAACCACTGG ATGGAGAATA TTTCACCCTT   60
   CAGATCCGTG GGCGTGAGCG CTTCGAGATG TTCCGAGAGA AGAATGAGGC CTTGGAACTC  120
   AAGGATGCCC AGGCTGGGAA GGAGCCAGGG                                    150

### (2) INFORMATION FOR SEQ ID NO:5:

(i) SEQUENCE CHARACTERISTICS:
   (A) LENGTH: 150 bases
   (B) TYPE: nucleotide
   (C) STRANDEDNESS: double-stranded
   (D) TOPOLOGY: linear
(xi) SEQUENCE DESCRIPTION: SEQ ID NO:5:
   AACACCAGCT CCTCTCCCCA GCCAAAGAAG AAACCACTGG ATGGAGAATA TTTCACCCTT 60
   CAGATCCGTG GGCGTGAGCG CTTCGAGATG TTCCGAGAGG CGAATGAGGC CTTGGAACTC 120
   AAGGATGCCC AGGCTGGGAA GGAGCCAGGG 150

### (2) INFORMATION FOR SEQ ID NO:6:

(i) SEQUENCE CHARACTERISTICS:
   (A) LENGTH: 150 bases
   (B) TYPE: nucleotide
   (C) STRANDEDNESS: double-stranded
   (D) TOPOLOGY: linear
(xi) SEQUENCE DESCRIPTION: SEQ ID NO:6:
   AACACCAGCT CCTCTCCCCA GCCAAAGAAG AAACCACTGG ATGGAGAATA TTTCACCCTT 60
   CAGATCCGTG GGCGTGAGCG CTTCGAGATG TTCCGAGAGT ACAATGAGGC CTTGGAACTC 120
   AAGGATGCCC AGGCTGGGAA GGAGCCAGGG 150

### (2) INFORMATION FOR SEQ ID NO:7:

(i) SEQUENCE CHARACTERISTICS:
   (A) LENGTH: 375 bases
   (B) TYPE: nucleotide
   (C) STRANDEDNESS: double-stranded
   (D) TOPOLOGY: linear
(xi) SEQUENCE DESCRIPTION: SEQ ID NO:7:
   TTAATACGAC TCACTATAGG GGAATTGTGA GCGGATAACA ATTCCCCTCT AGAAATAATT  60
   TTGTTTAACT TTAAGAAGGA GATATACCAT GGGCCATCAT CATCATCATC ATCATCATCA 120
   TCACAGCAGC GGCCATATCG ACGACGACGA CAAGCAAACA CCAGCTCCTC TCCCCAGCCA 180
   AAGAAGAAAC CACTGGATGG AGAATATTTC ACCCTTCAGA TCCGTGGGCG TGAGCGCTTC 240
   GAGATGTTCC GAGAGCTGAA TGAGGCCTTG GAACTCAAGG ATGCCCAGGC TGGGAAGGAG 300
   CCAGGGGATC CGGCTGCTAA CAAAGCCCGA AAGGAAGCTG AGTTGGCTGC TGCCACCGCT 360
   GAGCAATAAC TAGCA                                                  375

### (2) INFORMATION FOR SEQ ID NO:8:

(i) SEQUENCE CHARACTERISTICS:
   (A) LENGTH: 375 bases
   (B) TYPE: nucleotide
   (C) STRANDEDNESS: double-stranded
   (D) TOPOLOGY: linear
(xi) SEQUENCE DESCRIPTION: SEQ ID NO:8:
   TTAATACGAC TCACTATAGG GGAATTGTGA GCGGATAACA ATTCCCCTCT AGAAATAATT  60
   TTGTTTAACT TTAAGAAGGA GATATACCAT GGGCCATCAT CATCATCATC ATCATCATCA 120
   TCACAGCAGC GGCCATATCG ACGACGACGA CAAGCAAACA CCAGCTCCTC TCCCCAGCCA 180
   AAGAAGAAAC CACTGGATGG AGAATATTTC ACCCTTCAGA TCCGTGGGCG TGAGCGCTTC 240
   GAGATGTTCC GAGAGCAGAA TGAGGCCTTG GAACTCAAGG ATGCCCAGGC TGGGAAGGAG 300
   CCAGGGGATC CGGCTGCTAA CAAAGCCCGA AAGGAAGCTG AGTTGGCTGC TGCCACCGCT 360
   GAGCAATAAC TAGCA                                                  375

### (2) INFORMATION FOR SEQ ID NO:9:

(i) SEQUENCE CHARACTERISTICS:
   (A) LENGTH: 375 bases
   (B) TYPE: nucleotide
   (C) STRANDEDNESS: double-stranded
   (D) TOPOLOGY: linear
(xi) SEQUENCE DESCRIPTION: SEQ ID NO:9:
   TTAATACGAC TCACTATAGG GGAATTGTGA GCGGATAACA ATTCCCCTCT AGAAATAATT  60
   TTGTTTAACT TTAAGAAGGA GATATACCAT GGGCCATCAT CATCATCATC ATCATCATCA 120
   TCACAGCAGC GGCCATATCG ACGACGACGA CAAGCAAACA CCAGCTCCTC TCCCCAGCCA 180
   AAGAAGAAAC CACTGGATGG AGAATATTTC ACCCTTCAGA TCCGTGGGCG TGAGCGCTTC 240
   GAGATGTTCC GAGAGCGGAA TGAGGCCTTG GAACTCAAGG ATGCCCAGGC TGGGAAGGAG 300
   CCAGGGGATC CGGCTGCTAA CAAAGCCCGA AAGGAAGCTG AGTTGGCTGC TGCCACCGCT 360
   GAGCAATAAC TAGCA                                                  375

### (2) INFORMATION FOR SEQ ID NO:10:

(i) SEQUENCE CHARACTERISTICS:
   (A) LENGTH: 633 bases
   (B) TYPE: nucleotide
   (C) STRANDEDNESS: double-stranded
   (D) TOPOLOGY: linear
(xi) SEQUENCE DESCRIPTION: SEQ ID NO:10:
   CCGGCGGCCC CTGCACCAGC CCCCTCCTGG CCCCTGTCAT CTTCTGTCCC TTCCCAGAAA  60
   ACCTACCAGG GCAGCTACGG TTTCCGTCTG GGCTTCTTGC ATTCTGGGAC AGCCAAGTCT 120
   GTGACTTGCA CGTACTCCCC TGCCCTCAAC AAGATGTTTT GCCAACTGGC CAAGACCTGC 180
   CCTGTGCAGC TGTGGGTTGA TTCCACACCC CCGCCCGGCA CCCGCGTCCG CGCCATGGCC 240
   ATCTACAAGC AGTCACAGCA CATGACGGAG GTTGTGAGGC GCTGCCCCCA CCATGAGCGC 300
   TGCTCAGATA GCGATGGTCT GGCCCCTCCT CAGCATCTTA TCCGAGTGGA AGGAAATTTG 360
   CGTGTGGAGT ATTTGGATGA CAGAAACACT TTTCGACATA GTGTGGTGGT GCCCTATGAG 420
   CCGCCTGAGG TTGGCTCTGA CTGTACCACC ATCCACTACA ACTACATGTG TAACAGTTCC 480
   TGCATGGGCG GCATGAACCG GAGGCCCATC CTCACCATCA TCACACTGGA AGACTCCAGT 540
   GGTAATCTAC TGGGACGGAA CAGCTTTGAG GTGCGTGTTT GTGCCTGTCC TGGGAGAGAC 600
   CGGCGCACAG AGGAAGAGAA TCTCCGCAAG AAA                             633

### (2) INFORMATION FOR SEQ ID NO:11:

(i) SEQUENCE CHARACTERISTICS:
   (A) LENGTH: 150 bases
   (B) TYPE: nucleotide
   (C) STRANDEDNESS: double-stranded
   (D) TOPOLOGY: linear
(xi) SEQUENCE DESCRIPTION: SEQ ID NO:11:
   AACACCAGCT CCTCTCCCCA GCCAAAGAAG AAACCACTGG ATGGAGAATA TTTCACCCTT  60
   CAGATCCGTG GGCGTGAGCG CTTCGAGGAG TTCCGAGAGC TGAATGAGGC CTTGGAACTC 120
   AAGGATGCCC AGGCTGGGAA GGAGCCAGGG                                 150

### (2) INFORMATION FOR SEQ ID NO:12:

(i) SEQUENCE CHARACTERISTICS:
   (A) LENGTH: 150 bases
   (B) TYPE: nucleotide
   (C) STRANDEDNESS: double-stranded
   (D) TOPOLOGY: linear
(xi) SEQUENCE DESCRIPTION: SEQ ID NO:12:
   AACACCAGCT CCTCTCCCCA GCCAAAGAAG AAACCACTGG ATGGAGAATA TTTCACCCTT   60
   CAGATCCGTG GGCGTGAGCG CTTCGAGCAG TTCCGAGAGC TGAATGAGGC CTTGGAACTC  120
   AAGGATGCCC AGGCTGGGAA GGAGCCAGGG                                   150

### (2) INFORMATION FOR SEQ ID NO:13:

(i) SEQUENCE CHARACTERISTICS:
   (A) LENGTH: 150 bases
   (B) TYPE: nucleotide
   (C) STRANDEDNESS: double-stranded
   (D) TOPOLOGY: linear
(xi) SEQUENCE DESCRIPTION: SEQ ID NO:13:
   AACACCAGCT CCTCTCCCCA GCCAAAGAAG AAACCACTGG ATGGAGAATA TTTCACCCTT   60
   CAGATCCGTG GGCGTGAGCG CTTCCAGGAG TTCCGAGAGC GGAATGAGGC CTTGGAACTC  120
   AAGGATGCCC AGGCTGGGAA GGAGCCAGGG                                   150